# EUROPÄISCHE PATENTSCHRIFT

(11) **EP 1 641 799 B1**
(45) Veröffentlichungstag und Bekanntmachung des Hinweises auf die Patenterteilung: **12.03.2008**
(21) Anmeldenummer: 04736644.8
(22) Anmeldetag: 11.06.2004
(51) Int. Cl.: C07D 487/04, C07D 471/04, C07D 519/00, A61K 31/5025, A61P 3/10

(54) **IMIDAZOPYRIDAZINON- UND IMIDAZOPYRIDONDERIVATE, DEREN HERSTELLUNG UND DEREN VERWENDUNG ALS ARZNEIMITTEL**
IMIDAZO-PYRIDAZINONE DERIVATIVES AND IMIDAZO-PYRIDONE DERIVATIVES, PRODUCTION THEREOF, AND USE THEREOF AS MEDICAMENTS
DERIVES D'IMIDAZOPYRIDAZINONES ET D'IMIDAZOPYRIDONES, LEUR PREPARATION ET LEUR UTILISATION COMME MEDICAMENTS

(30) Priorität: 18.06.2003 DE 10327439
(43) Veröffentlichungstag der Anmeldung: 05.04.2006
(73) Patentinhaber: Boehringer Ingelheim International GmbH, 55216 Ingelheim am Rhein (DE); Boehringer Ingelheim Pharma GmbH, 55216 Ingelheim am Rhein (DE)
(72) Erfinder: ECKHARDT, Matthias, 88400 BIBERACH (DE); HAUEL, Norbert, 88433 SCHEMMERHOFEN (DE); LANGKOPF, Elke, 88447 WARTHAUSEN (DE); HIMMELSBACH, Frank, 88441 MITTELBIBERACH (DE); KAUFFMANN-HEFNER, Iris, 88448 ATTENWEILER (DE); TADAYYON, Mohammad, SG14 1HQ Hertford (GB); MARK, Michael, 88400 BIBERACH (DE)
(86) Internationale Anmeldenummer: PCT/EP2004/006303
(87) Internationale Veröffentlichungsnummer: WO 2004/111051

(56) Entgegenhaltungen:
- EP-A- 0 657 454
- WO-A-02/02560
- WO-A-99/29695
- WO-A-20/04050658
- US-A- 5 389 642
- US-A1- 2004 116 328

## Beschreibung

Gegenstand der vorliegenden Erfindung sind neue substituierte Imidazopyridazinone und Imidazopyridone der allgemeinen Formel 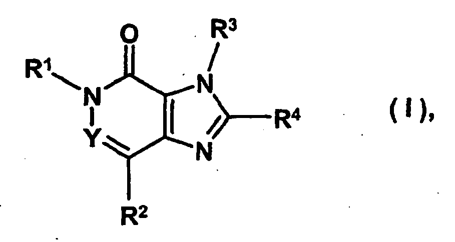 deren Tautomere, Enantiomere, Diastereomere, deren Gemische, deren Prodrugs und deren Salze, insbesonders deren physiologisch verträgliche Salze mit anorganischen oder organischen Säuren oder Basen, welche wertvolle pharmakologische Eigenschaften aufweisen, insbesondere eine Hemmwirkung auf die Aktivität des Enzyms Dipeptidylpeptidase-IV (DPP-IV), deren Herstellung, deren Verwendung zur Prävention oder Behandlung von Krankheiten oder Zuständen, die in Zusammenhang mit einer erhöhten DPP-IV Aktivität stehen oder die durch Reduktion der DPP-IV-Aktivität verhindert oder gemildert werden können, insbesondere von Diabetes mellitus Typ I oder Typ II, die eine Verbindung der allgemeinen Formel (I) oder ein physiologisch verträgliches Salz davon enthaltenden Arzneimittel sowie Verfahren zu deren Herstellung. Aus US-A-5389642 und EP-A- 0657454 sind Strukturöhnliche Imidazopyridazine mit Angiotensin II antagonistischen Eigenschaften bekannt.

Gegenstand der vorliegenden Erfindung sind somit die obigen Verbindungen der allgemeinen Formel I, welche wertvolle pharmakologische Eigenschaften aufweisen, die die pharmakologisch wirksamen Verbindungen enthaltenden Arzneimittel, deren Verwendung und Verfahren zu ihrer Herstellung.

In der obigen allgemeinen Formel 1 bedeuten
R¹ eine durch eine Gruppe Rₐ substituierte C₁₋₃-Alkylgruppe, wobei
   Rₐ eine 3,4-Dihydro-chinolinyl-, 3,4-Dihydro-isochinolinyl-, 1,4-Dihydro-chinazolinyl-, 3,4-Dihydro-chinazolinyl-, 1*H*-Benzo[*d*][1,2]oxazinyl-, 4*H-*Benzo[*e*][1,3]oxazinyl-, 4*H*-Benzo[*d*][1,3]oxazinyl- oder 2*H*-Benzo[1,4]oxazinylgruppe, in der jeweils
      im Benzoteil eine bis drei Methingruppen jeweils durch ein Stickstoffatom ersetzt sein können und im Heterocyclylteil eine Methylengruppe durch eine Carbonylgruppe ersetzt sein kann,
   eine 4*H*-Benzo[*e*][1,3]thiazinyl-, 4*H*-Benzo[*d*][1,3]thiazinyl- oder 2*H*-Benzo [1,4]thiazinylgruppe, in der jeweils
      im Benzoteil eine bis drei Methingruppen jeweils durch ein Stickstoffatom ersetzt sein können und im Heterocyclylteil eine Methylengruppe durch eine Carbonylgruppe ersetzt sein kann sowie das Schwefelatom durch eine Sulfinyl- oder Sulfonylgruppe ersetzt sein kann,
   eine 2-Oxo-2*H-*benzo[*e*][1,3]oxazinyl- oder 2,2-Dioxo-1*H*-benzo[*c*][1,2]thiazinylgruppe, in der jeweils im Benzoteil
      eine bis drei Methingruppen jeweils durch ein Stickstoffatom ersetzt sein können,
   eine 2,3-Dihydro-1*H*-benzo[*e*][1,4]diazepinyl-, 4,5-Dihydro-3*H*-benzo[*b*][1,4]diazepinyl- oder 5-Oxo-4,5-dihydro-3*H*-benzo[*e*][1,4]diazepinylgruppe, in der jeweils
      im Benzoteil eine bis drei Methingruppen jeweils durch ein Stickstoffatom ersetzt sein können und im Heterocyclylteil eine Methylengruppe durch eine Carbonylgruppe ersetzt sein kann,
   eine 2,3-Dihydro-benzo[*f*][1,4]oxazepinyl- oder 2,3-Dihydro-benzo[*b*][1,4]-oxazepinylgruppe, in der jeweils
      im Benzoteil eine bis drei Methingruppen jeweils durch ein Stickstoffatom ersetzt sein können und im Heterocyclylteil eine Methylengruppe durch eine Carbonylgruppe ersetzt sein kann,
   eine 2,3-Dihydro-benzo[*b*][1,4]thiazepinyl- oder 2,3-Dihydro-benzo[*f*][1,4]-thiazepinylgruppe, in der jeweils
      im Benzoteil eine bis drei Methingruppen jeweils durch ein Stickstoffatom ersetzt sein können und im Heterocyclylteil eine Methylengruppe durch eine Carbonylgruppe ersetzt sein kann und das Schwefelatom durch eine Sulfinyl- oder Sulfonylgruppe ersetzt sein kann,
   eine 5-Oxo-4,5-dihydro-benzo[*f*][1,3,4]oxadiazepinylgruppe, in der
      im Benzoteil eine bis drei Methingruppen jeweils durch ein Stickstoffatom ersetzt sein können,
   eine 11*H*-Dibenzo[*b,e*]azepinyl- oder 5*H*-Dibenzo[*a,d*]cycloheptenylgruppe, in der jeweils
      im Benzoteil eine bis drei Methingruppen jeweils durch ein Stickstoffatom ersetzt sein können und die Methylengruppe im Heterocyclylteil durch ein Sauerstoff- oder Schwefelatom, eine Carbonyl-, Sulfinyl- oder Sulfonylgruppe oder durch eine durch Rₓ substituierte Iminogruppe ersetzt sein kann, wobei
         Rₓ ein Wasserstoffatom oder eine C₁₋₄-Alkyl-, C₂₋₄-Alkenyl-, C₂₋₄-Alkinyl-, C₃₋₆-Cycloalkyl-, C₃₋₆-Cycloalkyl-C₁₋₃-alkyl-, Aryl-, Aryl-C₁₋₃-alkyl-, Hydroxy-C₂₋₄-alkyl-, C₁₋₃-Alkyloxy-C₂₋₄-alkyl-, C₃₋₆-Cycloalkyloxy-C₂₋₄-alkyl-, Amino-C₂₋₄-alkyl-, C₁₋₃-Alkylamino-C₂₋₄-alkyl, Di-(C₁₋₃-alkyl)-amino-C₂₋₄-alkyl-, C₁₋₃-Alkyl-carbonyl-, C₁₋₃-Alkyloxy-carbonyl-, C₁₋₃-Alkyloxy-carbonyl-C₁₋₃-alkyl-, Aryl-carbonyl-, C₁₋₃-Alkyl-sulfonyl- oder Aryl-sulfonylgruppe darstellt,
   eine Phenanthridinylgruppe, in der
      im Benzoteil eine bis drei Methingruppen jeweils durch ein Stickstoff atom ersetzt sein können, und
   eine 1,2,3,4-Tetrahydro-phenanthridinyl-, 1,2,3,4,4a,10b-Hexahydro-phenanthridinyl-, 2,3-Dihydro-1*H*-4-aza-cyclopenta[a]naphthyl- oder eine 8,9,10,11-Tetrahydro-7*H*-6-aza-cyclohepta[*a*]naphthylgruppe, in der jeweils
      im Benzoteil eine bis drei Methingruppen jeweils durch ein Stickstoffatom ersetzt sein können und ein oder zwei Methylengruppen jeweils durch ein Sauerstoffatom oder eine Carbonylgruppe ersetzt sein können, wobei, falls zwei Methylengruppen jeweils durch ein Sauerstoffatom ersetzt sind, die Sauerstoffatome durch mindestens zwei Methyleneinheiten voneinander getrennt sein müssen,
   eine Phenanthrenylgruppe, in der
      jeweils eine bis drei der Methingruppen in Position 1 bis 4 und 5 bis 8 durch jeweils ein Stickstoffatom ersetzt sein können,
   eine 1,2,3,4-Tetrahydro-phenanthrenyl- oder eine 1,2,3,4,5,6,7,8-Octahydrophenanthrenylgruppe, in der
      jeweils ein oder zwei der Methylengruppen in Position 1 bis 4 und 5 bis 8 durch jeweils ein Sauerstoffatom oder eine Carbonylgruppe ersetzt sein können, wobei, falls zwei Methylengruppen durch jeweils ein Sauerstoffatom ersetzt sind, die Sauerstoffatome durch mindestens zwei Methyleneinheiten voneinander getrennt sein müssen,
   eine 5*H*-Benzo[*e*]pyrrolo[1,2-*a*][1,4]diazepinyl-, Thieno[3,2-*b*][1,4]benzoxazepinyl-, 5*H*-Dibenzo[*d*,*f*][1,3]diazepinyl- oder eine 5-Oxa-7-aza-dibenzo[*a,c*]-cycloheptenylgruppe, in der jeweils
      im Benzoteil eine bis drei Methingruppen durch jeweils ein Stickstoffatom ersetzt sein können,
   eine Naphtho[1,2-*d*]oxazolyl-, Naphtho[2,1-*d*]oxazolyl -, Naphtho[1,2-*d*]thiazolyl-, Naphtho[2,1-*d*]thiazolyl-, Naphtho[1,2-*d*]imidazolyl-, Naphtho[1,2-*b*]furanyl-oder Naphtho[2,1-b]furanylgruppe, in der jeweils
      im Naphthylteil eine bis drei Methingruppen durch jeweils ein Stickstoffatom ersetzt sein können,
   oder eine Furo[3,2-*c*]isochinolinyl-, Pyrazolo[1,5-*c*]chinazolinyl- oder 1*H-*Perimidinylgruppe bedeutet,
   wobei die Methylen- und Methingruppen der vorstehend erwähnten Reste Rₐ durch die Gruppen R¹⁰ bis R¹³ und zusätzlich durch eine C₁₋₃-Alkylgruppe substituiert sein können und die Iminogruppen der vorstehend erwähnten Reste Rₐ durch die wie vorstehend definierten Reste Rₓ substituiert sein können und
      R¹⁰ ein Wasserstoffatom,
      ein Fluor-, Chlor-, Brom- oder Iodatom,
      eine C₁₋₄-Alkyl-, Hydroxy-, oder C₁₋₄-Alkyloxygruppe,
      eine Nitro-, Amino-, C₁₋₃-Alkylamino-, Di-(C₁₋₃-alkyl)amino-, Cyan-C₁₋₃-alkylamino-, N-(Cyan-C₁₋₃-alkyl)-N-(C₁₋₃-alkyl)amino-, C₁₋₃-Alkyloxycarbonyl-C₁₋₃-alkylamino-, Pyrrolidin-1-yl-, Piperidin-1-yl-, Morpholin-4-yl-, Piperazin-1-yl-, oder 4-(C₁₋₃-Alkyl)-piperazin-1-yl-Gruppe,
      eine C₁₋₃-Alkyl-carbonylamino-, Arylcarbonylamino-, Aryl-C₁₋₃-alkylcarbonylamino-, C₁₋₃-Alkyloxy-carbonylamino-, Aminocarbonylamino-, C₁₋₃-Alkylaminocarbonylamino-, Di-(C₁₋₃-alkyl)aminocarbonylamino-, Pyrrolidin-1-yl-carbonylamino-, Piperidin-1-yl-carbonylamino-, Morpholin-4-yl-carbonylamino-, Piperazin-1-yl-carbonylamino- oder 4-(C₁₋₃-Alkyl)-piperazin-1-yl-carbonylamino-, C₁₋₃-Alkyl-sulfonylamino-, Bis-(C₁₋₃-alkylsulfonyl)-amino-, Aminosulfonylamino-, C₁₋₃-Alkylamino-sulfonylamino-, Di-(C₁₋₃-alkyl)amino-sulfonylamino-, Pyrrolidin-1-yl-sulfonylamino-, Piperidin-1-yl-sulfonylamino-, Morpholin-4-yl-sulfonylamino-, Piperazin-1-yl-sulfonylamino- oder 4-(C₁₋₃-Alkyl)-piperazin-1-yl-sulfonylamino-, (C₁₋₃-Alkylamino)thiocarbonylamino-, (C₁₋₃-Alkyloxy-carbonylamino)carbonylamino-, Arylsulfonylamino- oder Aryl-C₁₋₃-alkyl-sulfonylaminogruppe,
      eine N-(C₁₋₃-Alkyl)-C₁₋₃-alkyl-carbonylamino-, N-(C₁₋₃-Alkyl)-arylcarbonylamino-, N-(C₁₋₃-Alkyl)-aryl-C₁₋₃-alkyl-carbonylamino-, N-(C₁₋₃-Alkyl)-C₁₋₃-alkyloxy-carbonylamino-, N-(Aminocarbonyl)-C₁₋₃-alkylamino-, N-(C₁₋₃-Alkyl-aminocarbonyl)-C₁₋₃-alkylamino -, N-[Di-(C₁₋₃-alkyl)aminocarbonyl]-C₁₋₃-alkylamino-, N-(C₁₋₃-Alkyl)-C₁₋₃-alkyl-sulfonylamino-, N-(C₁₋₃-Alkyl)-arylsulfonylamino-, oder N-(C₁₋₃-Alkyl)-aryl-C₁₋₃-alkyl-sulfonylaminogruppe,
      eine 2-Oxo-imidazolidin-1-yl-, 2,4-Dioxo-imidazolidin-1-yl-, 2,5-Dioxoimidazolidin-1-yl- oder 2-Oxo-hexahydropyrimidin-1-ylgruppe, in der das Stickstoffatom in 3-Stellung jeweils durch eine Methyl- oder Ethylgruppe substituiert sein kann,
      eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-, Piperazin-1-yl-carbonyl- oder 4-(C₁₋₃-Alkyl)-piperazin-1-yl-carbonylgruppe,
      eine C₁₋₃-Alkyl-carbonyl- oder eine Arylcarbonylgruppe,
      eine Carboxy-C₁₋₃-alkyl-, C₁₋₃-Alkyloxy-carbonyl-C₁₋₃-alkyl-, Cyan-C₁₋₃-alkyl-, Aminocarbonyl-C₁₋₃-alkyl-, C₁₋₃-Alkyl-aminocarbonyl-C₁₋₃-alkyl-, Di-(C₁₋₃-alkyl)-aminocarbonyl-C₁₋₃-alkyl-, Pyrrolidin-1-yl-carbonyl-C₁₋₃-alkyl-, Piperidin-1-yl-carbonyl-C₁₋₃-alkyl-, Morpholin-4-yl-carbonyl-C₁₋₃-alkyl-, Piperazin-1 -yl-carbonyl-C₁₋₃-alkyl- oder 4-(C₁₋₃-Alkyl)-piperazin-1-yl-carbonyl-C₁₋₃-alkylgruppe,
      eine Carboxy-C₁₋₃-alkyloxy-, C₁₋₃-Alkyloxy-carbonyl-C₁₋₃-alkyloxy-, Cyan-C₁₋₃-alkyloxy-, Aminocarbonyl-C₁₋₃-alkyloxy-, C₁₋₃-Alkyl-aminocarbonyl-C₁₋₃-alkyloxy-, Di-(C₁₋₃-alkyl)-aminocarbonyl-C₁₋₃-alkyloxy-, Pyrrolidin-1-yl-carbonyl-C₁₋₃-alkyloxy-, Piperidin-1-yl-carbonyl-C₁₋₃-alkyloxy-, Morpholin-4-yl-carbonyl-C₁₋₃-alkyloxy-, Piperazin-1-yl-carbonyl-C₁₋₃-alkyloxy- oder 4-(C₁₋₃-Alkyl)-piperazin-1-yl-carbonyl-C₁₋₃-alkyloxygruppe,
      eine Hydroxy-C₁₋₃-alkyl-, C₁₋₃-Alkyloxy-C₁₋₃-alkyl-, Amino-C₁₋₃-alkyl-, C₁₋₃-Alkylamino-C₁₋₃-alkyl-, Di-(C₁₋₃-alkyl)-amino-C₁₋₃-alkyl-, Pyrrolidin-1-yl-C₁₋₃-alkyl-, Piperidin-1-yl-C₁₋₃-alkyl-, Morpholin-4-yl-C₁₋₃-alkyl-, Piperazin-1 -yl-C₁₋₃-alkyl- oder 4-(C₁₋₃-Alkyl)-piperazin-1-yl-C₁₋₃-alkylgruppe,
      eine Hydroxy-C₁₋₃-alkyloxy-, C₁₋₃-Alkyloxy-C₁₋₃-alkyloxy-, C₁₋₃-Alkylsulfanyl- C₁₋₃-alkyloxy-, C₁₋₃-Alkylsulfinyl-C₁₋₃-alkyloxy-, C₁₋₃-Alkylsulfonyl-C₁₋₃-alkyloxy-, Amino-C₁₋₃-alkyloxy-, C₁₋₃-Alkylamino-C₁₋₃-alkyloxy-, Di-(C₁₋₃-alkyl)-amino-C₁₋₃-alkyloxy-, Pyrrolidin-1-yl-C₁₋₃-alkyloxy-, Piperidin-1-yl-C₁₋₃-alkyloxy-, Morpholin-4-yl-C₁₋₃-alkyloxy-, Piperazin-1 -yl-C₁₋₃-alkyloxy- oder 4-(C₁₋₃-Alkyl)-piperazin-1-yl-C₁₋₃-alkyloxygruppe,
      eine Mercapto-, C₁₋₃-Alkylsulfanyl-, C₁₋₃-Alkysulfinyl-, C₁₋₃-Alkylsulfonyl-, C₁₋₃-Alkylsulfonyloxy-, Arylsulfonyloxy-, Trifluormethylsulfanyl-, Trifluor methylsulfinyl- oder Trifluormethylsulfonylgruppe,
      eine Sulfo-, Aminosulfonyl-, C₁₋₃-Alkyl-aminosulfonyl-, Di-(C₁₋₃-alkyl)-aminosulfonyl-, Pyrrolidin-1-yl-sulfonyl-, Piperidin-1-yl-sulfonyl-, Morpholin-4-yl-sulfonyl-, Piperazin-1-yl-sulfonyl- oder 4-(C₁₋₃-Alkyl)-piperazin-1-yl-sulfonylgruppe,
      eine durch 1 bis 3 Fluoratome substituierte Methyl- oder Methoxygruppe,
      eine durch 1 bis 5 Fluoratome substituierte Ethyl- oder Ethoxygruppe,
      eine C₂₋₄-Alkenyl- oder C₂₋₄-Alkinylgruppe,
      eine C₃₋₄-Alkenyloxy- oder C₃₋₄-Alkinyloxygruppe,
      eine C₃₋₆-Cycloalkyl- oder C₃₋₆-Cycloalkyloxygruppe,
      eine C₃₋₆-Cycloalkyl-C₁₋₃-alkyl- oder C₃₋₆-Cycloalkyl-C₁₋₃-alkyloxygruppe oder
      eine Aryl-, Aryloxy-, Aryl-C₁₋₃-alkyl- oder Aryl-C₁₋₃-alkyloxygruppe,
      R¹¹ und R¹², die gleich oder verschieden sein können, jeweils ein Wasserstoffatom, ein Fluor-, Chlor-, Brom- oder Iodatom, eine C₁₋₃-Alkyl-, Trifluormethyl-, Hydroxy-, C₁₋₃-Alkyloxy- oder Cyangruppe, oder
      R¹¹ zusammen mit R¹², sofern diese an benachbarte Kohlenstoffatome gebunden sind, auch eine Methylendioxy-, Difluormethylendioxy-, Ethylendioxy- oder eine geradkettige C₃₋₅-Alkylengruppe und
      R¹³ ein Wasserstoffatom, ein Fluor-, Chlor oder Bromatom, eine Trifluormethyl-, C₁₋₃-Alkyl- oder C₁₋₃-Alkyloxygruppe bedeuten,
R² ein Wasserstoff-, Fluor- oder Chloratom,
eine C₁₋₆-Alkylgruppe,
eine C₂₋₄-Alkenylgruppe,
eine C₃₋₄-Alkinylgruppe,
eine C₃₋₆-Cycloalkylgruppe,
eine C₃₋₆-Cycloalkyl-C₁₋₃-alkylgruppe,
eine Tetrahydrofuran-3-yl-, Tetrahydropyran-3-yl-, Tetrahydropyran-4-yl-, Tetrahydrofuranylmethyl- oder Tetrahydropyranylmethylgruppe,
eine Arylgruppe,
eine Aryl-C₁₋₄-alkylgruppe,
eine Aryl-C₂₋₃-alkenylgruppe,
eine Arylcarbonylgruppe,
eine Arylcarbonyl-C₁₋₂-alkylgruppe,
eine Heteroarylgruppe,
eine Heteroaryl-C₁₋₃-alkylgruppe,
eine Furanylcarbonyl-, Thienylcarbonyl-, Thiazolylbarbonyl- oder Pyridylcarbonylgruppe,
eine Furanylcarbonylmethyl-, Thienylcarbonylmethyl-, Thiazolylcarbonylmethyl- oder Pyridylcarbonylmethylgruppe,
eine C₁₋₄-Alkyl-carbonyl-Gruppe,
eine C₁₋₄-Alkyl-carbonyl-C₁₋₂-alkyl-Gruppe,
eine C₃₋₆-Cycloalkyl-carbonyl-Gruppe,
eine C₃₋₆-Cycloalkyl-carbonyl-C₁₋₂-alkyl-Gruppe,
eine Aryl-A- oder Aryl-A-C₁₋₃-alkyl-gruppe, wobei A ein Sauerstoff- oder Schwefelatom, eine Imino-, C₁₋₃-Alkylimino-, Sulfinyl- oder Sulfonylgruppe bedeutet,
eine Gruppe R_{b}, wobei
   R_{b} eine Cyano-, Carboxy-, C₁₋₃-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl-, Morpholin-4-ylcarbonyl-, Piperazin-1-ylcarbonyl-, 4-Methylpiperazin-1-ylcarbonyl-, 4-Ethylpiperazin-1-ylcarbonyl-, Hydroxy-, Mercapto-,. C₁₋₃-Alkyloxy-, C₁₋₃-Alkylsulfenyl-, C₁₋₃-Alkylsulfinyl-, C₁₋₃-Alkylsulfonyl-, Amino-, C₁₋₃-Alkylamino-, Di-(C₁₋₃-alkyl)-amino-, Pyrrolidin-1-yl-, Piperidin-1-yl-, Morpholin-4-yl-, Piperazin-1-yl-, 4-Methyl-piperazin-1-yl- oder 4-Ethyl-piperazin-1-yl-gruppe bedeutet,
oder eine durch eine Gruppe R_{b} substituierte C₁₋₄-Alkylgruppe, wobei R_{b} wie oben erwähnt definiert ist,
Y ein Stickstoffatom oder eine Gruppe der Formel C-R⁵,
   wobei R⁵ wie R² definiert ist und jeweils einer der beiden Reste R² und R⁵ ein Wasserstoffatom oder ein C₁₋₃-Alkylgruppe sein muss,
R³ eine C₃₋₈-Alkylgruppe,
eine durch eine Gruppe R_{c} substituierte C₁₋₃-Alkylgruppe, wobei
   R_{c} eine gegebenenfalls durch eine oder zwei C₁₋₃-Alkylgruppen substituierte C₃₋₇-Cycloalkylgruppe,
   eine gegebenenfalls durch eine oder zwei C₁₋₃-Alkylgruppen substituierte C₅₋₇-Cycloalkenylgruppe,
   eine Arylgruppe oder
   eine Furanyl-, Thienyl-, Oxazolyl-, Isoxazolyl-, Thiazolyl-, Isothiazolyl-, Pyridyl-, Pyridazinyl-, Pyrimidyl- oder Pyrazinylgruppe bedeutet, wobei die vorstehend erwähnten heterocyclischen Reste jeweils durch eine oder zwei C₁₋₃-Alkylgruppen oder durch ein Fluor-, Chlor-, Brom- oder Iodatom oder durch eine Trifluormethyl-, Cyan- oder C₁₋₃-Alkyloxygruppe substituiert sein können,
eine C₃₋₈-Alkenylgruppe,
eine durch ein Fluor , Chlor- oder Bromatom oder durch eine Trifluormethylgruppe substituierte C₃₋₆-Alkenylgruppe,
eine C₃₋₈-Alkinylgruppe,
eine Arylgruppe oder
eine Aryl-C₂₋₄-alkenylgruppe,
und
R⁴ eine Azetidin-1-yl- oder Pyrrolidin-1-ylgruppe, die in 3-Stellung durch eine Amino-, C₁₋₃-Alkylamino- oder eine Di-(C₁₋₃-alkyl)amino-Gruppe substituiert ist und zusätzlich durch eine oder zwei C₁₋₃-Alkylgruppen substituiert sein kann,
eine Piperidin-1-yl- oder Hexahydroazepin-1-ylgruppe, die in 3-Stellung oder in 4-Stellung durch eine Amino-, C₁₋₃-Alkylamino- oder eine Di-(C₁₋₃-alkyl)amino-Gruppe. substituiert ist und zusätzlich durch eine oder zwei C₁₋₃-Alkylgruppen substituiert sein kann,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyanthiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-aminogruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1yl-, Piperidin-1-yl- oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine gegebenenfalls am Kohlenstoffgerüst durch eine oder zwei C₁₋₃-Alkylgruppen substituierte Piperazin-1-yl- oder [1,4]Diazepan-1-ylgruppe,
eine gegebenenfalls am Kohlenstoffgerüst durch eine oder zwei C₁₋₃-Alkylgruppen substituierte 3-Imino-piperazin-1-yl-, 3-Imino-[1,4]diazepan-1-yl- oder 5-Imino-[1,4]diazepan-1-ylgruppe,
eine gegebenenfalls durch eine oder zwei C₁₋₃-Alkylgruppen substituierte [1,4]Diazepan-1-ylgruppe, die in 6-Stellung durch eine Aminogruppe substituiert ist,
eine C₃₋₇-Cycloalkylgruppe, die durch eine Amino-, C₁₋₃-Alkylamino- oder Di-(C₁₋₃-alkyl)-aminogruppe substituiert ist,
eine C₃₋₇-Cycloalkylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkylgruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkylgruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkylaminogruppe, in der der Cycloalkylteil durch eine Amino-, C₁₋₃-Alkylamino- oder Di-(C₁₋₃alkyl)-aminogruppe substituiert ist, wobei die beiden Stickstoffatome am Cycloalkylteil durch mindestens zwei Kohlenstoffatome voneinander getrennt sind,
eine N-(C₃₋₇-Cycloalkyl)-N-(C₁₋₃-alkyl)-aminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine C₃₋₇-Cycloalkylaminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl)-N-(C₁₋₃-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkyl-aminogruppe, in der der Cycloalkylteil durch eine Amino-, C₁₋₃-Alkylamino- oder Di-(C₁₋₃-alkyl)-aminogruppe substituiert ist,
eine N-(C₃₋₇-Cycloalkyl-C₁₋₂-alkyl)-N-(C₁₋₂-alkyl)-aminogruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkyl-aminogruppe, in der der Cycloalkylteil durch eine Amino-C₁₋₃-alkyl-, C₁₋₃-Alkylamino-C₁₋₃-alkyl- oder eine D₁-(C₁₋₃-alkyl)amino-C₁₋₃-alkylgruppe substituiert ist,
eine N-(C₃₋₇-Cycloalkyl-C₁₋₂-alkyl)-N-(C₁₋₂-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine R¹⁹-C₂₋₄-Alkylamino-Gruppe, in der R¹⁹ durch mindestens zwei Kohlenstoffatome vom Stickstoffatom des C₂₋₄-Alkylamino-Teils getrennt ist und
   R¹⁹ eine Amino-, C₁₋₃-Alkylamino- oder Di-(C₁₋₃-alkyl)-aminogruppe darstellt,
eine R¹⁹-C₂₋₄-Alkylamino-Gruppe, in der das Stickstoffatom des C₂₋₄-Alkylamino-Teils durch eine C₁₋₃-Alkylgruppe substituiert ist und R¹⁹ durch mindestens zwei Kohlenstoffatome vom Stickstoffatom des C₂₋₄-Alkylamino- Teils getrennt ist, wobei R¹⁹ wie vorstehend erwähnt definiert ist,
eine durch den Rest R²⁰ substituierte Aminogruppe, in der
   R²⁰ eine Azetidin-3-yl, Azetidin-2-ylmethyl-, Azetidin-3-ylmethyl-, Pyrrolidin-3-yl-, Pyrrolidin-2-ylmethyl-, Pyrrolidin-3-ylmethyl-, Piperidin-3-yl-, Piperidin-4-yl-, Piperidin-2-ylmethyl-, Piperidin-3-ylmethyl- oder Piperidin-4-ylmethylgruppe darstellt, wobei die für R²⁰ erwähnten Reste jeweils durch eine oder zwei C₁₋₃-Alkylgruppen substituiert sein können,
eine durch den Rest R²⁰ und eine C₁₋₃-Alkylgruppe substituierte Aminogruppe, in der R²⁰ wie vorstehend erwähnt definiert ist, wobei die für R²⁰ erwähnten Reste jeweils durch eine oder zwei C₁₋₃-Alkylgruppen substituiert sein können,
eine R¹⁹-C₃₋₄-alkyl-gruppe, in der der C₃₋₄-Alkylteil geradkettig ist und zusätzlich durch eine oder zwei C₁₋₃-Alkylgruppen substituiert sein kann, wobei R¹⁹ wie vorstehend erwähnt definiert ist,
eine 3-Amino-2-oxo-piperidin-5-yl- oder 3-Amino-2-oxo-1-methyl-piperidin-5-ylGruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino-oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piperidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können,
wobei unter den bei der Definition der vorstehend genannten Reste erwähnten Arylgruppen Phenyl- oder Naphthylgruppen zu verstehen sind, welche durch Rₕ mono-oder disubstituiert sein können, wobei die Substituenten gleich oder verschieden sein können und Rₕ ein Fluor-, Chlor-, Brom- oder Iodatom, eine Trifluormethyl-, Cyan-, Nitro-, Amino-, Aminocarbonyl-, Aminosulfonyl-, Methylsulfonyl, Acetylamino-, Methylsulfonylamino-, C₁₋₃-Alkyl-, Cyclopropyl-, Ethenyl-, Ethinyl-, Hydroxy-, **C₁₋₃-**Alkyloxy-, Difluormethoxy- oder Trifluormethoxygruppe darstellt,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-,Chinolinyl- oder lsochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
   und die vorstehend erwähnten Heteroarylgruppen durch Rₕ mono- oder disubstituiert sein können, wobei die Substituenten gleich oder verschieden sein können und Rₕ wie vorstehend erwähnt definiert ist,
wobei, soweit nichts anderes erwähnt wurde, die vorstehend erwähnten Alkyl-, Alkenyl- und Alkinylgruppen geradkettig oder verzweigt sein können,
und wobei die Wasserstoffatome der in den Definitionen enthaltenen Methyl- oder Ethylgruppen ganz oder teilweise durch Fluoratome ersetzt sein können,
deren Tautomere, Enantiomere, Diastereomere, deren Gemische, deren Prodrugs und deren Salze.

Verbindungen der obigen allgemeinen Formel I, die einen oder mehrere in-vivo abspaltbare Reste enthalten, stellen sogenannte Prodrugs dar.

Die bei der Definition der vorstehend erwähnten Reste erwähnten Carboxygruppen können durch eine in-vivo in eine Carboxygruppe überführbare Gruppe oder durch eine unter physiologischen Bedingungen negativ geladene Gruppe ersetzt sein,

Des weiteren können die bei der Definition der vorstehend erwähnten Reste erwähnten Amino- und Iminogruppen durch einen in-vivo abspaltbaren Rest substituiert sein.

Derartige Gruppen werden beispielsweise in der WO 98/46576 und von N.M. Nielsen et al. in International Journal of Pharmaceutics 39, 75-85 (1987) beschrieben.

Unter einer in-vivo in eine Carboxygruppe überführbare Gruppe ist beispielsweise eine Hydroxymethylgruppe, eine mit einem Alkohol veresterte Carboxygruppe, in der der alkoholische Teil vorzugsweise ein C₁₋₆-Alkanol, ein Phenyl-C₁₋₃-alkanol, ein C₃₋₉-Cycloalkanol, wobei ein C₅₋₈Cycloalkanol zusätzlich durch ein oder zweiC₁₋₃-Alkylgruppen substituiert sein kann, ein C₅₋₈-Cycloalkanol, in dem eine Methylengruppe in 3- oder 4-Stellung durch ein Sauerstoffatom oder durch eine gegebenenfalls durch eine C₁₋₃-Alkyl-, Phenyl-C₁₋₃-alkyl-, Phenyl-C₁₋₃-alkyloxycarbonyl- oder C₂₋₆-Alkanoylgruppe substituierte Iminogruppe ersetzt ist und der Cycloalkanolteil zusätzlich durch ein oder zwei C₁₋₃-Alkylgruppen substituiert sein kann, ein C₄₋₇-Cycloalkenol, ein C₃₋₅-Alkenol, ein Phenyl-C₃₋₅-alkenol, ein C₃₋₅-Alkinol oder Phenyl-C₃₋₅-alkinol mit der Maßgabe, daß keine Bindung an das Sauerstoffatom von einem Kohlenstoffatom ausgeht, welches eine Doppel- oder Dreifachbindung trägt, ein C₃₋₈-Cycloalkyl-C₁₋₃-alkanol, ein Bicycloalkanol mit insgesamt 8 bis 10 Kohlenstoffatomen, das im Bicycloalkylteil zusätzlich durch eine oder zwei C₁₋₃-Alkylgruppen substituiert sein kann, ein 1,3-Dihydro-3-oxo-1-isobenzfuranol oder ein Alkohol der Formel

Rₚ-CO-O-(R_{q}CRᵣ)-OH,

in dem
Rₚ eine C₁₋₈-Alkyl-, C₅₋₇-Cycloalkyl-, C₁₋₈-Alkyloxy-, C₅₋₇-Cycloalkyloxy-, Phenyl- oder Phenyl- C₁₋₃-alkylgruppe,
R_{q} ein Wasserstoffatom, eine C₁₋₃Alkyl-, C₅₋₇-Cycloalkyl- oder Phenylgruppe und
Rᵣ ein Wasserstoffatom oder eine C₁₋₃-Alkylgruppe darstellen,
unter einer unter physiologischen Bedingungen negativ geladenen Gruppe wie eine Tetrazol-5-yl-, Phenylcarbonylaminocarbonyl-, Trifluormethylcarbonylaminocarbonyl-, C₁₋₆-Alkylsulfonylamino-, Phenylsulfonylamino-, Benzylsulfonylamino-, Trifluormethylsulfonylamino-, C₁₋₆-Alkylsulfonylaminocarbonyl-, Phenylsulfonylaminocarbonyl-, Benzylsulfonylaminocarbonyl- oder Perfluor-C₁₋₆-alkylsulfonylaminocarbonylgruppe
und unter einem von einer Imino- oder Aminogruppe in-vivo abspaltbaren Rest beispielsweise eine Hydroxygruppe, eine Acylgruppe wie eine gegebenenfalls durch Fluor-, Chlor-, Brom- oder Jodatome, durch C₁₋₃-Alkyl- oder C₁₋₃-Alkyloxygruppen mono- oder disubstituierte Phenylcarbonylgruppe, wobei die Substituenten gleich oder verschieden sein können, eine Pyridinoylgruppe oder eine C₁₋₁₆-Alkanoylgruppe wie die Formyl-, Acetyl-, Propionyl-, Butanoyl-, Pentanoyl- oder Hexanoylgruppe, eine 3,3,3-Trichlorpropionyl- oder Allyloxycarbonylgruppe, eine C₁₋₁₆-Alkyloxycarbonyl- oder C₁₋₁₆₋Alkylcarbonyloxygruppe, in denen Wasserstoffatome ganz oder teilweise durch Fluor- oder Chloratome ersetzt sein können, wie die Methoxycarbonyl-, Ethoxycarbonyl-, Propoxycarbonyl-, Isopropoxycarbonyl-, Butoxycarbonyl-, tert.-Butoxycarbonyl-, Pentoxycarbonyl-, Hexoxycarbonyl-, Octyloxycarbonyl-, Nonyloxycarbonyl-, Decyloxycarbonyl-, Undecyloxycarbonyl-, Dodecyloxycarbonyl-, Hexadecyloxycarbonyl-, Methylcarbonyloxy-, Ethylcarbonyloxy-, 2,2,2-Trichlorethylcarbonyloxy-, Propylcarbonyloxy-, Isopropylcarbonyloxy-, Butylcarbonyloxy-, tert.Butylcarbonyloxy-, Pentylcarbonyloxy-, Hexylcarbonyloxy-, Octylcarbonyloxy-, Nonylcarbonyloxy-, Decylcarbonyloxy-, Undecylcarbonyloxy-, Dodecylcarbonyloxy-oder Hexadecylcarbonyloxygruppe, eine Phenyl-C₁₋₆-alkyloxycarbonylgruppe wie die Benzyloxycarbonyl-, Phenylethoxycarbonyl- oder Phenylpropoxycarbonylgruppe, eine 3-Amino-propionylgruppe, in der die Aminogruppe durch C₁₋₆-Alkyl- oder C₃₋₇-Cycloalkylgruppen mono- oder disubstituiert und die Substituenten gleich oder verschieden sein können, eine C₁₋₃-Alkylsulfonyl-C₂₋₄-alkyloxycarbonyl-, C₁₋₃-Alkyloxy-C₂₋₄-alkyloxy-C₂₋₄-alkyloxycarbonyl-, Rₚ-CO-O-(R_{q}CRᵣ)-O-CO-, C₁₋₆-Alkyl-CO-NH-(RₛCRₜ)-O-CO- oder C₁₋₆-Alkyl-CO-O-(RₛCRₜ)-(RₛCRₜ)-O-CO-Gruppe, in denen Rₚ bis Rᵣ wie vorstehend erwähnt definiert sind,
   Rₛ und Rₜ, die gleich oder verschieden sein können, Wasserstoffatome oder C₁₋₃-Alkylgruppen darstellen,
zu verstehen.

Des Weiteren schließen die in den vor- und nachstehenden Definitionen erwähnten gesättigten Alkyl- und Alkyloxyteile, die mehr als 2 Kohlenstoffatome enthalten, soweit nichts Anderes erwähnt würde, auch deren verzweigte Isomere wie beispielsweise die Isopropyl-, tert.Butyl-, lsobutylgruppe etc. ein.

Bevorzugt sind diejenigen Verbindungen der obigen allgemeinen Formel I, in denen
R¹ eine durch eine Gruppe Rₐ substituierte Methylgruppe, wobei
   Rₐ eine 3,4-Dihydro-chinolinylgruppe,
   eine 3,4-Dihydro-isochinolinylgruppe,
   eine 1,4-Dihydro-chinazolinyl- oder 4-Oxo-1,4-dihydro-chinazolinylgruppe,
   eine 3,4-Dihydro-chinazolinyl- oder 4-Oxo-3,4-dihydro-chinazolinylgruppe,
   eine 1*H*-Benzo[*d*][1,2]oxazinyl- oder 1-Oxo-1*H*-benzo[*d*][1,2]oxazinylgruppe,
   eine 4*H*-Benzo[*e*][1,3]oxazinyl- oder 4-Oxo-4H-benzo[*e*][1,3]oxazinylgruppe,
   eine 4*H*-Benzo[*d*][1,3]oxazinyl- oder 4-Oxo-4*H*-benzo[*d*][1,3]oxazinylgruppe,
   eine 2*H*-Benzo[1,4]oxazinyl- oder 2-Oxo-2*H*-benzo[1,4]oxazinylgruppe,
   eine 4*H*-Benzo[*e*][1,3]thiazinyl- oder 4-Oxo-4*H*-benzo[*e*][1,3]thiazinylgruppe,
   eine 4*H*-Benzo[*d*][1,3]thiazinyl- oder 2*H*-Benzo [1,4]thiazinylgruppe,
   eine 2-Oxo-2*H*-benzo[*e*][1,3]oxazinyl- oder 2,2-Dioxo-1*H*-benzo[*c*][1,2]thiazinylgruppe,
   eine 2,3-Dihydro-1*H*-benzo[*e*][1,4]diazepinyl- oder 2-Oxo-2,3-dihydro-1*H-*benzo[*e*][1,4]diazepinylgruppe,
   eine 4,5-Dihydro-3*H*-benzo[*b*][1,4]diazepinyl- oder 4-Oxo-4,5-dihydro-3*H* benzo[*b*][1,4]diazepinylgruppe,
   eine 5-Oxo-4,5-dihydro-3*H*-benzo[*e*][1,4]diazepinylgruppe,
   eine 2,3-Dihydro-benzo[*f*][1,4]oxazepinyl- oder 2,3-Dihydro-benzo[*b*][1,4]-oxazepinylgruppe,
   eine 2,3-Dihydro-benzo[*f*][1,4]thiazepinyl- oder 2,3-Dihydro-benzo[*b*][1,4]-thiazepinylgruppe,
   eine 5-Oxo-4,5-dihydro-benzo[*f*][1,3,4]oxadiazepinylgruppe,
   eine 11*H*-Dibenzo[*b*,*e*]azepinyl- oder 11-Oxo-11*H*-dibenzo[*b*,*e*]azepinylgruppe,
   eine 11*H*-Benzo[*e*]pyrido[3,2-*b*]azepinyl- oder eine 5*H*-1,9,10-Triaza-dibenzo-[a,d]cycloheptenylgruppe,
   eine 5*H*-Dibenzo[*b*,*e*][1,4]diazepinyl- oder Dibenzo[*b*,*f*][1,4]oxazepinylgruppe,
   eine Dibenzo[*b*,*f*][1,4]thiazepinyl-, 5-Oxo-dibenzo[*b*,*f*][1,4]thiazepinyl- oder 5,5-Dioxo-dibenzo[*b*,*f*][1,4]thiazepinylgruppe,
   eine 5*H*-Dibenzo[*a*,*d*]cycloheptenyl- oder 5*H*-Dibenzo[*b*,*f*]azepinylgruppe,
   eine Phenanthridinyl-, Benzo[*c*][1,5]naphthyridinyl-, Benzo[*h*][1,6]naphthyridinyl-, Benzo[*c*][1,8]naphthyridinyl-, Benzo[*f*][1,7]naphthyridinyl-oder 1,5,9-Triaza-phenanthrenylgruppe,
   eine 1,2,3,4-Tetrahydro-phenanthridinyl-, 1,2,3,4,4a,10b-Hexahydro-phenanthridinyl-, 2,3-Dihydro-1*H*-4-aza-cyclopenta[a]naphthyl- oder 8,9,10,11-Tetrahydro-7*H*-6-aza-cyclohepta[*a*]naphthylgruppe,
   eine 2,3-Dihydro-1*H*-4-oxa-10-aza-phenanthrenyl- oder 1-Oxo-2,3-dihydro-1*H-*4-oxa-10-aza-phenanthrenylgruppe,
   eine Phenanthrenyl-, Benzo[*h*]chinolinyl-, Benzo[*f*]chinolinyl- oder Benzo[*f*]chinoxalinylgruppe,
   eine 5*H*-Benzo[*e*]pyrrolo[1,2-*a*][1,4]diazepinyl-, Thieno[3,2-*b*][1,4]benzoxazepinyl-, 5*H*-Dibenzo[*d,f*][1,3]diazepinyl- oder 5-Oxa-7-aza-dibenzo[*a*,*c*]-cycloheptenylgruppe,
   eine Naphtho[1,2-*d*]oxazolyl-, Naphtho[2,1-*d*]oxazolyl -, Naphtho[1,2-*d*]thiazolyl-, Naphtho[2,1-*d*]thiazolyl-, Naphtho[1,2-*d*]imidazolyl-, Naphtho[1,2-*b*]furanyl- oder Naphtho[2,1-*b*]furanylgruppe,
   oder eine Furo[3,2-*c*]isochinolinyl-, Pyrazolo[1,5-*c*]chinazolinyl- oder 1*H-*Perimidinylgruppe bedeutet,
   wobei die Benzogruppen der vorstehend erwähnten Reste Rₐ durch die Gruppen R¹⁰ bis R¹³ substituiert sind und die Alkyleneinheiten der vorstehend erwähnten Reste Rₐ durch ein oder zwei Fluoratome oder ein oder zwei C₁₋₃-Alkyl- oder C₁₋₃-Alkyloxy-carbonylgruppen substituiert sein können und die Iminogruppen der vorstehend erwähnten Reste Rₐ durch eine C₁₋₃-Alkylgruppe substituiert sein können und
      R¹⁰ ein Wasserstoffatom,
      ein Fluor , Chlor-, Brom- oder lodatom,
      eine C₁₋₃-Alkyl- oder Cyclopropylgruppe,
      eine Hydroxy-, C₁₋₃-Alkyloxy- oder Cyclopropyloxygruppe,
      eine Nitro-, Amino-, C₁₋₃-AlKylamino- oder Di-(C₁₋₃-alkyl)aminogruppe,
      eine C₁₋₃-Alkyl-carbonylamino- oder C₁₋₃-Alkyl-sulfonylaminogruppe,
      eine Cyan-, Carboxy-, C₁₋₃-Alkyloxy-carbonyl-, Aminocarbonyl-, C₁₋₃-Alkyl-aminocarbonyl- oder Di-(C₁₋₃-alkyl)-aminocarbonylgruppe,
      eine Mercapto-, C₁₋₃-Alkylsulfanyl-, C₁₋₃-Alkysulfinyl-, C₁₋₃-Alkylsulfonyl-oder Aminosulfonylgruppe oder
      eine Difluormethyl-, Trifluormethyl-, Difluormethoxy- oder Trifluormethoxygruppe und
      R¹¹, R¹² und R¹³, die gleich oder verschieden sein können, jeweils ein Wasserstoffatom, ein Fluor-, Chlor oder Bromatom, eine Methyl-, Trifluormethyl- oder Methoxygruppe bedeuten,
R² ein Wasserstoffatom oder
eine C₁₋₃-Alkyl-, Cyclopropyl-, Trifluormethyl-, Cyanomethyl- oder 2-Cyanoethylgruppe,
Y ein Stickstoffatom oder eine Gruppe der Formel C-R⁵,
   wobei R⁵ ein Wasserstoffatom oder eine C₁₋₃-Alkylgruppe bedeutet,
R³ eine 2-Buten-1-yl- oder 3-Methyl-2-buten-1-ylgruppe,
eine 1-Buten-1-ylgruppe,
eine 2-Butin-1-ylgruppe oder
eine 1-Cyclopenten-1-ylmethylgruppe
und
R⁴ eine (3-Amino-piperidin-1-yl)gruppe bedeuten,
wobei, soweit nichts anderes erwähnt wurde, die vorstehend erwähnten Alkylgruppen geradkettig oder verzweigt sein können,
deren Tautomere, Enantiomere, Diastereomere, deren Gemische und deren Salze.

Besonders bevorzugt sind diejenigen Verbindungen der obigen allgemeinen Formel I, in denen
R¹ eine durch eine Gruppe Rₐ substituierte Methylgruppe, wobei
   Rₐ eine 3,4-Dihydro-chinolin-2-ylgruppe,
   eine 3,4-Dihydro-isochinolin-1-ylgruppe,
   eine 1,4-Dihydro-chinazolin-2-yl- oder 4-Oxo-1,4-dihydro-chinazolin-2-ylgruppe,
   eine 3,4-Dihydro-chinazolin-2-yl- oder 4-Oxo-3,4-dihydro-chinazolin-2-yigruppe,
   eine 1*H* Benzo[*d*][1,2]oxazin-4-yl- oder 1 -Oxo-1*H* benzo[*d*][1,2]oxazin-4-ylgruppe,
   eine 4*H*-Benzo[*e*][1,3]oxazin-2-yl- oder 4-Oxo-4*H*-benzo[*e*][1,3]oxazin-2-ylgruppe,
   eine 4*H*-Benzo[*d*][1,3]oxazin-2-yl- oder 4-Oxo-4*H*-benzo[*d*][1,3]oxazin-2-ylgruppe,
   eine 2*H*-Benzo[1,4]oxazin-3-yl- oder 2-Oxo-2*H*-benzo[1,4]oxazin-3-ylgruppe,
   eine 4*H*-Benzo[*e*][1,3]thiazin-2-yl- oder 4-Oxo-4*H*-benzo[*e*][1,3]thiazin-2-ylgruppe,
   eine 4*H*-Benzo[*d*][1,3]thiazin-2-yl- oder 2*H*-Benzo[1,4]thiazin-3-ylgruppe,
   eine 2-Oxo-2*H*-benzo[*e*][1,3]oxazin-4-yl- oder 2,2-Dioxo-1*H*-benzo[*c*][1,2]thiazin-4-ylgruppe,
   eine 2,3-Dihydro-1*H*-benzo[*e*][1,4]diazepin-5-yl- oder 2-Oxo-2,3-dihydro-1*H-*benzo[*e*][1,4]diazepin-5-ylgruppe,
   eine 4,5-Dihydro-3*H* benzo[*b*][1,4]diazepin-2-yl- oder 4-Oxo-4,5-dihydro-3*H-*benzo[*b*][1,4]diazepin-2-ylgruppe,
   eine 5-Oxo-4,5-dihydro-3*H*-benzo[*e*][1,4]diazepin-2-ylgruppe,
   eine 2,3-Dihydro-benzo[*f*][1,4]oxazepin-5-yl- oder 2,3-Dihydro-benzo[*b*][1,4]-oxazepin-4-ylgruppe,
   eine 2,3-Dihydro-benzo[*f*][1,4]thiazepin-5-yl- oder 2,3-Dihydro-benzo[*b*][1,4]-thiazepin-4-ylgruppe,
   eine 5-Oxo-4,5-dihydro-benzo[*f*][1,3,4]oxadiazepin-2-ylgruppe,
   eine 11*H*-Dibenzo[*b,e*]azepin-6-yl- oder 11-Oxo-11*H*-dibenzo[*b,e*]azepin-6-ylgruppe,
   eine 11*H*-Benzo[*e*]pyrido[3,2-*b*]azepin-6-yl- oder eine 5*H*-1,9,10-Triazadibenzo[a,d]cyclohepten-11-ylgruppe,
   eine 5*H*-Dibenzo[*b*,*e*][1,4]diazepin-11-yl- oder Dibenzo[*b*,*f*][1,4]oxazepin-11-ylgruppe,
   eine Dibenzo[*b*,*f*][1,4]thiazepin-11-yl-, 5-Oxo-dibenzo[*b*,*f*][1,4]thiazepin-11-yl-oder 5,5-Dioxo-dibenzo[*b*,*f*]][1,4]thiazepin-11-ylgruppe,
   eine 5H-Dibenzo[*a*,*d*]cyclohepten-10-yl- oder 5*H*-Dibenzo[*b*,*f*]azepin-10-ylgruppe,
   eine Phenanthridin-6-yl-, Benzo[c][1,5]naphthyridin-6-yl-, Benzo[*h*][1,6]naphthyridin-5-yl-, Benzo[*c*][1,8]naphthyridin-6-yl-, Benzo[*f*][1,7]naphthyridin-5-yl-oder 1,5,9-Triaza-phenanthren-10-ylgruppe,
   eine 1,2,3,4-Tetrahydro-phenanthridin-6-yl, 1,2,3,4,4a,10b-Hexahydrophenanthridin-6-yl-, 2,3-Dihydro-1*H*-4-aza-cyclopenta[*a*]naphth-5-yl- oder 8,9,10,11-Tetrahydro-7*H*-6-aza-cyclohepta[*a*]naphth-5-ylgruppe,
   eine 2,3-Dihydro-1*H*-4-oxa-10-aza-phenanthren-9-yl- oder 1-Oxo-2,3-dihydro-1 H-4-oxa-10-aza-phenanthren-9-ylgruppe,
   eine Phenanthren-9-yl-, Benzo[*h*]chinolin-6-yl-, Benzo[*f*]chinolin-6-yl- oder Benzo[*f*]chinoxalin-6-ylgruppe,
   eine 5*H*-Benzo[*e*]pyrrolo[1,2-*a*][1,4]diazepin-11-yl-, Thieno[3,2-*b*][1,4]benzoxazepin-9-yl-, 5*H*-Dibenzo[*d,t*][1,3]diazepin-6-yl- oder 5-Oxa-7-aza-dibenzo[*a,c*]cyclohepten-6-ylgruppe,
   eine Naphtho[1,2-d]oxazol-2-yl-, Naphtho[2,1-d]oxazol-2-yl-, Naphtho[1,2-*d*]thiazol-2-yl-, Naphtho[2,1-*d*]thiazol-2-yl-, Naphtho[1,2-*d*]imidazol-2-yl-, Naphtho[1,2-*b*]furan-2-yl- oder Naphtho[2,1-*b*]furan-2-ylgruppe,
   oder eine Furo[3,2-*c*]isochinolin-5-yl-, Pyrazolo[1,5-*c*]chinazolin-5-yl- oder 1*H-*Perimidin-2-ylgruppe bedeutet,
   wobei die Benzogruppen der vorstehend erwähnten Reste Rₐ durch die Gruppen R¹⁰ bis R¹³ substituiert sind und die Alkyleneinheiten der vorstehend erwähnten Reste Rₐ durch ein oder zwei Fluoratome oder ein oder zwei Methylgruppen substituiert sein können und die Iminogruppen der vorstehend erwähnten Reste Rₐ durch eine Methylgruppe substituiert sein können und
      R¹⁰ ein Wasserstoffatom,
      ein Fluor-, Chlor-, Brom- oder lodatom,
      eine Methyl- oder Ethylgruppe,
      eine Hydroxy-, Methoxy- oder Ethoxygruppe oder
      eine Difluormethyl-, Trifluormethyl-, Difluormethoxy-, oder Trifluormethoxygruppe und
      R¹¹, R¹² und R¹³, die gleich oder verschieden sein können, jeweils ein Wasserstoff-,Fluor , Chlor- oder Bromatom oder eine Methyl-, Trifluormethyl- oder Methoxygruppe bedeuten,
R² ein Wasserstoffatom oder
eine Methyl-, Cyanomethyl-, Trifluormethyl-, Ethyl-, 2-Cyano-ethyl-, Propyl-, Cyclopropyl- oder Isopropylgruppe,
Y ein Stickstoffatom oder eine Gruppe der Formel C-R⁵,
   wobei R⁵ ein Wasserstoffatom oder eine Methyl-, Ethyl-, Propyl- oder Isopropylgruppe bedeutet,
R³ eine 2-Buten-1-yl- oder 3-Methyl-2-buten-1-ylgruppe,
eine 1-Buten-1-ylgruppe,
eine 2-Butin-1-ylgruppe oder
eine 1-Cyclopenten-1-ylmethylgruppe
und
R⁴ eine (3-Amino-piperidin-1-yl)gruppe bedeuten,
deren Tautomere, Enantiomere, Diastereomere, deren Gemische und deren Salze.

Ganz besonders bevorzugt sind diejenigen Verbindungen der obigen allgemeinen Formel I, in denen
R¹ eine 4-Oxo-3,4-dihydro-chinazolin-2-ylmethylgruppe,
eine Dibenzo[*b,f*][1,4]oxazepin-11-ylmethylgruppe,
eine Phenanthridin-6-ylmethylgruppe,
eine Phenanthren-9-ylmethylgruppe oder
eine Naphtho[1,2-*d*]oxazol-2-ylmethyl- oder Naphtho[2,1-*d*]oxazol-2-ylmethylgruppe,
R² ein Wasserstoffatom oder eine Methylgruppe,
Y ein Stickstoffatom,
R³ eine 2-Butin-1-ylgruppe
und
R⁴ eine (3-Amino-piperidin-1-yl)gruppe bedeuten,
deren Tautomere, Enantiomere, Diastereomere, deren Gemische und deren Salze.

Insbesondere sind folgende Verbindungen der allgemeinen Formel 1 zu nennen:
(1) 2-(3-Amino-piperidin-1-yl)-3-(2-butin-1-yl)-5-[(dibenzo[*b,f*][1,4]oxazepin-11-yl)methyl]-3,5-dihydro-imidazo[4,5-*d*]pyridazin-4-on 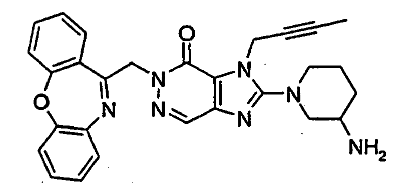
(2) 2-(3-Amino-piperidin-1-yl)-3-(2-butin-1-yl)-5-[(phenanthridin-6-yl)methyl]-3,5-dihydro-imidazo[4,5-*d*]pyridazin-4-on 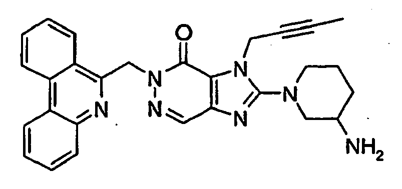
(3) 2-(3-Amino-piperidin-1-yl)-3-(2-butin-1-yl)-5-[(phenanthren-9-yl)methyl]-3,5-dihydro-imidazo[4,5-*d*]pyridazin-4-on 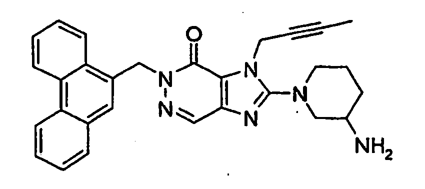
(4) 2-((R)-3-Amino-piperidin-1-yl)-3-(2-butin-1-yl)-5-[(phenanthridin-6-yl)methyl]-7-methyl-3,5-dihydro-imidazo[4,5-*d*]pyridazin-4-on 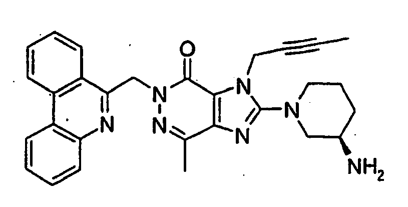
(5) 2-((*R*)-3-Amino-piperidin-1-yl)-3-(2-butin-1-yl)-5-[(dibenzo[*b,f*][1,4]oxazepin-11-yl)methyl]-7-methyl-3,5-dihydro-imidazo[4,5-*d*]pyridazin-4-on 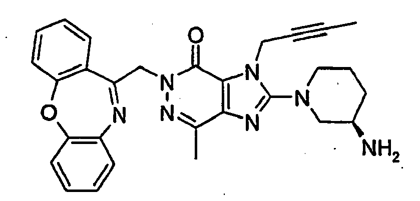
(6) 2-((*S*)-3-Amino-piperidin-1-yl)-3-(2-butin-1-yl)-5-[(dibenzo[*b*,*f*][1,4]oxazepin-11-yl)methyl]-3,5-dihydro-imidazo[4,5-*d*]pyridazin-4-on 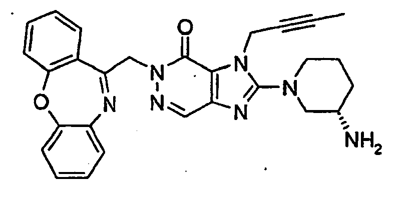
(7) 2-((*R*)-3-Amino-piperidin-1-yl)-3-(2-butin-1-yl)-5-[(dibenzo[*b,f*][1,4]oxazepin-11-yl)methyl]-3,5-dihydro-imidazo[4,5-*d*]pyridazin-4-on 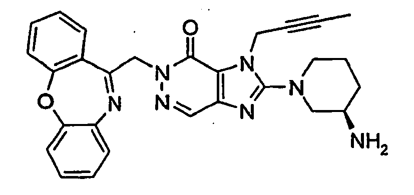
(8) 2-((*R*)-3-Amino-piperidin-1-yl)-3-(2-butin-1-yl)-5-[(naphtho[2,1-*d*]oxazol-2-yl)methyl]-3,5-dihydro-imidazo[4,5-*d*]pyridazin-4-on 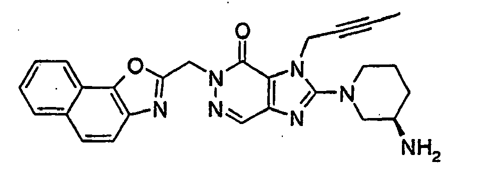
(9) 2-((*R*)-3-Amino-piperidin-1-yl)-3-(2-butin-1-yl)-5-[(naphtho[1,2-d]oxazol-2-yl)methyl]-3,5-dihydro-imidazo[4,5-*d*]pyridazin-4-on 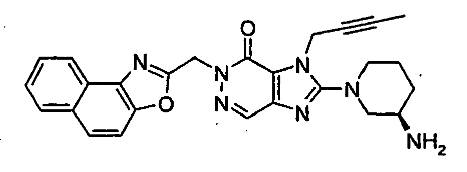
(10) 2-((*R*)-3-Amino-piperidin-1-yl)-3-(2-butin-1-yl)-5-[(4-oxo-3,4-dihydro-chinazolin-2-yl)methyl]-3,5-dihydro-imidazo[4,5-*d*]pyridazin-4-on 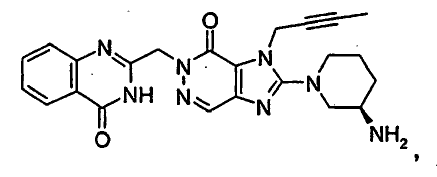
deren Enantiomere, deren Gemische und deren Salze.

Erfindungsgemäß erhält man die Verbindungen der allgemeinen Formel I nach an sich bekannten Verfahren, beispielsweise nach folgenden Verfahren:
a) Entschützung einer Verbindung der allgemeinen Formel

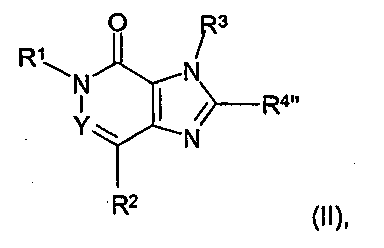

in der R¹, R², Y und R³ wie eingangs erwähnt definiert sind und
R⁴' eine der eingangs für R⁴ erwähnten Gruppen bedeutet, die eine Imino-, Amino-oder Alkylaminogruppe enthalten, wobei die Imino-, Amino- bzw. Alkylaminogruppe durch eine Schutzgruppe substituiert ist.

Die Freisetzung einer Aminogruppe aus einer geschützten Vorstufe ist eine Standardreaktion in der synthetischen organischen Chemie. Als Schutzgruppen kommen eine Vielzahl von Gruppen in Frage. Eine Übersicht über die Chemie der Schutzgruppen findet sich in Theodora W. Greene und Peter G. M. Wuts, Protective Groups in Organic Synthesis, Second Edition, 1991, Verlag John Wiley and Sons sowie in Philip J. Kocienski, Protecting Groups, Georg Thieme Verlag, 1994.

Als Beispiele für Schutzgruppen seien genannt:
die tert.-Butyloxycarbonylgruppe, die sich durch Behandeln mit einer Säure wie beispielsweise Trifluoressigsäure oder Salzsäure oder durch Behandlung mit Bromtrimethylsilan oder lodtrimethylsilan gegebenenfalls unter Verwendung eines Lösungsmittels wie Methylenchlorid, Essigester, Dioxan, Methanol, Isopropanol oder Diethylether bei Temperaturen zwischen 0 und 80°C abspalten lässt,
die 2.2.2-Trichlorethoxycarbonylgruppe; die sich abspalten lässt durch Behandeln mit Metallen wie beispielsweise Zink oder Cadmium in einem Lösungsmittel wie Essigsäure oder einem Gemisch aus Tetrahydrofuran und einer schwachen wässrigen Säure bei Temperaturen zwischen 0°C und der Siedetemperatur des verwendeten Lösungsmittels und
die Carbobenzyloxycarbonylgruppe, die sich beispielsweise abspalten lässt durch Hydrogenolyse in Gegenwart eines Edelmetallkatalysators wie beispielsweise Palladium-Kohle und einem Lösungsmittel wie beispielsweise Alkohole, Essigester, Dioxan, Tetrahydrofuran oder Gemische dieser Lösungsmittel bei Temperaturen zwischen 0°C und dem Siedepunkt des Lösungsmittels, durch Behandeln mit Bortribromid in Methylenchlorid bei Temperaturen zwischen -20°C und Raumtemperatur oder durch Behandeln mit Aluminiumchlorid/Anisol bei Temperaturen zwischen 0°C und Raumtemperatur.

Ferner können die erhaltenen, Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem Stereozentrum in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel 1, welche in Racematen auftreten, nach an sich bekannten Methoden (siehe Allinger N. L. und Eliel E. L. in "Topics in Stereochemistry", Vol. 6, Wiley Inter science, 1971) in ihre optischen Antipoden und Verbindungen der allgemeinen Formel I mit mindestens zwei asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren aufgetrennt werden können.

Die Enantiomerentrennung erfolgt vorzugsweise durch Säulentrennung an chiralen Phasen oder durch Umkristallisieren aus einem optisch aktiven Lösungsmittel oder durch Umetzen mit einer, mit der racernischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-p-toluoyl-weinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Des Weiteren können die erhaltenen Verbindungen der Formel 1 in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen der allgemeinen Formel II sind entweder literaturbekannt oder man erhält diese nach an sich literaturbekannten Verfahren (siehe Beispiele I bis XVI).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym DPP-IV.

Die biologischen Eigenschaften der neuen Verbindungen wurden wie folgt geprüft:

Die Fähigkeit der Substanzen und ihrer entsprechenden Salze, die DPP-IV Aktivität zu hemmen, kann in einem Versuchsaufbau gezeigt werden, in dem ein Extrakt der humanen Koloncarcinomzelllinie Caco-2 als DPP IV Quelle benutzt wird. Die Differenzierung der Zellen, um die DPP-IV Expression zu induzieren, wurde nach der Beschreibung von Reiher et al. in einem Artikel mit dem Titel "Increased expression of intestinal cell line Caco-2" , erschienen in Proc. Natl. Acad. Sci. Vol. 90, Seiten 5757-5761 (1993), durchgeführt. Der Zellextrakt wurde von in einem Puffer (10mM Tris HCI, 0.15 M NaCI, 0.04 t.i.u. Aprotinin, 0.5% Nonidet-P40, pH 8.0) solubilisierten Zellen durch Zentrifugation bei 35,000 g für 30 Minuten bei 4°C (zur Entfernung von Zelltrümmern) gewonnen.

Der DPP-IV Assay wurde wie folgt durchgeführt:

50 µl Substratlösung (AFC; AFC ist Amido-4-trifluormethylcoumarin), Endkonzentration 100 µM, wurden in schwarze Mikrotiterplatten vorgelegt. 20 µl Assay Puffer (Endkonzentrationen 50 mM Tris HCI pH 7.8, 50 mM NaCl, 1 % DMSO) wurde zupipettiert. Die Reaktion wurde durch Zugabe von 30 µl solubilisiertern Caco-2 Protein (Endkonzentration 0.14 µg Protein pro Weil) gestartet. Die zu überprüfenden Testsubstanzen wurden typischerweise in 20 µl vorverdünnt zugefügt, wobei das Assaypuffervolumen dann entsprechend reduziert wurde. Die Reaktion wurde bei Raumtemperatur durchgeführt, die Inkubationsdauer betrug 60 Minuten. Danach wurde die Fluoreszenz in einem Victor 1420 Multilabel Counter gemessen, wobei die Anregungswellenlänge bei 405 nm und die Emissionswellenlänge bei 535 nm lag. Leerwerte (entsprechend 0 % Aktivität) wurden in Ansätzen ohne Caco-2 Protein (Volumen ersetzt durch Assay Puffer), Kontrollwerte (entsprechend 100 % Aktivität) wurden in Ansätzen ohne Substanzzusatz erhalten. Die Wirkstärke der jeweiligen Testsubstanzen, ausgedrückt als IC₅₀ Werte, wurden aus Dosis-Wirkungs Kurven berechnet, die aus jeweils 11 Meßpunkten bestanden. Hierbei wurden folgende Ergebnisse erhalten:

| Verbindung (Beispiel Nr.) | DPP IV-Hemmung IC₅₀ [nM] |
|---|---|
| 1 | 14 |
| 1(1) | 17 |
| 1(2) | 58 |
| 1(3) | 8 |
| 1(4) | 9 |
| 1(7) | 3 |
| 1(8) | 7 |
| 1(9) | 3 |

Die erfindungsgemäß hergestellten Verbindungen sind gut verträglich, da beispielsweise nach oraler Gabe von 10 mg/kg der Verbindung des Beispiels 1 an Ratten keine Änderungen im Verhalten der Tiere beobachtet werden konnten.

Im Hinblick auf die Fähigkeit, die DPP-IV Aktivität zu hemmen, sind die erfindungsgemäßen Verbindungen der allgemeinen Formel I und ihre entsprechenden pharmazeutisch akzeptablen Salze geeignet, alle diejenigen Zustände oder Krankheiten zu beeinflussen, die durch eine Hemmung der DPP-IV Aktivität beeinflusst werden können. Es ist daher zu erwarten, daß die erfindungsgemäßen Verbindungen zur Prävention oder Behandlung von Krankheiten oder Zuständen wie Diabetes mellitus Typ I und Typ II, diabetische Komplikationen, metabolische Azidose oder Ketose, Insulinresistenz, Dyslipidämien unterschiedlichster Genese, Arthritis, Atherosklerose und verwandte Erkrankungen, Adipositas, Allograft Transplantation und durch Calcitonin verursachte Osteoporose geeignet sind. Darüberhinaus sind diese Substanzen geeignet, die B-Zelldegeneration wie z.B. Apoptose oder Nekrose von pankreatischen B-Zellen zu verhindern. Die Substanzen sind weiter geeignet, die Funktionalität von pankreatischen Zellen zu verbessern oder wiederherzustellen, daneben die Anzahl und Größe von pankreatischen B-Zellen zu erhöhen. Zusätzlich und begründet durch die Rolle der Glucagon-Like Peptide, wie z.B. GLP-1 und GLP-2 und deren Verknüpfung mit DPP-IV Inhibition, wird erwartet, daß die erfindungsgemäßen Verbindungen geeignet sind, um unter anderem einen sedierenden oder angstlösenden Effekt zu erzielen, darüberhinaus katabole Zustände nach Operationen oder hormonelle Stressantworten günstig zu beeinflussen oder die Mortalität und Morbidität nach Myokardinfarkt reduzieren zu können. Darüberhinaus sind sie geeignet zur Behandlung von allen Zuständen, die im Zusammenhang mit oben genannten Effekten stehen und durch GLP-1 oder GLP-2 vermittelt sind. Die erfindungsgemäßen Verbindungen sind ebenfalls als Diuretika oder Antihypertensiva einsetzbar und zur Prävention und Behandlung des akuten Nierenversagens geeignet. Ebenso sind sie zur Prävention und Therapie von chronischen entzündlichen Darmerkrankungen geeignet. Darüberhinaus wird erwartet, daß DPP-IV Inhibitoren und somit auch die erfindungsgemäßen Verbindungen zur Behandlung der Unfruchtbarkeit oder zur Verbesserung der Fruchtbarkeit beim Menschen oder im Säugetierorgamsmus verwendet werden können, insbesondere dann, wenn die Unfruchtbarkeit im Zusammenhang mit einer Insulinresistenz oder mit dem polyzystischen Ovarialsyndrom steht. Des weiteren sind die Substanzen geeignet, Mangelzustände von Wachstumshormon, die mit Minderwuchs einhergehen, zu beeinflussen.

Die erfindungsgemäßen Verbindungen können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. Gl 262570), alpha-Glucosidasehemmer (z.B. Acarbose, Voglibose), alpha2-Antagonisten, Insulin und Insulinanaloga, GLP-1 und GLP-1 Analoga (z.B. Exendin-4) oder Amylin. Daneben Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, Cholesterolresorptionsinhibitoren wie zum Beispiel Ezetimibe, gallensäurebindende Substanzen wie zum Beispiel Colestyramin, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin oder β3-Agonisten wie SB-418790 oder AD-9677.
Daneben ist eine Kombination mit Medikamenten zur Beeinflussung des Bluthochdrucks wie z.B. All Antagonisten oder ACE Inhibitoren, Diuretika, β-Blocker und andere oder Kombinationen daraus geeignet.

Die zur Erzielung einer entsprechenden Wirkung erforderliche Dosierung beträgt zweckmäßigerweise bei intravenöser Gabe 1 bis 100 mg, vorzugsweise 1 bis 30 mg, und bei oraler Gabe 1 bis 1000 mg, vorzugsweise 1 bis 100 mg, jeweils 1 bis 4 x täglich. Hierzu lassen sich die erfindungsgemäß hergestellten Verbindungen der Formel 1, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol; Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die nachfolgenden Beispiele sollen die Erfindung näher erläutern:

Herstellung der Ausgangsverbindungen:

### Beispiel I

### 2-[3-(tert.-Butyloxycarbonylamino)-piperidin-1-yl]-3-(2-butin-1-yl)-5-[(dibenzo[b,f][1,4]-oxazepin-11-yl)methyl]-3,5-dihydro-imidazo[4,5-d]pyridazin-4-on

Zu 400 mg 2-[3-(tert.-Butyloxycarbonylamino)-piperidin-1-yl]-3-(2-butin-1-yl)-3,5-dihydro-imidazo[4,5-d]pyridazin-4-on und 276 mg Kaliumcarbonat in 4 ml N,N-Dimethylformamid werden 317 mg 11-Chlormethyl-dibenzo[*b,f*][1,4]oxazepin gegeben. Das Reaktionsgemisch wird zwei Stunden bei 80°C gerührt. Zur Aufarbeitung wird es mit Wasser versetzt und der entstandene Niederschlag wird abgesaugt. Das Rohprodukt wird chromatographisch über eine Kieselgelsäule mit Methylenchlorid/Methanol (100:0 auf 70:30) als Laufmittel gereinigt.
Ausbeute: 120 mg (20 % der Theorie)
Massenspektrum (ESI⁺): m/z = 594 [M+H]⁺

Analog Beispiel 1 werden folgende Verbindungen erhalten:
(1) 2-[3-(tert.-Butyloxycarbonylamino)-piperidin-1-yl]-3-(2-butin-1-yl)-5-[(phenanthridin-6-yl)methyl]-3,5-dihydro-imidazo[4,5-d]pyridazin-4-on
(2) 2-[3-(tert.-Butyloxycarbonylamino)-piperidin-1-yl]-3-(2-butin-1-yl)-5-[(phenanthren-9-yl)methyl]-3,5-dihydro-imidazo[4,5-d]pyridazin-4-on
(3) 2-[(*R*)-3-(tert.-Butyloxycarbonylamino)-piperidin-1-yl]-3-(2-butin-1-yl)-5-[(phenanthridin-6-yl)methyl]-7-methyl-3,5-dihydro-imidazo[4,5-d]pyridazin-4-on R_{f}-Wert: 0.41 (Kieselgel, Cyclohexan/Essigester = 3:7) Massenspektrum (ESI⁺): m/z = 592 [M+H]⁺
(4) 2-[(*R*)-3-(tert.-Butyloxycarbonylamino)-piperidin-1-yl]-3-(2-butin-1-yl)-5-[(dibenzo[*b,f*][1,4]oxazepin-11-yl)methyl]-7-methyl-3,5-dihydro-imidazo[4,5-d]pyridazin-4-on
   R_{f}-Wert: 0.50 (Kieselgel, Cyclohexan/Essigester = 2:8)
   Massenspektrum (ESI⁺): m/z = 608 [M+H]⁺
(5) 2-Brom-3-(2-butin-1-yl)-5-[(dibenzo[*b,t*][1,4]oxazepin-11-yl)methyl]-3,5-dihydroimidazo[4,5-d]pyridazin-4-on
   Massenspektrum (ESI⁺): m/z = 474, 476 [M+H]⁺
(6) 2-Brom-3-(2-butin-1-yl)-5-[(naphtho[2,1-d]oxazol-2-yl)methyl]-3,5-dihydroimidazo[4,5-d]pyridazin-4-on
   R_{f}-Wert: 0.80 (Kieselgel, Methylenchlorid/Ethanol = 9:1)
(7) 2-Brom-3-(2-butin-1-yl)-5-[(naphtho[1,2-d]oxazol-2-yl)methyl]-3,5-dihydroimidazo[4,5-*d*]pyridazin-4-on
   R_{f}-Wert: 0.50 (Kieselgel, Methylenchlorid/Methanol = 19:1)
(8) 2-[(*R*)-3-(tert.-Butyloxycarbonylamino)-piperidin-1 -yl]-3-(2-butin-1-yl)-5-cyanomethyl-3,5-dihydro-imidazo[4,5-*d*]pyridazin-4-on
   R_{f}-Wert: 0.40 (Kieselgel, Petrolether/Essigester = 1:4)
   Massenspektrum (ESI⁺): m/z = 426 [M+H]⁺

### Beispiel II

### 2-[3-(tert.-Butyloxycarbonylamino)-piperidin-1-yl]-3-(2-butin-1-yl)-3,5-dihydroimidazo[4,5-d]pyridazin-4-on

Zu 2.65 g 2-Brom-3-(2-butin-1-yl)-3,5-dihydro-imidazo[4,5-d]pyridazin-4-on und 2.12 g Natriumcarbonat in 5 ml Dimethylsulfoxid werden 2.50 g 3-(tert.-Butyloxycarbonylamino)-piperidin gegeben. Das Reaktionsgemisch wird über Nacht bei 85°C gerührt. Nach Abkühlung auf Raumtemperatur wird es mit Wasser versetzt und mit Essigester extrahiert. Die vereinigten organischen Phasen werden über Magnesiumcarbonat getrocknet und eingeengt. Das Rohprodukt wird ohne weitere Reinigung weiter umgesetzt.
Massenspektrum (ESI⁺): m/z = 387 [M+H]⁺

Analog Beispiel II werden folgende Verbindungen erhalten:
(1) 2-[(*R*)-3-(tert.-Butyloxycarbonylamino)-piperidin-1-yl]-3-(2-butin-1-yl)-7-methyl-3,5-dihydro-imidazo[4,5-*d*]pyridazin-4-on
   R_{f}-Wert: 0.15 (Kieselgel, Cyclohexan/Essigester = 3:7)
   Massenspektrum (ESI⁺): m/z = 401 [M+H]⁺
(2) 2-[(*S*)3-(tert.-Butyloxycarbonylamino)-piperidin-1-yl]-3-(2-butin-1-yl)-5-[(dibenzo[*b*,*f*][1,4]oxazepin-11-yl)methyl]-3,5-dihydro-imidazo[4,5-*d*]pyridazin-4-on
   Massenspektrum (ESI⁺): m/z = 594 [M+H]⁺
(3) 2-[(*R*)-3-(tert.-Butyloxycarbonylamino)-piperidin-1-yl]-3-(2-butin-1-yl)-5-[(dibenzo[*b*,*f*][1,4]oxazepin-11-yl)methyl]-3,5-dihydro-imidazo[4,5-d]pyridazin-4-on
   Massenspektrum (ESI⁺): m/z = 594 [M+H]⁺
(4) 2-[(*R*)-3-(tert.-Butyloxycarbonylamino)-piperidin-1-yl]-3-(2-butin-1-yl)-5-[(naphtho[2,1-*d*]oxazol-2-yl)methyl]-3,5-dihydro-imidazo[4,5-*d*]pyridazin-4-on
   R_{f}-Wert: 0.70 (Kieselgel, Methylenchlorid/Ethanol = 9:1)
(5) 2-[(*R*)-3-(tert.-Butyloxycarbonylamino)-piperidin-1-yl]-3-(2-butin-1-yl)-5-[(naphtho[1,2-*d*]oxazol-2-yl)methyl]-3,5-dihydro-imidazo[4,5-*d*]pyridazin-4-on R_{f}-Wert: 0.65 (Kieselgel, Methylenchlorid/Methanol = 9:1)
   Massenspektrum (ESI⁺): m/z = 568 [M+H]⁺
(6) 2-[(*R*)-3-(tert.-Butyloxycarbonylamino)-piperidin-1-yl]-3-(2-butin-1-yl)-3,5-dihydroimidazo[4,5-*d*]pyridazin-4-on
   Massenspektrum (ESI⁺): m/z = 387 [M+H]⁺
   R_{f}-Wert: 0.50 (Kieselgel, Methylenchlorid/Ethanol = 9:1)

### Beispiel III

### 2-Brom-3-(2-butin-1-yl)-3,5-dihydro-imidazo[4,5-d]pyridazin-4-on

Zu 3.68 g 2-Brom-3-(2-butin-1-yl)-5-formyl-3*H*-imidazol-4-carbonsäure-methylester in 50 ml Ethanol werden 0.63 ml Hydrzinhydrat getropft. Das Reaktionsgemisch wird eine Stunde bei Raumtemperatur gerührt, dann werden 3 ml Essigsäure zugegeben und das Reaktionsgemisch wird eine weitere Stunde unter Rückfluß erhitzt. Der entstandene Niederschlag wird abgesaugt, mit Ethanol und Diethylether nachgewaschen und getrocknet
Ausbeute: 2.65 g (77 % der Theorie)
Massenspektrum (ESI⁺): m/z = 267, 269 [M+H]⁺

### Beispiel IV

### 2-Brom-3-(2-butin-1-yl)-5-formyl-3H-imidazol-4-carbonsäure-methylester

Zu 12.45 g 2-Brom-3-(2-butin-1-yl)-1*H*-imidazol-4,5-dicarbonsäure-dimethylester in 150 ml Tetrahydrofuran werden unter Argonatmosphäre bei -65°C 45 ml Diisobutylaluminiumhydrid-Lösung (1 M in Toluol) getropft. Das Reaktionsgemisch wird zwei Stunden bei -65°C gerührt, dann werden nochmals 9 ml Diisobutylaluminiumhydrid-Lösung zugegeben. Nach einer weiteren Stunde wird bei -65°C mit einem Gemisch aus 1 M Salzsäure und Tetrahydrofuran (1:1) gequencht und zehn Minuten nachgerührt. Anschließend wird das Kühlbad entfernt, das Reaktionsgemisch mit Wasser verdünnt und mit Essigester extrahiert. Die vereinigten organischen Phasen werden über Magnesiumsulfat getrocknet und eingeengt. Das Rohprodukt wird chromatographisch über eine Kieselgelsäule mit Cyclohexan/Essigester (2:1 auf 1:1) gereinigt. Ausbeute: 9.58 g (85 % der Theorie)
Massenspektrum (ESI⁺): m/z = 285, 287 [M+H]⁺

Analog Beispiel IV werden folgende Verbindungen erhalten:
(1) 2-Brom-3-(3-methyl-2-buten-1-yl)-5-formyl-3H-imidazol-4-carbonsäuremethylester
Massenspektrum (ESI⁺): m/z = 301, 303 [M+H]⁺

### Beispiel V

### 2-Brom-3-(2-butin-1-yl)-1H imidazol-4,5-dicarbonsäure-dimethylester

Zu 13.20 g 2-Brom-1*H*-imidazol-4,5-dicarbonsäure-dimethylester und 8.57 g Kaliumcarbonat in 70 ml N,N-Dimethylformamid werden 4.53 ml 1-Brom-2-butin gegeben und das Reaktionsgemisch wird über Nacht bei Raumtemperatur gerührt. Zur Aufarbeitung wird es mit Wasser versetzt und mit Essigester extrahiert. Die vereinigten organischen Phasen werden über Magnesiumsulfat getrocknet und eingeengt.
Ausbeute: 14.58 g (92 % der Theorie)
Massenspektrum (ESI⁺): m/z = 315, 317 [M+H]⁺

Analog Beispiel V werden folgende Verbindungen erhalten:
(1) 2-Brom-3-(3-methyl-2-buten-1-yl)-1*H*-imidazol-4,5-dicarbonsäure-dimethylester Massenspektrum (ESI⁺): m/z = 331, 333 [M+H]⁺

### Beispiel VI

### 2-Brom-1H-imidazol-4,5-dicarbonsäure-dimethylester

Zu 19.80 g 1*H*-Imidazol-4,5-dicarbonsäure-dimethylester und 14.92 g Kaliumcarbonat in 600 ml Methylenchlorid werden mit 6.11 ml Brom versetzt. Das Reaktionsgemisch wird eine Stunde bei Raumtemperatur gerührt, dann wird eine Gemisch aus gesättigter Natriumsulfitlösung und gesättigter Natriumchloridlösung (1:1) zugegeben. Die organische Phase wird weitgehend abgetrennt und die wässrige Phase mehrmals mit Essigester extrahiert. Die vereinigten organischen Phasen werden über Magnesiumsulfat getrocknet und eingeengt, wobei etwa 7.40 g Rohprodukt zurückbleiben. Die wässrige Phase wird mit Essigester versetzt und über Nacht in einer Extraktionsapparatur extrahiert. Der Essigesterextrakt wird eingeengt und der Kolbenrückstand mit dem bereits gewonnen Rohprodukt vereinigt.
Ausbeute: 13.10 g (46 % der Theorie)
Massenspektrum (ESI⁺): m/z = 263, 265 [M+H]⁺

### Beispiel VII

### 2-Brom-3-(2-butin-1-yl)-7-methyl-3,5-dihydro-imidazo[4,5-d]pyridazin-4-on

Zu 1.30 g 2-Brom-7-methyl-3,5-dihydro-imidazo[4,5-d]pyridazin-4-on und 0.99 ml Hünigbase in 30 ml N,N-Dimethylformamid werden 0.50 ml 1-Brom-2-butin gegeben. Das Reaktionsgemisch wird drei Stunden bei Raumtemperatur gerührt. Anschließend wird das Lösungsmittel am Rotationsverdampfer im Vakkum abdestilliert. Der Kolbenrückstand wird mit 40 ml Wasser und 0.5 ml konzentrierter wäßriger Ammoniaklösung verrührt, abgesaugt und mit Ethanol sowie Diethylether nachgewaschen. Ausbeute: 1.30 g (82 % der Theorie)
R_{f}-Wert: 0.60 (Kieselgel, Cyclohexan/Essigester = 3:7)
Massenspektrum (ESI⁺): m/z = 281, 283 [M+H]⁺

### Beispiel VIII

### 2-Brom-7-methyl-3,5-dihydro-imidazo[4,5-d]pyridazin-4-on

Zu 1.40 g 7-Methyl-3,5-dihydro-imidazo[4,5-*d*]pyridazin-4-on und 1.30 g Kaliumcarbonat in 40 ml Acetonitril werden langsam 5.20 ml einer 1.8 M Lösung von Brom in Acetonitril getropft. Anschließend wird das Reaktionsgemisch auf 70°C erwärmt, wobei eine rasche Entfärbung eintritt. Es wird nochmals portionsweise Bromlösung und Kaliumcarbonat zugegeben, bis die Umsetzung laut HPLC-MS. vollständig ist. Zur Aufarbeitung wird das Reaktionsgemisch eingeengt, mit 100 ml Wasser verrührt und abgesaugt. Das Filtrat wird mit 1 M Salzsäure angesäuert und mit Essigester extrahiert. Die vereinigten Extrakte werden über Natriumsulfat getrocknet und eingeengt.
Ausbeute: 1.30 g (61 % der Theorie)
R_{f}-Wert: 0.37 (Kieselgel, Methylenchlorid/Methanol = 9:1)
Massenspektrum (ESI⁺): m/z = 229, 231 [M+H]⁺

### Beispiel IX

### 7-Methyl-3,5-dihydro-imidazo[4,5-d]pyridazin-4-on

Zu 2.20 g 4-Amino-7-methyl-3*H*-imidazo[4,5-*d*]pyridazin in einem Gemisch aus 30 ml Essigsäure, 5 ml Wasser und 0.5 ml konzentrierter Schwefelsäure wird bei 50°C eine Lösung aus 4.00 g Natriumnitrit in 15 ml Wasser getropft. Das Reaktionsgemisch wird noch zwei Stunden bei 50°C gerührt und anschließend eine Stunde auf 90 °C erhitzt. Nach Abkühlung auf Raumtemperatur wird das Reaktionsgemisch mit 30 ml Wasser verdünnt. Der entstandene Niederschlag wird abgesaugt, mit Wasser, Ethanol und Diethylether nachgewaschen und getrocknet.
Ausbeute: 1.00 g (45 % der Theorie)
Massenspektrum (ESI⁺): m/z = 151 [M+H]⁺

### Beispiel X

### 4-Amino-7-methyl-3H-imidazo[4,5-d]pyridazin

Ein Gemisch aus 2.00 g 5-Acetyl-3*H*-imidazol-4-carbonitril und 4.00 ml Hydrazinhydrat in 50 ml Ethanol wird auf 100 °C erhitzt, bis die Umsetzung laut HPLC-MS komplett ist. Nach Abkühlung auf Raumtemperatur wird das Reaktionsgemisch eingeengt, mit 20 ml kaltem Etanol verrührt und abgesaugt. Der Filterkuchen wird mit Diethylether nachgewaschen und getrocknet.
Ausbeute: 2.10 g (95 % der Theorie)
Massenspektrum (ESI⁺): m/z = 150 [M+H]⁺

### Beispiel XI

### 5-Acetyl-3H-imidazol-4-carbonitril

Zu 7.00 g 4,5-Dicyano-imidazol in 80 ml Tetrahydrofuran werden unter Argonatmosphäre 57 ml einer 3 M Lösung von Methylmagnesiumbromid in Diethylether gegeben, wobei die Temperatur zwischen 5°C und 15°C gehalten wird.
Nach zwei Stunden ist die Umsetzung laut Dünnschichtchromatogramm vollständig und das Reaktionsgemisch wird mit 400 ml Essigester verdünnt. Anschließend wird es langsam mit 400 ml gesättigter Ammoniumchloridlösung versetzt. Nach zehn Minuten wird das Gemisch mit halbkonzentrierter Schwefelsäure angesäuert und noch weitere zwanzig Minuten gerührt, bevor die organische Phase abgetrennt wird. Die wässrige Phase wird mit Essigester extrahiert und die vereinigten organischen Phasen werden über Natriumsulfat getrocknet und eingeengt. Der Kolbenrückstand wird mit Essigester verrührt, abgesaugt und mit Essigester und Diethylether nachgewaschen.
Ausbeute: 3.30 g (43 % der Theorie)
Massenspektrum (ESI⁺): m/z = 136 [M+H]⁺

### Beispiel XII

### 2-Chlormethyl-naphtho[2,1-d]oxazol

Hergestellt durch Umsetzung von 2.93 g 2-Amino-1-naphthol mit 3.54 g 2-Chlor-1,1,1-triethoxy-ethan in 25 ml Ethanol bei 60°C.
Ausbeute: 1.90 g (58 % der Theorie)
R_{f}-Wert: 0.55 (Kieselgel, Petrolether/Essigester = 9:1)
Massenspektrum (ESI⁺): *m*/*z* = 218, 220 [M+H]⁺

Analog Beispiel XII werden folgende Verbindungen erhalten:
(1) 2-Chlormethyl-naphtho[1,2-*d*]oxazol
R_{f}-Wert: 0.90 (Kieselgel, Methylenchlorid/Methanol = 19:1)
Massenspektrum (ESI⁺): m/z = 218, 220 [M+H]⁺

### Beispiel XIII

### 2-Brom-3-(3methyl-2-buten-1-yl)-3,5-dihydro-imidazo[4,5-c]pyridin-4-on

Zu 1.60 g 2-Brom-7-hydroxy-3-(3-methyl-2-buten-1-yl)-3,5,6,7-tetrahydro-imidazo-[4,5-c]pyridin-4-on in 20 ml Methylenchlorid und 4 ml Tetrahydrofuran werden 1.55 g Burgess-Reagenz (Methoxycarbonylsulfamoyl-triethylammonium-N-betain) gegeben. Das Reaktionsgemisch wird acht Stunden bei 60°C gerührt, dann werden noch einmal 0.3 Äquivalente Burgess-Reagenz zugegeben. Nach weiteren zwei Stunden wird das abgekühlte Reaktionsgemisch mit wässriger Natriumhydrogencarbonatlösung versetzt und mit Essigester extrahiert. Die vereinigten organischen Phasen werden über Magnesiumsulfat getrocknet und eingeengt. Der Kolbenrückstand wird über eine Kieselgelsäule mit Methylenchlorid/Methanol (1:0 auf 10:1) als Laufmittel chromatographiert.
Ausbeute: 1.06 g (60 % der Theorie)
Massenspektrum (ESI⁺): m/z = 282, 284 [M+H]⁺

### Beispiel XIV

### 2-Brom-7-hydroxy-3-(3-methyl-2-buten-1-yl)-3,5,6,7-tetrahydro-imidazo[4,5-c]pyridin-4-on

Zu 4.15 g 2-Brom-5-(1-hydroxy-2-nitro-ethyl)-3-(3-methyl-2-buten-1-yl)-3*H*-imidazol-4-carbonsäure-methylester in 270 ml Ethanol werden 90 ml Wasser und 5.40 g Eisenpulver gegeben. Das Gemisch wird zum Rückfluß erhitzt, mit 36 ml Eisessig versetzt und anderthalb Stunden unter Rückfluß gerührt. Die abgekühlte Reaktionslösung wird über Celite filtriert. Das Filtrat wird eingeengt, mit Ethanol versetzt und mit festem Kaliumcarbonat basisch gestellt. Das Gemisch wird drei Stunden bei 60°C gerührt. Anschließend wird das Ethanol abdestilliert, der Kolbenrückstand mit Wasser versetzt und mit Essigester extrahiert. Die vereinigten Extrakte werden über Magnesiumsulfat getrocknet und eingeengt. Das Rohprodukt wird chromatographisch über eine Kieselgelsäule mit Methylenchlorid/Methanol (1:1 auf 7:1) als Laufmittel gereinigt.
Ausbeute: 1.62 g (47 % der Theorie)
Massenspektrum (ESI⁺): m/z = 300, 302 [M+H]⁺

### Beispiel XV

### 2-Brom-5-(1-hydroxy-2-nitro-ethyl)-3-(3-methyl-2-buten-1-yl)-3H-imidazol-4-carbonsäure-methylester

Zu 1.14 g Cäsiumcarbonat in 15 ml Methanol werden bei Raumtemperatur 35 ml Nitromethan gegeben. Anschließend wird das Gemisch mit einer Lösung aus 3.50 g 2-Brom-3-(3-methyl-2-buten-1-yl)-5-formyl-3H-imidazol-4-carbonsäure-methylester in 20 ml Methanol und 5 ml Methylenchlorid versetzt und 15 Minuten bei Raumtemperatur gerührt. Dann werden 0.5 ml Essigsäure zugegeben und die Lösung wird im Vakuum eingeengt. Der Kolbenrückstand wird mit wässriger Natriumhydrogencarbonatlösung versetzt und mit Essigester extrahiert. Die vereinigten organischen Phasen werden über Magnesiumsulfat getrocknet und eingeengt.
Ausbeute: 4.15 g (99 % der Theorie)
Massenspektrum (ESI⁺): m/z = 362, 364 [M+H]⁺

### Beispiel XVI

### 2-[(R)-3-(tert.-Butyloxycarbonylamino)-piperidin-1-yl]-3-(2-butin-1-yl)-5-[(4-oxo-3,4-dihydro-chinazolin-2-yl)methyl]-3,5-dihydro-imidazo[4,5-d]pyridazin-4-on

Zu einer Lösung aus 605 mg 2-[(*R*)-3-(tert.-Butyloxycarbonylamino)-piperidin-1-yl]-3-(2-butin-1-yl)-5-cyanomethyl-3,5-dihydro-imidazo[4,5-*d*]pyridazin-4-on in 9 ml Methanol werden 40 mg Natriummethylat (95 %) gegeben. Das Gemisch wird eine Stunde bei Raumtemperatur gerührt und anschließend mit 41 µL Eisessig neutralisiert. Dann wird eine Lösung aus 195 mg Anthranilsäure in 2 ml Methanol zugegeben und das Reaktionsgemisch wird auf 70°C erwärmt. Nach etwa zwei Stunden fällt eine weißer, voluminöser Niederschlag aus und das Reaktionsgemisch wird auf Raumtemperatur abgekühlt. Der entstandene Niederschlag wird abgesaugt, mit kaltem Methanol gewaschen und getrocknet.
Ausbeute: 234 mg (30 % der Theorie)
Massenspektrum (ESI⁺): m/z = 545 [M+H]⁺

Herstellung der Endverbindungen:

### Beispiel 1

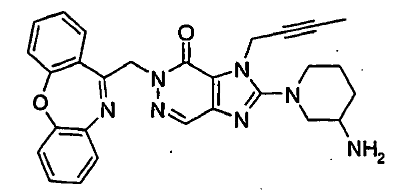

### 2-(3-Amino-piperidin-1-yl)-3-(2-butin-1-yl)-5-[(dibenzo[b,f][1,4]oxazepin-11-yl)methyl]-3,5-dihydro-imidazo[4,5-d]pyridazin-4-on

Zu 120 mg 2-[3-(tert.-Butyloxycarbonylamino)-piperidin-1-yl]-3-(2-butin-1-yl)-5-[(dibenzo[*b*,*f*][1,4]oxazepin-11-yl)methyl]-3,5-dihydro-imidazo[4,5-*d*]pyridazin-4-on in 3 ml Methylenchlorid werden unter Eisbad-Kühlung 0.33 ml Trifluoressigsäure gegeben. Das Reaktionsgemisch wird über Nacht bei Raumtemperatur gerührt. Zur Aufarbeitung wird es auf gekühlte gesättigte Kaliumcarbonatlösung gegossen und mit Methylenchlorid extrahiert. Die organische Phase wird abgetrennt und eingeengt. Das Rohprodukt wird chromatographisch über eine Kieselgelsäule mit Methylenchlorid/Methanol (100:0 auf 70:30) als Laufmittel gereinigt.
Ausbeute: 63 mg (63 % der Theorie)
Massenspektrum (ESI⁺): m/z = 494 [M+H]⁺

Analog Beispiel 1 werden folgende Verbindungen erhalten:
(1) 2-(3-Amino-piperidin-1-yl)-3-(2-butin-1-yl)-5-[(phenanthridin-6-yl)methyl]-3,5-dihydro-imidazo[4,5-*d*]pyridazin-4-on 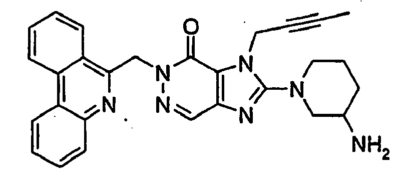 Massenspektrum (ESI⁺): m/z = 478 [M+H]⁺
(2) 2-(3-Amino-piperidin-1-yl)-3-(2-butin-1-yl)-5-[(phenanthren-9-yl)methyl]-3,5-dihydro-imidazo[4,5-d]pyridazin-4-on 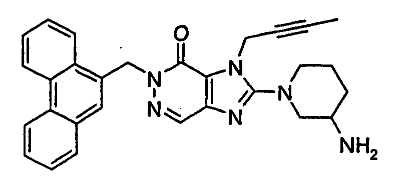 Massenspektrum (ESI⁺): m/z = 477 [M+H]⁺
(3) 2-((*R*)-3-Amino-piperidin-1-yl)-3-(2-butin-1-yl)-5-[(phenanthridin-6-yl)methyl]-7-methyl-3,5-dihydro-imidazo[4,5-*d*]pyridazin-4-on x Trifluoressigsäure 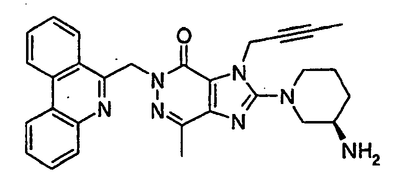 x CF₃COOH
   R_{f}-Wert: 0.45 (Reversed Phase DC-Fertigplatte (E. Merck), Acetonitril/Wasser/
   Trifluoressigsäure = 50:50:0.1)
   Massenspektrum (ESI⁺): m/z = 492 [M+H]⁺
(4) 2-((*R*)-3-Amino-piperidin-1-yl)-3-(2-butin-1-yl)-5-[(dibenzo[*b*,*f*][1,4]oxazepin-11-yl)methyl]-7-methyl-3,5-dihydro-imidazo[4,5-*d*]pyridazin-4-on 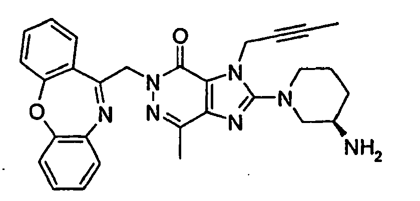 Durchführung mit isopropanolischer Salzsäure (5-6 M) in Methylenchlorid.
   Massenspektrum (ESI⁺): m/z = 508 [M+H]⁺
(5) 2-((*S*)-3-Amino-piperidin-1-yl)-3-(2-butin-1-yl)-5-[(dibenzo[*b,f*][1,4]oxazepin-11 - yl)methyl]-3,5-dihydro-imidazo[4,5-*d*]pyridazin-4-on 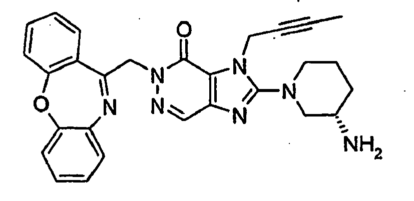 Massenspektrum (ESI⁺): m/z = 494 [M+H]⁺
(6) 2-((*R*)-3-Amino-piperidin-1-yl)-3-(2-butin-1-yl)-5-[(dibenzo[b,*f*][1,4]oxazepin-11-yl)methyl]-3,5-dihydro-imidazo[4,5-*d*]pyridazin-4-on 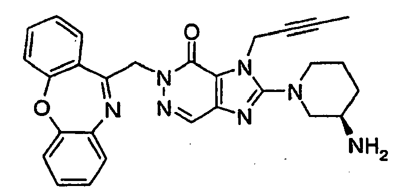 Massenspektrum (ESI⁺): m/z = 494 [M+H]⁺
(7) 2-((R)-3-Amino-piperidin-1-yl)-3-(2-butin-1-yl)-5-[(naphtho[2,1-*d*]oazol-2-yl)methyl]-3,5-dihydro-imidazo[4,5-*d*]pyddazin-4-on 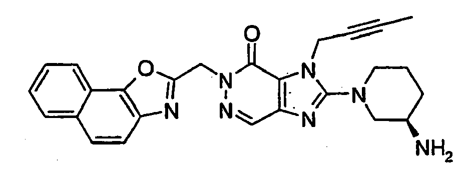 R_{f}-Wert: 0.40 (Kieselgel, Methylenchlorid/Ethanol/konz. wässriges Ammoniak =
   90:10:2)
   Massenspektrum (ESI⁺): m/z = 468 [M+H]⁺
(8) 2-((*R*)-3-Amino-piperidin-1-yl)-3-(2-butin-1-yl)-5-[(naphtho[1,2-*d*]oxazol-2-yl)methyl]-3,5-dihydro-imidazo[4,5-*d*]pyridazin-4-on 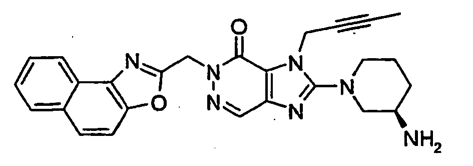 Massenspektrum (ESI⁺): m/z = 468 [M+H]⁺
(9) 2-((*R*)-3-Amino-piperidin-1-yl)-3-(2-butin-1-yl)-5-[(4-oxo-3,4-dihydro-chinazolin-2-yl)methyl]-3,5-dihydro-imidazo[4,5-*d*]pyridazin-4-on 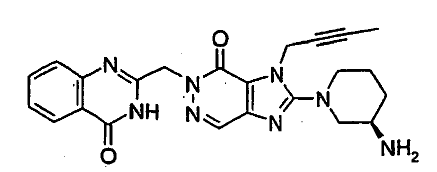 Massenspektrum (ESI⁺): m/z = 445 [M+H]⁺

Analog den vorstehenden Beispielen und anderen literaturbekannten Verfahren können auch die folgenden Verbindungen erhalten werden:

| Nr. | Name | Strukturformel |
|---|---|---|
| (1) | 2-(3-Amino-piperidin-1-yl)-3-(2-buten-1-yl)-5-[(3,4-dihydro-chinolin-2-yl)methyl]-6,7-dimethyl-3,5-dihydro-imidazo[4,5-*c*]pyridin-4-on | 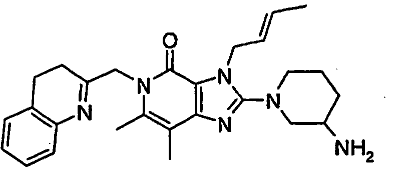 |
| (2) | 2-(3-Amino-piperidin-1-yl)-3-(2-butin-1-yl)-5-[(3,4-dihydro-isochinolin-1-yl)methyl]-7-cyclopropyl-3,5-dihydro-imidazo[4,5-*d*]pyridazin-4-on | 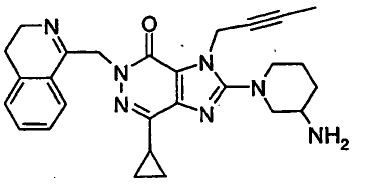 |
| (3) | 2-(3-Amino-piperidin-1-yl)-3-(2-butin-1-yl)-5-[(3,3-dimethyl-3,4-dihydro-isochinolin-1-yl)methyl]-3,5-dihydro-imidazo[4,5-*d*]pyridazin-4-on | 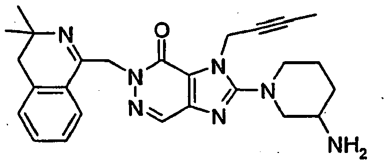 |
| (4) | 2-(3-Amino-piperidin-1-yl)-3-(2-butin-1-yl)-5-[(4,4-dimethy)-3,4-dihydro-isochino)in-1-yl)methyl]-3,5-dihydro-imidazo[4,5-*d*]pyridazin-4-on | 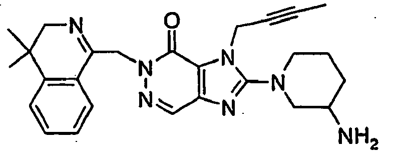 |
| (5) | 2-(3-Amino-piperidin-1-yl)-3-(2-butin-1-yl)-5-[(1-methyl-1,4-dihydro-chinazolin-2-yl)methyl]-3,5-dihydro-imidazo[4,5-*d*]pyridazin-4-on | 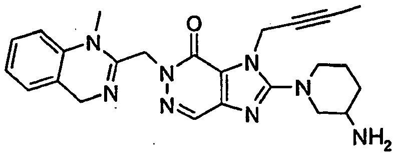 |
| (6) | 2-(3-Amino-piperidin-1-yl)-3-(2-butin-1-yl)-5-[(1-methyl-4-oxo-1,4-dihydro-chinazolin-2-yl)methyl)-3,5-dihydro-imidazo[4,5-*d*]pyridazin-4-on | 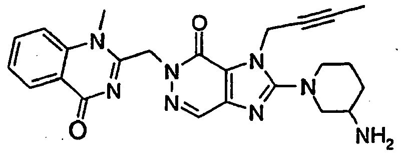 |
| (7) | 2-(3-Amino-piperidin-1-yl)-3-(2-buten-1-yl)-5-[(5,6,7,8-tetrafluoro-1-methyl-1,4-dihydro-chinazolin-2-yl)methyl]-3,5-dihydro-imidazo[4,5-*c*]pyridin-4-on | 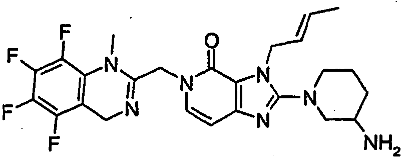 |
| (8) | 2-(3-Amino-piperidin-1-yl)-3-(2-buten-1-yl)-5-[(3,4-dihydro-chinazolin-2-yl)methyl]-. 3,5-dihydro-imidazo[4,5-*d*]pyridazin-4-on | 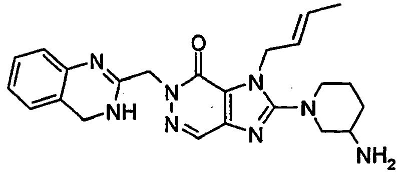 |
| (9) | 2-(3-Amino-piperidin-1-yl)-3-(2-butin-1-yl)-5-[(3-methyl-3,4-dihydro-chinazolin-2-yl)methyl]-3,5-dihydro-imidazo[4,5-*d*]pyridazin-4-on | 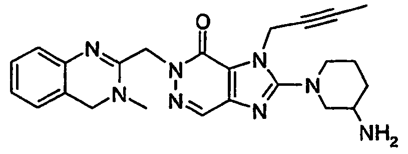 |
| (10) | 2-(3-Amino-piperidin-1-yl)-3-(2-butin-1-yl)-5-[(3-methyl-3,4-dihydro-chinazolin-2-yl)methyl]-3,5-dihydro-imidazo[4,5-*c*]pyridin-4-on | 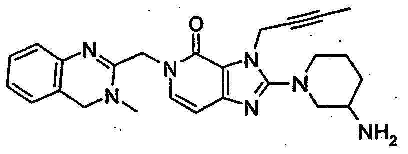 |
| (11) | 2-(3-Amino-piperidin-1-yl)-3-(1-buten-1-yl)-5-[(1*H* benzo[*d*][1,2]oxazin-4-yl)methyl]-3,5-dihydro-imidazo[4,5-*d*]pyridazin-4-on | 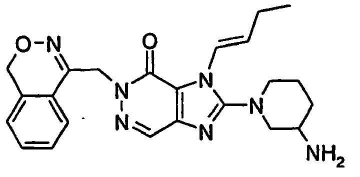 |
| (12) | 2-(3-Amino-piperidin-1-yl)-3-(2-butin-1-yl)-5-[(1-oxo-1 *H*-benzo[*d*][1,2]oxazin-4-yl)methyl]-3,5-dihydro-imidazo[4,5-*d*]pyridazin-4-on | 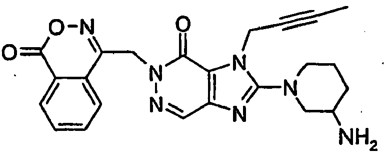 |
| (13) | 2-(3-Amino-piperidin-1-yl)-3-(2-butin-1-yl)-5-[(4*H*-benzo[*e*][1,3]oxazin-2-yl)methyl]-3, 5-dihydro-imidazo[4,5-*d*]pyridazin-4-on | 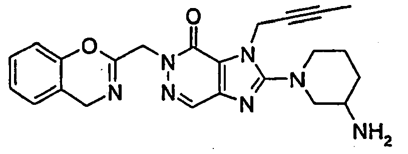 |
| (14) | 2-(3-Amino-piperidin-1-yl)-3-(2-buten-1-yl)-5-[(4,4-dimethyl-4*H*-benzo[*e*][1,3]oxazin-2-yl)methyl]-3,5-dihydro-imidazo[4,5-*d*]pyridazin-4-on | 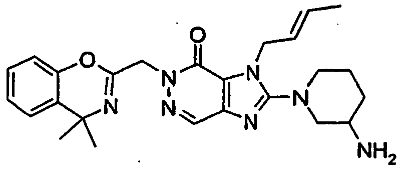 |
| (15) | 2-(3-Amino-piperidin-1-yl)-3-(2-butin-1-yl)-5-[(4-oxo-4*H*-benzo [*e*] [1,3]oxazin-2-yl)methyl]-3,5-dihydro-imidazo[4,5-*d*]pyridazin-4-on | 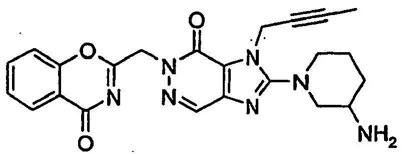 |
| (16) | 2-(3-Amino-piperidin-1-yl)-3-(2-butin-1-yl)-5-[(4*H* benzo[*d*][1,3]oxazin-2-yl)methyl]-3,5-dihydro-imidazo[4,5-*d*]pyridazin-4-on | 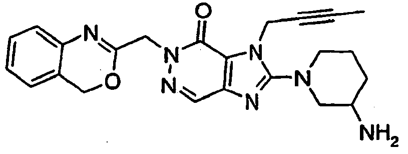 |
| (17) | 2-(3-Amino-piperidin-1-yl)-3-(2-butin-1-yl) 5-[(4*H*-benzo[*d*][1,3]oxazin-2-yl)methyl]-7-methyl-3,5-dihydro-imidazo[4,5-*c*]pyridin-4-on | 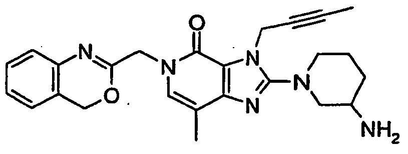 |
| (19) | 2-(3-Amino-piperidin-1-y1)-3-(2-butin-1-yl)-5-[(4,4-dimethyl-4*H*-benzo[*d*][1,3]oxazin-2-yl) methyl]-3,5-dihydro-imidazo[4,5-d]pyridazin-4-on | 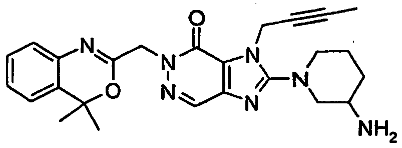 |
| (20) | 2-(3-Amino-piperidin-1-yl)-3-(2-butin-1-yl)-5-[(4-oxo-4*H*-benzo[*d*][1,3]oxazin-2-yl)methyl]-3,5-dihydro-imidazo[4,5-*d*]pyridazin-4-on | 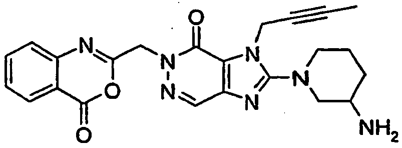 |
| (21) | 2-(3-Amino-piperidin-1-yl)-3-(2-butin-1-yl)-5-[(2*H*-benzo[1,4]oxazin-3-yl)methyl]-3,5-dihydro-imidazo[4,5-*d*]pyridazin-4-on | 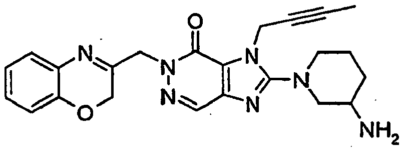 |
| (22) | 2-(3-Amino-piperidin-1-yl)-3-(2-butin-1-yl)-5-[(2-oxo-2*H*-benzo[1,4]oxazin-3-yl)methyl]-3,5-dihydro-imidazo[4,5-*d*]pyridazin-4-on | 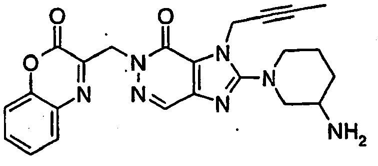 |
| (23) | 2-(3-Amino-piperidin-1-yl)-3-(2-butin-1-yl)-5-[(2,2-dimethyl-2*H*-benzo[1,4]oxazin-3-yl)methyl]-3,5-dihydro-imidazo[4,5-d]pyridazin-4-on | 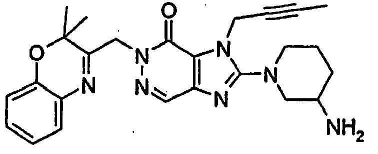 |
| (24) | 2-(3-Amino-piperidin-1-yl)-3-(2-butin-1-yl)-5-[4*H-*benzo[*e*][1,3]thiazin-2-yl)methyl]-3,5-dihydro-imidazo[4,5-*d*]pyridazin-4-on | 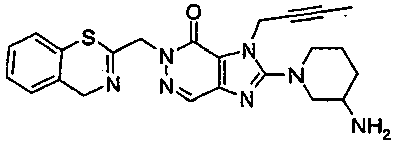 |
| (25) | 2-(3-Amino-piperidin-1-yl)-3-(2-butin-1-yl)-5-[4,4-dimethyl-4*H*-benzo[*e*][1,3]thiazin-2-yl)methyl]-3,5-dihydro-imidazo[4,5-*d*]pjrridazin-4-on | 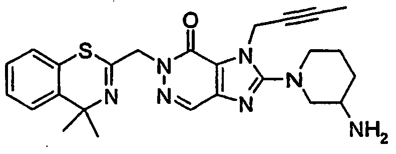 |
| (26) | 2-(3-Amino-piperidin-1-yl)-3-(2-butin-1-yl)-5-[4-oxo-4*H* benzo[*e*][1,3]thiazin-2-yl)methyl]-3,5-dihydro-imidazo[4,5-d]pyridazin-4-on | 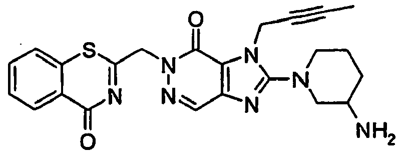 |
| (27) | 2-(3-Amino-piperidin-1-yl)-3-(2-butin-1-yl)-5-[(4*H*-benzo[*d*][1,3]thiazin-2-yl)methyl]-3,5-dihydro-imidazo[4,5-*d*]pyridazin-4-on | 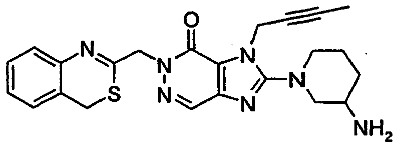 |
| (28) | 2-(3-Amino-piperidin-1-yl)-3-(2-butin-1-yl)-5-[(2*H*-benzo[1,4]thiazin-3-yl)methyl]-3,5-dihydro-imidazo[4,5-*d*]pyridazin-4-on | 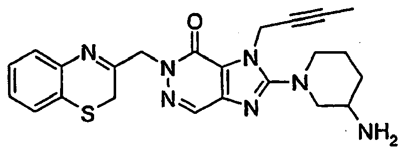 |
| (29) | 2-(3-Amino-piperidin-1-yl)-3-(2-butin-1-yl)-5-[(2-oxo-2*H*-benzo[*e*][1,3]oxazin-4-yl)methyl]-3,5-dihydro-imidazo[4,5-*d*]pyridazin-4-on | 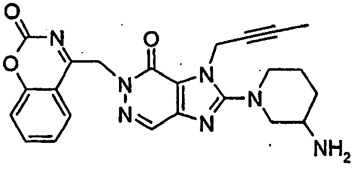 |
| (30) | 2-(3-Amino-piperidin-1-yl)-3-(2-butin-1-yl)-5-[(1-methyl-2, 2-dioxo-1*H*-benzo[*c*][1,2]thiazin-4-yl)methyl]-3,5-dihydro-imidazo[4,5-*d*]pyridazin-4-on | 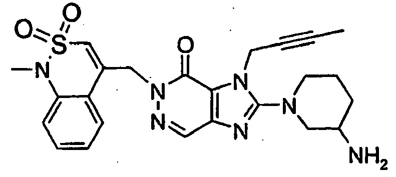 |
| (31) | 2-(3-Amino-piperidin-1-yl)-3-(2-butin-1-yl)-5-[(2,3-dihydro-1*H*-benzo[*e*][1,4]diazepin-5-yl)methyl]-3,5-dihydro-imidazo[4,5-*d*]pyridazin-4-on | 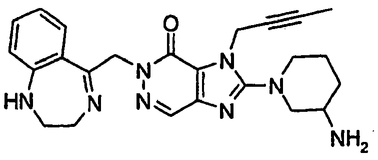 |
| (32) | 2-(3-Amino-piperidin-1-yl)-3-(2-butin-1-yl)-5-[(2-oxo-2,3-dihydro-1*H*-benzo[*e*][1,4] diazepin-5-yl)methyl]-3,5-dihydro-imidazo[4,5-*d*]pyridazin-4-on | 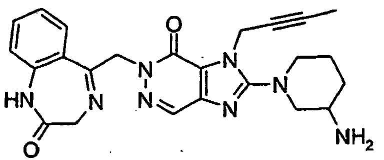 |
| (33) | 2-(3-Amino-piperidin-1-yl)-3-(2-butin-1-yl)-5-[(1-methyl-2,3-dihydro-1*H-*benzo[*e*][1,4]diazepin-5-yl)methyl]-3,5-dihydro-imidazo[4,5-*d*]pyridazin-4-on | 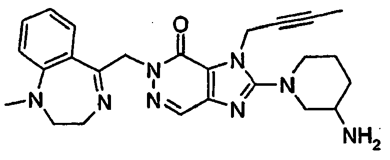 |
| (34) | 2-(3-Amino-piperidin-1-yl)-3-(2-butin-1-yl)-5-[(1-methyl-2-oxo-2,3-dihydro-1*H-*benzo[*e*][1,4]diazepin-5-yl)methyl]-3,5-dihydro-imidazo[4,5-*d*]pyridazin-4-on | 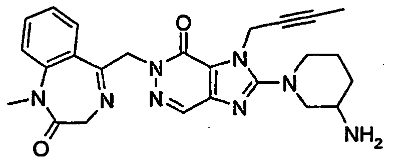 |
| (35) | 2-(3-Amino-piperidin-1-yl)-3-(2-butin-1-yl)-5-[(4-oxo-4,5-dihydro-3*H*-benzo[*b*][1,4]diazepin-2-yl)methyl]-3,5-dihydro-imidazo[4,5-*d*]pyridazin-4-on | 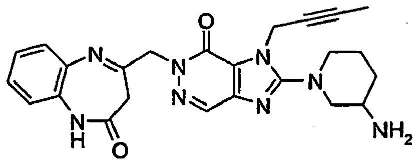 |
| (36) | 2-(3-Amino-piperidin-1-yl)-3-(2-butin-1-yl)-5-[(5-methyl-4-oxo-4, 5-dihydro-3*H*-benzo[*b*][1,4]diazepin-2-yl)methyl]-3,5-dihydro-imidazo[4,5-*d*]pyridazin-4.-on | 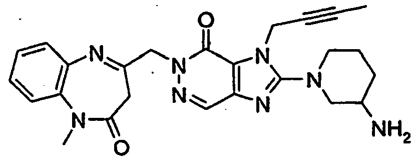 |
| (37) | 2-(3-Amino-piperidin-1-yl)-3-(2-butin-1-yl)-5-[5-oxo-4,5-dihydro-3*H*-benzo[*e*][1,4]diazepin-2-yl)methyl]-3,5-dihydro-imidazo[4,5-*d*]pyridazin-4-on | 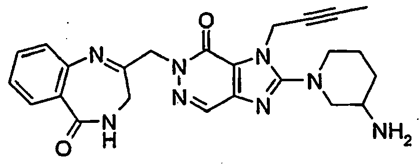 |
| (38) | 2-(3-Amino-piperidin-1-yl)-3-(2-butin-1-yl)-5-[4-methyl-5-oxo-4,5-dihydro-3*H*-benzo[*e*][1,4]diazepin-2-yl)methyl]-3,5-dihydro-imidazo[4,5-*d*]pyridazin-4-on | 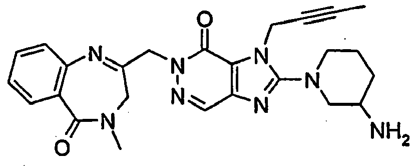 |
| (39) | 2-(3-Amino-piperidin-1-yl)-3-(2-butin-1-yl)-5-[(2,3-dihydro-benzo[*f*][1,4]oxazepin-5-yl)methyl]-3,5-dihydro-imidazo[4,5-*d*]pyridazin-4-on | 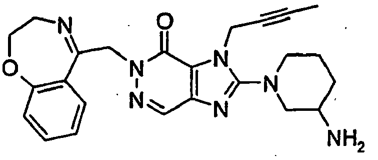 |
| (40) | 2-(3-Amino-piperidin-1-yl)-3-(2-butin-1-yl)-5-[(3,3-dimethyl-2,3-dihydro-benzo[*f*][1,4]oxazepin-5-yl)methyl]-3,5-dihydro-imidazo[4,5-*d*]pyridazin-4-on | 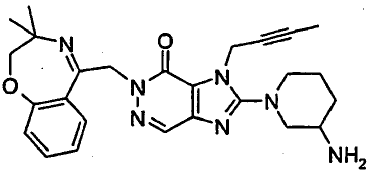 |
| (41) | 2-(3-Amino-piperidin-1-yl)-3-(2-butin-1-yl)-5-[(2,2-dimethyl-2,3-dihydro-benzo[*f*][1,4]oxazepin-5-yl)methyl]-7-methyl-3,5-dihydro-imidazo[4,5-d]-pyridazin-4-on | 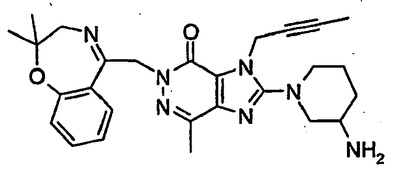 |
| (42) | 2-(3-Amino-piperidin-1-yl)-3-(2-butin-1-yl)-5-[(2,3-dihydro-benzo[b][1,4]oxazepin-4-yl)methyl]-7-methyl-3,5-dihydro-imidazo[4,5-*d*]pyridazin-4-on | 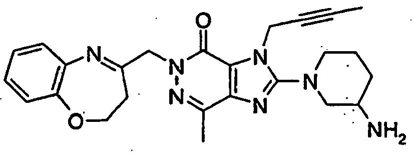 |
| (43) | 2-(3-Amino-piperidin-1-yl)-3-(2-butin-1-yl)-5-[(6,6-dimethyl-2,3-dihydro-benzo[*b*][1,4]oxazepin-4-yl)methyl]-7-methyl-3,5-dihydro-imidazo[4,5-*d*]pyridazin-4-on | 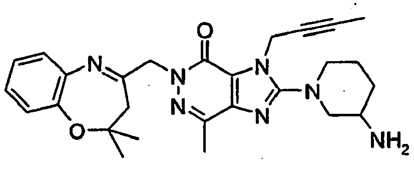 |
| (44) | 2-(3-Amino-piperidin-1-yl)-3-(2-butin-1-yl)-5-[(2,3-dihydro-benzo[*b*][1,4]thiazepin-4-yl)methyl]-7-methyl-3,5-dihydro-imidazo[4,5-*d*]pyridazin-4-on | 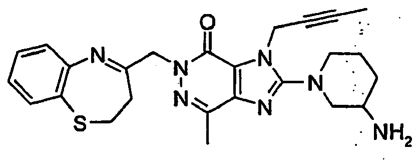 |
| (45) | 2-(3-Amino-piperidin-1-yl)-3-(2-butin-1-yl)-5-[(2,2-dimethyl-2,3-dihydro-benzo[*b*][1,4]thiazepin-4-yl)methyl]-7-methyl-3,5-dihydro-imidazo[4,5-d]pyridazin-4-on | 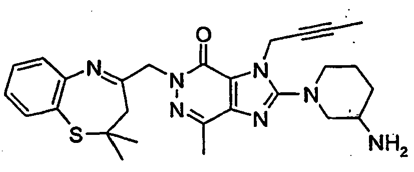 |
| (46) | 2-(3-Amino-piperidin-1-yl)-3-(2-butin-1-yl)-5-[(2,3-dihydro-benzo[*f*][1,4]thiazepin-5-yl)methyl]-7-methyl-3,5-dihydro-imidazo[4,5-*d*]pyridazin-4-on | 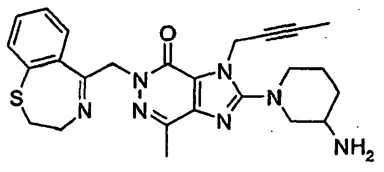 |
| (47) | 2-(3-Amino-piperidin-1-yl)-3-(2-butin-1-yl)-5-[(5-oxo-4,5-dihydro-benzo[*f*] [1,3;4]oxadiazepin-2-yl)methyl]-7-methyl-3,5-dihydro-imidazo[4,5-*d*]pyridazin-4-on | 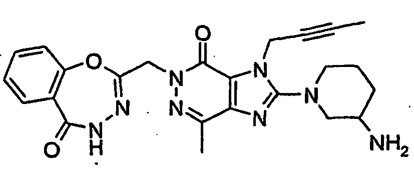 |
| (48) | 2-(3-Amino-piperidin-1-yl)-3-(2-butin-1-yl)-5-[(11*H* dibenzo[*b,e*]azepin-6-yl)methyl]-7-ethyl-3,5-dihydro-imidazo[4,5-*d*]pyridazin-4-on | 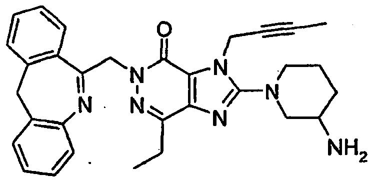 |
| (49) | 2-(3-Amino-piperidin-1-yl)-3-(2-butin-1-yl)-5-[(11*H*-dibenzo[*b,e*]azepin-6-yl)methyl]-7-cyanmethyl-3,5-dihydro-imidazo[4,5-*d*]pyridazin-4-on | 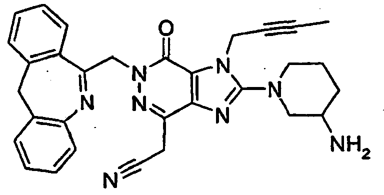 |
| (50) | 2-(3-Amino-piperidin-1-yl)-3-(2-butin-1-yl)-5-[(11,11-difluoro-11*H*-dibenzo[*b*,*e*]aze-pin-6-yl)methyl]-3,5-dihydro-imidazo[4,5-*c*]pyridin-4-on | 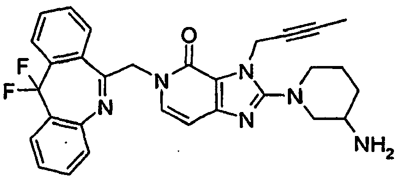 |
| (51) | 2-(3-Amino-piperidin-1-yl)-3-(2-butin-1-yl)-5-[(11-oxo-11*H*-dibenzo[*b*,*e*]azepin-6-yl)methyl]-7-(2-cyanethyl)-3,5-dihydro-imidazo[4,5-*d*]pyridazin-4-on | 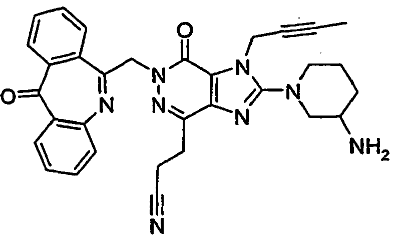 |
| (52) | 2-(3-Amino-piperidin-1-yl)-3-(1-buten-1-yl)-5-[(11*H*-benzo[*e*]pyrido[3,2-b]azepin-6-yl)methyl]-7-methyl-3,5-dihydro-imidazo-[4,5-*d*]pyridazin-4-on | 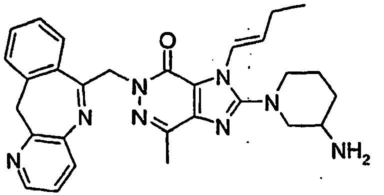 |
| (53) | 2-(3-Amino-piperidin-1-yl-(2-butin-1-yl)-5-[(5*H*-1,9,10-triaza-dibenzo[*a,d*]cyclo-hepten-11-yl)methyl]-3,5-dihydro-imidazo[4,5-c]pyridin-4-on | 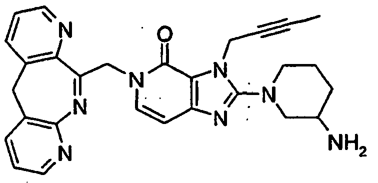 |
| (54) | 2-(3-Amino-piperidin-1-yl)-3-(2-buten-1-yl)-5-[(5-methyl-5*H*-dibenzo[*b,e*][1,4]-diazepin-11-yl)methyl]-7-methyl-3,5-dihydro-imidazo[4,5-*d*]pyridazin-4-on | 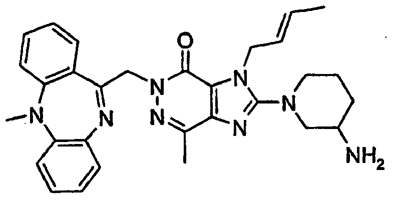 |
| (55) | 2-(3-Amino-piperidin-1-yl)-3-(1 -buten-1 -yl)-5-[(dibenzo[*b*,*f*][1,4]oxazepin-11-yl)methyl]-3,5-dihydro-imidazo[4,5-*c*]pyridin-4-on | 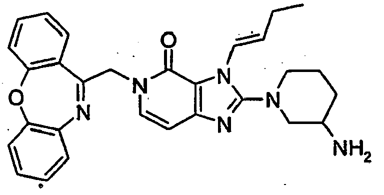 |
| (56) | 2-(3-Amino-piperidin-1-yl)-3-(2-butin-1-yl)-5-[(dibenzo[*b*,*f*][1,4]thiazepin-11-yl)methyl]-7-methyl-3,5-dihydro-imidazo[4,5-*d*]pyridazin-4-on | 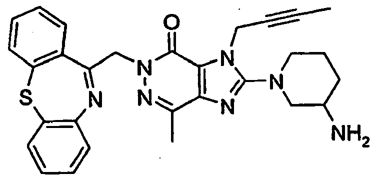 |
| (57) | 2-(3-Amino-piperidin-1-yl)-3-(1-buten-1-yl)-5-[(dibenzo[*b,f*][1,4]thiazepin-11-yl)methyl]-3,5-dihydro-imidazo[4,5-c]pyridin-4-on | 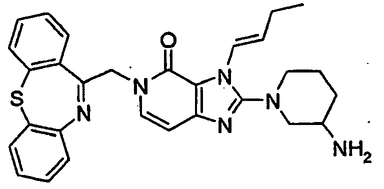 |
| (58) | 2-(3-Amino-piperidin-1-yl)-3-(2-butin-1-yl)-5-[(5-oxo-dibenzo[*b*,*f*][1,4]thiazepin-11-yl)methyl]-7-methyl-3,5-dihydro-imidazo[4,5-*d*]pyridazin-4-on | 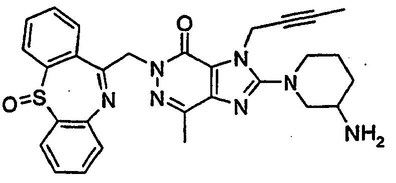 |
| (59) | 2-(3-Amino-piperidin-1-yl)-3-(2-butin-1-yl)-5-[(5,5-dioxo-dibenzo[*b*,*f*][1,4]thiazepin-11-yl)methyl]-7-methyl-3,5-dihydro-imidazo[4,5-*d*]pyridazin-4-on | 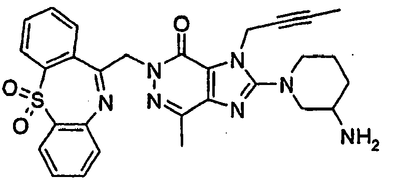 |
| (60) | 2-(3-Amino-piperidin-1-yl)-3-(2-butin-1-yl)-5-[(5*H*-dibenzo[*a*,*d*]cyclohepten-10-yl)methyl]-7-methyl-3, 5-dihydro-imidazo[4,5-*d*]pyridazin-4-on | 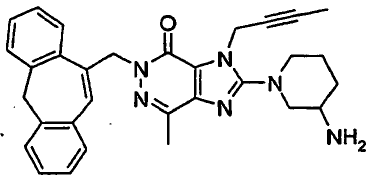 |
| (61) | 2-(3-Amino-piperid in-1-yl)-3-(2-butin-1-yl)-5-[(5-methyl-5*H*-dibenzo[*b,f*]azepin-10-yl)methyl]-7-trifluormethyr-3,5-dihydro-imidazo[4,5-*d*]pyridazin-4-on | 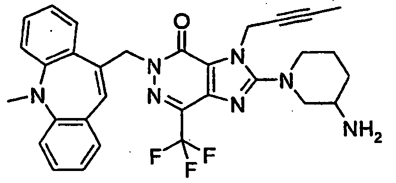 |
| (62) | 2-(3-Amino-piperidin-1-yl)-3-(3-methyl-2-buten-1-yl)-5-[(phenanthridin-6-yl)methyl]-7-methyl-3,5-dihydro-imidazo[4,5-d]pyridazin-4-on | 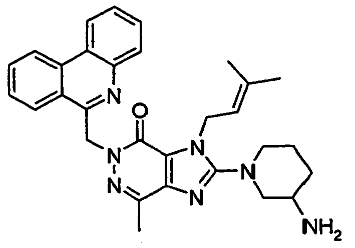 |
| (63) | 2-(3-Amino-piperidin-1-yl)-3-(2-buten-1-yl)-5-[(phenanthridin-6-yl)methyl]-7-methyl-3,5-dihydro-imidazo[4,5-d]pyridazin-4-on | 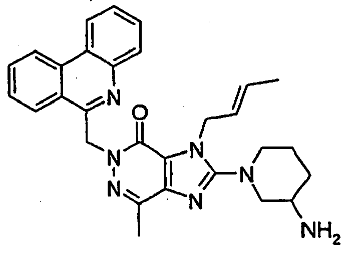 |
| (64) | 2-(3-Amino-piperidin-1-yl)-3-(1-buten-1-yl)-5-[(phenanthridin-6-yl)methyl]-7-methyl-3,5-dihydro-imidazo[4,5-*d*]pyridazin-4-on | 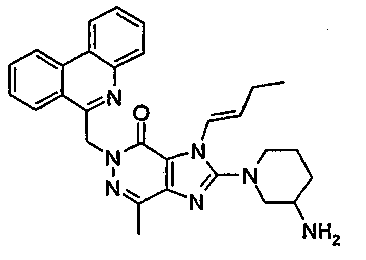 |
| (65) | 2-(3-Amino-piperidin-1-yl)-3-[(1-cyclopenten-1-yl)methyl]-5-[(phenanthridin-6-yl)methyl]-7-methyl-3,5-dihydro-imidazo[4,5-*d*]pyridazin-4-on | 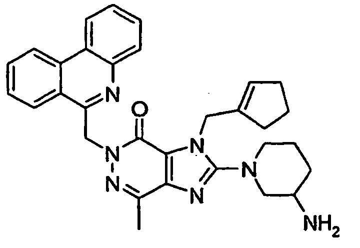 |
| (66) | 2-(3-Amino-piperidin-1-yl)-3-(1-buten-1-yl)-5-[(phenanthridin-6-yl)methyl]-3,5-dihydro-imidazo[4,5-*c*]pyridin-4-on | 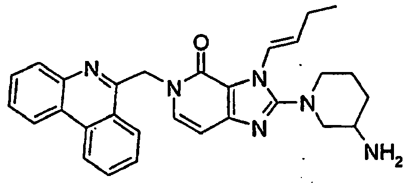 |
| (67) | 2-(3-Amino-piperidin-1-yl)-3-(2-butin-1-yl)-5-[(benzo[*c*][1,5]naphthyridin-6-yl)methyl]-7-cyclopropyl-3,5-dihydro-imidazo[4,5-*d*]pyridazin-4-on | 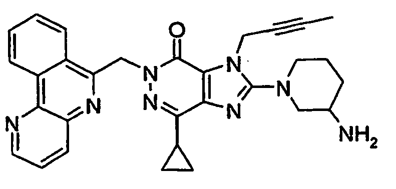 |
| (68) | 2-(3-Amino-piperidin-1-yl)-3-(2-butin-1-yl)-5-[(benzo[*h*][1,6]naphthyridin-5-yl)methyl]-3,5-dihydro-imidazo[4,5-*d*]pyridazin-4-on | 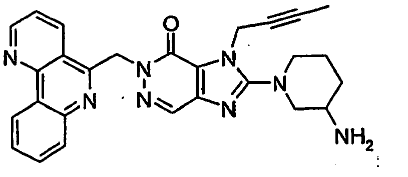 |
| (69) | 2-(3-Amino-piperidin-1-yl)-3-(2-butin-1-yl)-5-[(benzo[*c*][1,8]naphthyridin-6-yl)methyl]-3,5-dihydro-imidazo[4,5-*d*]pyridazin-4-on | 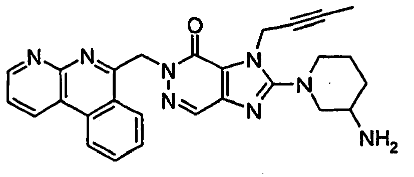 |
| (70) | 2-(3-Amino-piperidin-1-yl)-3-(2-butin-1-yl)-5-[(5-benzo[*f*][1,7]naphthyridin-5-yl)methyl]-3,5-dihydro-imidazo[4,5-*c*]pyridin-4-on | 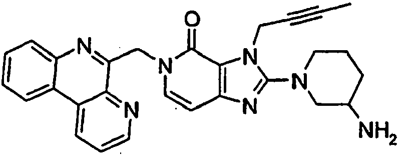 |
| (71) | 2-(3-Amino-piperidin-1-yl)-3-(2-butin-1-yl)-5-[(1,5,9-triaza-phenanthren-10-yl)methyl]-3,5-dihydro-imidazo[4,5-*c*]pyridin-4-on | 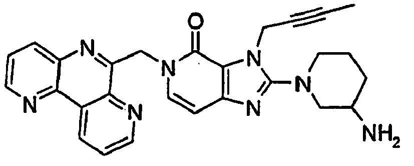 |
| (72) | 2-(3-Amino-piperidin-1-yl)-3-(2-butin-1-yl)-5-[(1,2,3,4-tetrahydrophenanthridin-6-yl)methyl]-3,5-dihydro-imidazo[4,5-*c*]pyridin-4-on | 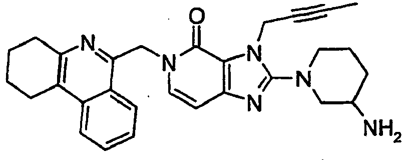 |
| (73) | 2-(3-Amino-piperidin-1-yl)-3-(2-butin-1-yl)-5-[(1,2,3,4,4a,10b-hexahydro-phenanthridin-6-yl)methyl]-3,5-dihydro-imidazo[4,5-*c*]pyridin-4-on | 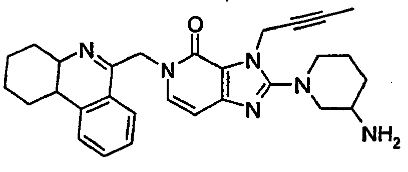 |
| (74) | 2-(3-Amino-piperidin-1-yl)-3-(2-butin-1-yl)-5-[(2,3-dihydro-1*H*-4-aza-cyclopenta[a]naphth-5-yl)methyl]-3,5-dihydro-imidazo[4,5-*c*]pyridin-4-on | 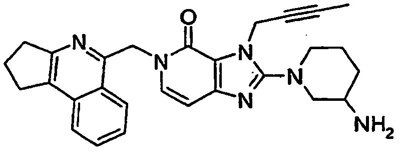 |
| (75) | 2-(3-Amino-piperidin-1-yl)-3-(2-butin-1-yl)-5-[(8, 9,10,11-tetrahydro-7*H*-6-aza-cyclohepta[*a*]naphth-5-yl)methyl]-3,5-dihydro-imidazo[4,5-*c*]pyridin-4-on | 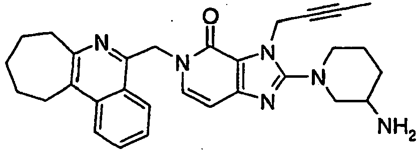 |
| (76) | 2-(3-Amino-piperidin-1-yl)-3-(2-butin-1-yl)-5-[(2,3-dihydro-1*H*-4-oxa-10-aza-phenanthren-9-yl)methyl]-3,5-dihydro-imidazo[4,5-*c*]pyridin-4-on | 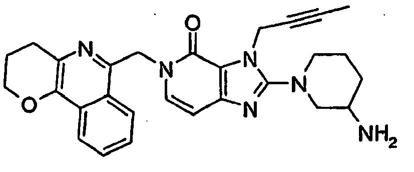 |
| (77) | 2-(3-Amino-piperidin-1-yl)-3-(2-butin-1-yl)-5-[(1-oxo-2,3-dihydro-1*H*-4-oxa-10-aza-phenanthren-9-yl)methyl]-3,5-dihydro-imidazo[4,5-*c*]pyridin-4-on | 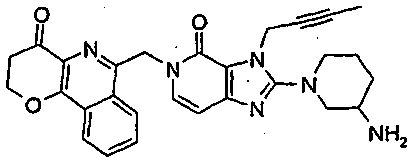 |
| (78) | 2-(3-Amino-piperidin-1-yl)-3-(2-butin-1-yl)-5-[(10-cyanophenanthren-9-yl)methyl]-3,5-dihydro-imidazo[4,5-*c*]pyridin-4-on | 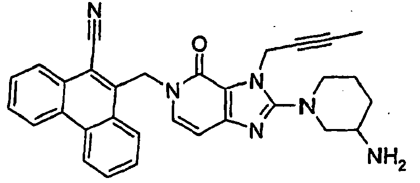 |
| (79) | 2-(3-Amino-piperidin-1-yl)-3-(2-butin-1-yl)-5-[(benzo[*h*]chinolin-6-yl)methyl]-3,5-dihydro-imidazo[4,5-*c*]pyridin-4-on | 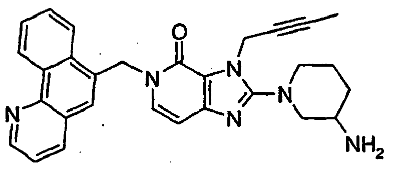 |
| (80) | 2-(3-Amino-piperidin-1-yl)-3-(2-butin-1-yl)-5-[(benzo[*f*]chinolin-6-yl)methyl]-3,5-dihydro-imidazo[4,5-*d*]pyridazin-4-on | 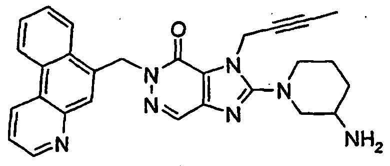 |
| (81) | 2-(3-Amino-piperidin-1-yl)-3-(2-butin-1-yl)-5-[(benzo[*f*]chinoxalin-6-yl)methyl]-3,5-dihydro-imidazo[4,5-*d*]pyridazin-4-on | 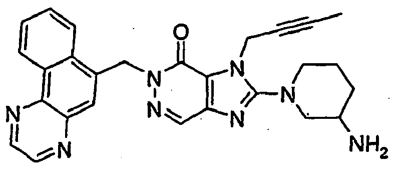 |
| (82) | 2-(3-Amino-piperidin-1-yl)-3-(2-butin-1-yl)-5-[(5*H*-benzo[*e*]pyrrolo[1,2-*a*][1,4]diaze-pin-11-yl)methyl]-7-methyl-3,5-dihydro-imidazo[4,5-*d*]pyridazin-4-on | 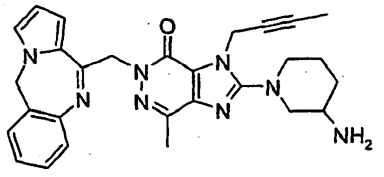 |
| (83) | 2-(3-Amino-piperidin-1-yl)-3-(2-butin-1-yl)-5-[(thieno[3,2-*b*][1,4]benzoxazepin-9-yl)methyl]-7-trifluormethyl-3,5-dihydro-imidazo[4,5-*d*]pyridazin-4-on | 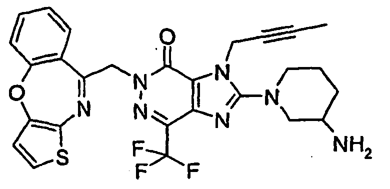 |
| (84) | 2-(3-Amino-piperidin-1-yl)-3-(2-butin-1-yl)-5-[(thieno[3,2-b][1,4]benzoxazepin-9-yl)methyl]-3,5-dihydro-imidazo[4,5-*c*]pyridin-4-on | 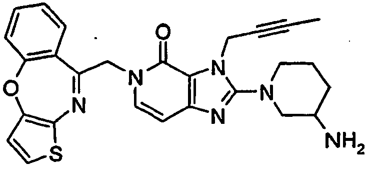 |
| (85) | 2-(3-Amino-piperidin-1-yl)-3-(2-butin-1-yl)-5-[(5*H*-dibenzo[*d*,*f*][1,3]diazepin-6-yl)methyl]-7-methyl-3,5-dihydro-imidazo[4,5-*d*]pyridazin-4-on | 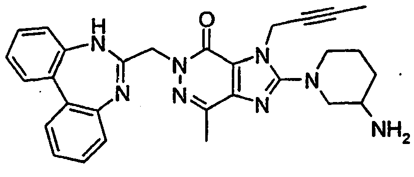 |
| (86) | 2-(3-Amino-piperidin-1-yl)-3-(2-butin-1-yl)-5-[(5-methyl-5*H*-dibenzo[*d,f*][1,3]diazepin-6-yl)methyl]-3,5-dihydro-imidazo[4,5-c]pyridin-4-on | 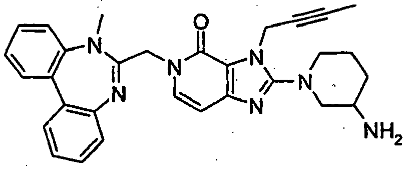 |
| (87) | 2-(3-Amino-piperidin-1-yl)-3-(3-methylbut-2-en-1-yl)-5-[(5-oxa-7-aza-dibenzo[*a,c*]cyclohepten-6-yl)methyl]-7-methyl-3,5-dihydro-imidazo[4,5-*c*]pyridin-4-on | 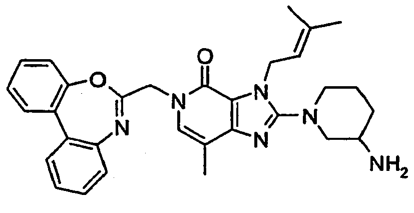 |
| (88) | 2-(3-Amino-piperidin-1-yl)3-(2-butin-1-yl)-5-[(naphtho[1,2-d]thiazol-2-yl)methyl]-3,5-dihydro-imidazo[4,5-*d*]pyridazin-4-on | 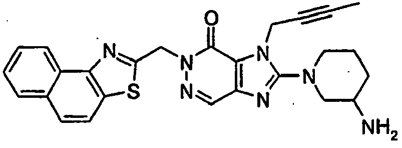 |
| (89) | 2-(3-Amino-piperidin-1-yl)-3-(2-butin-1-yl)-5-[(naphtho[2,1-*d*]thiazol-2-yl)methyl]-3,5-dihydro-imidazo[4,5-*d*]pyridazin-4-on | 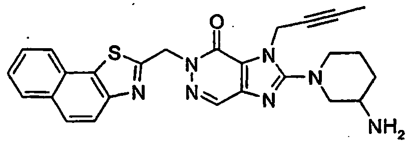 |
| (90) | 2'-(3-Amino-piperidin-1-yl)-3-(2-butin-1-yl)-5-[(3*H*-naphtho[1,2-*d*]imidazol-2 -yl)methyl]-3,5-dihydro-imidazo[4,5-*d*]pyridazin-4-on | 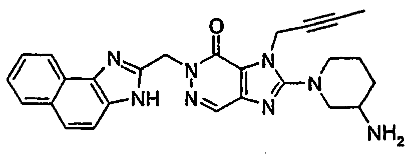 |
| (91) | 2-(3-Amino-piperidin-1-yl)-3-(2-butin-1-yl)-5-[(naphtho[1,2-*b*]furan-2-yl)methyl]-3,5-dihydro-imidazo[4,5-*d*]pyridazin-4-on | 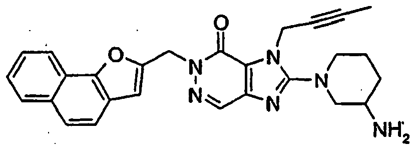 |
| (92) | 2-(3-Amino-piperidin-1-yl)-3-(2-butin-1-yl)-5-[(naphtho[2,1-*b*]furan-2-yl)methyl]-3,5-dihydro-imidazo[4,5-*d*]pyridazin-4-on | 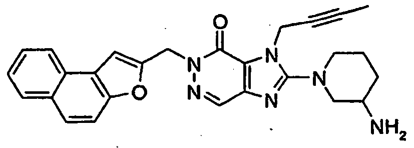 |
| (93) | 2-(3-Amino-piperidin-1-yl)-3-(2-butin-1-yl)-5-[(2-methyl-furo[3,2-*c*]isochinolin-5-yl)methyl]-3,5-dihydro-imidazo[4,5-*c*]pyridin-4-on | 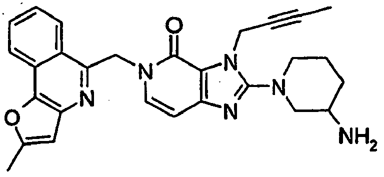 |
| (94) | 2-(3-Amino-piperidin-1-yl)-3-(2-butin-1-yl)-5-[(pyrazolo[1,5-*c*]chinazolin-5-yl)methyl]-3,5-dihydro-imidazo[4,5-*d*]pyridazin-4-on | 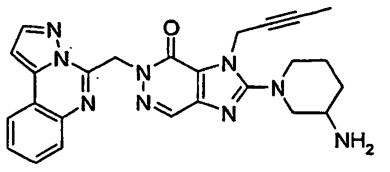 |
| (95) | 2-(3-Amino-piperidin-1-yl)-3-(2-butin-1-yl)-5-[(1*H*-perimidin-2-yl)methyl]-3,5-dihydro-imidazo[4,5-*d*]pyridazin-4-on | 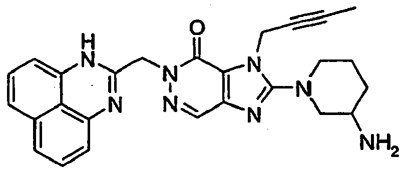 |

### Beispiel 2

### Dragées mit 75 mg Wirksubstanz

1 Dragéekern enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Drageekerne werden mit einem Film überzogen, der im wesentlichen aus Hydroxypropylmethylcellulose besteht. Die fertigen Filmdragees werden mit Bienenwachs geglänzt.
Dragéegewicht: 245 mg.

### Beispiel 3

### Tabletten mit 100 mg Wirksubstanz

Zusammensetzung:

**1 Tablette enthält:**

| | |
|---|---|
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2.0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel 4

### Tabletten mit 150 mg Wirksubstanz

Zusammensetzung:

**1 Tablette enthält:**

| | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 5

### Hartgelatine-Kapseln mit 150 mg Wirksubstanz

**1 Kapsel enthält:**

| | |
|---|---|
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt. Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

### Beispiel 6

### Suppositorien mit 150 mg Wirksubstanz

**1 Zäpfchen enthält:**

| | |
|---|---|
| Wirkstoff | 150,0 mg |
| Polyethylenglykol 1500 | 550,0 mg |
| Polyethylenglykol 6000 | 460,0 mg |
| Polyoxyethylensorbitanmonostearat | 840,0 mg |
| | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 7

### Suspension mit 50 mg Wirksubstanz

**100 ml Suspension enthalten:**

| | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 8

### Ampullen mit 10 mg Wirksubstanz

Zusammensetzung:

| | |
|---|---|
| Wirkstoff | 10,0 mg |
| 0,01 n Salzsäure s.q. | |
| Aqua bidest | ad 2,0 ml |

### Herstellung:

Die Wirksubstanz wird in der erforderlichen Menge 0,01 n HCI gelöst, mit Kochsalz isotonisch gestellt, sterilfiltriert und in 2 ml Ampullen abgefüllt.

### Beispiel 9

### Ampullen mit 50 mg Wirksubstanz

Zusammensetzung:

| | |
|---|---|
| Wirkstoff | 50,0 mg |
| 0,01 n Salzsäure s.q. | |
| Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

## Patentansprüche

1. Verbindungen der allgemeinen Formel 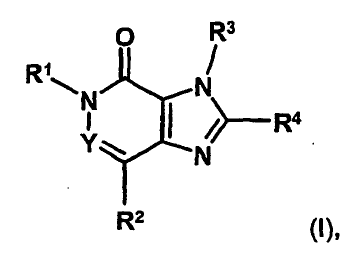 in der
R¹ eine durch eine Gruppe Rₐ substituierte C₁₋₃-Alkylgruppe, wobei
Rₐ eine 3,4-Dihydro-chinolinyl-, 3,4-Dihydro-isochinolinyl-, 1,4-Dihydro-chinazolinyl-, 3,4-Dihydro-chinazolinyl-, 1*H*-Benzo[*d*][1,2]oxazinyl-, 4*H-*Benzo[*e*][1,3]oxazinyl-, 4*H*-Benzo[*d*][1,3]oxazinyl- oder 2*H*-Benzo[1,4]oxazinyl-gruppe, in der jeweils
im Benzoteil eine bis drei Methingruppen jeweils durch ein Stickstoffatom ersetzt sein können und im Heterocyclylteil eine Methylengruppe durch eine Carbonylgruppe ersetzt sein kann,
eine 4*H*-Benzo[*e*][1,3]thiazinyl-, 4*H*-Benzo[*d*][1,3]thiazinyl- oder 2*H*-Benzo [1,4]thiazinylgruppe, in der jeweils
im Benzoteil eine bis drei Methingruppen jeweils durch ein Stickstoffatom ersetzt sein können und im Heterocyclylteil eine Methylengruppe durch eine Carbonylgruppe ersetzt sein kann sowie das Schwefelatom durch eine Sulfinyl- oder Sulfonylgruppe ersetzt sein kann,
eine 2-Oxo-2*H*-benzo[*e*][1,3]oxazinyl- oder 2,2-Dioxo-1*H*-benzo[*c*][1,2]thiazinylgruppe, in der jeweils im Benzoteil
eine bis drei Methingruppen jeweils durch ein Stickstoffatom ersetzt sein können,
eine 2,3-Dihydro-1*H*-benzo[*e*][1,4]diazepinyl-, 4,5-Dihydro-3*H*-benzo[*b*][1,4]diazepinyl- oder 5-Oxo-4,5-dihydro-3*H*-benzo[*e*][1,4]diazepinylgruppe, in der jeweils
im Benzoteil eine bis drei Methingruppen jeweils durch ein Stickstoffatom ersetzt sein können und im Heterocyclylteil eine Methylengruppe durch eine Carbonylgruppe ersetzt sein kann,
eine 2,3-Dihydro-benzo[*f*][1,4]oxazepinyl- oder 2,3-Dihydro-benzo[*b*][1,4]-oxazepinylgruppe, in der jeweils
im Benzoteil eine bis drei Methingruppen jeweils durch ein Stickstoffatom ersetzt sein können und im Heterocyclylteil eine Methylengruppe durch eine Carbonylgruppe ersetzt sein kann,
eine 2,3-Dihydro-benzo[b][1,4]thiazepinyl- oder 2,3-Dihydro-benzo[t][1,4]-thiazepinylgruppe, in der jeweils
im Benzoteil eine bis drei Methingruppen jeweils durch ein Stickstoffatom ersetzt sein können und im Heterocyclylteil eine Methylengruppe durch eine Carbonylgruppe ersetzt sein kann und das Schwefelatom durch eine Sulfinyl- oder Sulfonylgruppe ersetzt sein kann,
eine 5-Oxo-4,5-dihydro-benzo[*f*][1,3,4]oxadiazepinylgruppe, in der
im Benzoteil eine bis drei Methingruppen jeweils durch ein Stickstoffatom ersetzt sein können,
eine 11*H*-Dibenzo[*b,e*]azepinyl- oder 5H-Dibenzo[a,d]cycloheptenylgruppe, in der jeweils
im Benzoteil eine bis drei Methingruppen jeweils durch ein Stickstoffatom ersetzt sein können und die Methylengruppe im Heterocyclylteil durch ein Sauerstoff- oder Schwefelatom, eine Carbonyl-, Sulfinyl- oder Sulfonylgruppe oder durch eine durch Rₓ substituierte Iminogruppe ersetzt sein kann, wobei
Rₓ ein Wasserstoffatom oder eine C₁₋₄-Alkyl-, C₂₋₄-Alkenyl-, C₂₋₄-Alkinyl-, C₃₋₆-Cycloalkyl-, C₃₋₆-Cycloalkyl-C₁₋₃-alkyl-, Aryl-, Aryl-C₁₋₃-alkyl-, Hydroxy-C₂₋₄-alkyl-, C₁₋₃-Alkyloxy-C₂₋₄-alkyl-, C₃₋₆-Cycloalkyloxy-C₂₋₄-alkyl-, Amino-C₂₋₄-alkyl-, C₁₋₃-Alkylamino-C₂₋₄-alkyl, Di-(C₁₋₃-alkyl)-amino-C₂₋₄-alkyl-, C₁₋₃-Alkyl-carbonyl-, C₁₋₃-Alkyloxy-carbonyl-, C₁₋₃-Alkyloxy-carbonyl-C₁₋₃-alkyl-, Aryl-carbonyl-, C₁₋₃-Alkyl-sulfonyl- oder Aryl-sulfonylgruppe darstellt,
eine Phenanthridinylgruppe, in der
im Benzoteil eine bis drei Methingruppen jeweils durch ein Stickstoff atom ersetzt sein können, und
eine 1,2,3,4-Tetrahydro-phenanthridinyl-, 1,2,3,4,4a,10b-Hexahydro-phenanthridinyl-, 2,3-Dihydro-1*H*-4-aza-cyclopenta[*a*]naphthyl- oder eine 8,9,10,11-Tetrahydro-7*H*-6-aza-cyclohepta[*a*]naphthylgruppe, in der jeweils
im Benzoteil eine bis drei Methingruppen jeweils durch ein Stickstoffatom ersetzt sein können und ein oder zwei Methylengruppen jeweils durch ein Sauerstoffatom oder eine Carbonylgruppe ersetzt sein können, wobei, falls zwei Methylengruppen jeweils durch ein Sauerstoffatom ersetzt sind, die Sauerstoffatome durch mindestens zwei Methyleneinheiten voneinander getrennt sein müssen,
eine Phenanthrenylgruppe, in der jeweils eine bis drei der Methingruppen in Position 1 bis 4 und 5 bis 8 durch jeweils ein Stickstoffatom ersetzt sein können,
eine 1,2,3,4-Tetrahydro-phenanthrenyl- oder eine 1,2,3,4,5,6,7,8-Octahydrophenanthrenylgruppe, in der
jeweils ein oder zwei der Methylengruppen in Position 1 bis 4 und 5 bis 8 durch jeweils ein Sauerstoffatom oder eine Carbonylgruppe ersetzt sein können, wobei, falls zwei Methylengruppen durch jeweils ein Sauerstoffatom ersetzt sind, die Sauerstoffatome durch mindestens zwei Methyleneinheiten voneinander getrennt sein müssen,
eine 5*H*-Benzo[*e*]pyrrolo[1,2-*a*][1,4]diazepinyl-, Thieno[3,2-*b*][1,4]benzoxazepinyl-, 5*H*-Dibenzo[*d*,*f*][1,3]diazepinyl- oder eine 5-Oxa-7-aza-dibenzo[*a,c*]-cycloheptenylgruppe, in der jeweils
im Benzoteil eine bis drei Methingruppen durch jeweils ein Stickstoffatom ersetzt sein können,
eine Naphtho[1,2-*d*]oxazolyl-, Naphtho[2,1-*d*]oxazolyl -, Naphtho[1,2-*d*]thiazolyl-, Naphtho[2,1-*d*]thiazolyl-, Naphtho[1,2-*d*]imidazolyl-, Naphtho[1,2-*b*]furanyl-oder Naphtho[2,1-b]furanylgruppe, in der jeweils
im Naphthylteil eine bis drei Methingruppen durch jeweils ein Stickstoffatom ersetzt sein können,
oder eine Furo[3,2-*c*]isochinolinyl-, Pyrazolo[1,5-*c*]chinazotinyl- oder 1*H-*Perimidinylgruppe bedeutet,
wobei die Methylen- und Methingruppen der vorstehend erwähnten Reste Rₐ durch die Gruppen R¹⁰ bis R¹³ und zusätzlich durch eine C₁₋₃-Alkylgruppe substituiert sein können und die Iminogruppen der vorstehend erwähnten
Reste Rₐ durch die wie vorstehend definierten Reste Rₓ substituiert sein können und
R¹⁰ ein Wasserstoffatom,
ein Fluor-, Chlor-, Brom- oder lodatom,
eine C₁₋₄ Alkyl-, Hydroxy-, oder C₁₋₄ Alkyloxygruppe,
eine Nitro-, Amino-, C₁₋₃-Alkylamino-, Di-(C₁₋₃-alkyl)amino-, Cyan-C₁₋₃-alkylamino-, N-(Cyan-C₁₋₃-alkyl)-N-(C₁₋₃-alkyl)-amino-, C₁₋₃-Alkyloxycarbonyl-C₁₋₃-alkylamino-, Pyrrolidin-1-yl-, Piperidin-1-yl-, Morpholin-4-yl-, Piperazin-1-yl-, oder 4-(C₁₋₃-Alkyl)-piperazin-1-yl-Gruppe,
eine C₁₋₃-Alkyl-carbonylamino-, Arylcarbonylamino-, Aryl-C₁₋₃-alkyl-carbonylamino-, C₁₋₃-Alkyloxy-carbonylamino-, Aminocarbonylamino-, C₁₋₃-Alkylaminocarbonylamino-, Di-(C₁₋₃-alkyl)aminocarbonylamino-, Pyrrolidin-1-yl-carbonylamino-, Piperidin-1-yl-carbonylamino-, Morpholin-4-yl-carbonylamino-, Piperazin-1-yl-carbonylamino- oder 4-(C₁₋₃-Alkyl)-piperazin-1-yl-carbonylamino-, C₁₋₃-Alkyl-sulfonylamino-, Bis-(C₁₋₃-alkylsulfonyl)-amino-, Aminosulfonylamino-,C₁₋₃-Alkylamino-sulfonylamino-, Di-(C₁₋₃-alkyl)amino-sulfonylamino-, Pyrrofidin-1-yl-sulfonylamino-, Piperidin-1-yl-sulfonylamino-, Morpholin-4-yl-sulfonylamino-, Piperazin-1-yl-sulfonylamino- oder 4-(C₁₋₃-Alkyl)-piperazin-1-yl-sulfonylamino-, (C₁₋₃-Alkylamino)thiocarbonylamino-, (C₁₋₃-Alkyloxy-carbonylamino)carbonylamino-, Arylsulfonylamino- oder Aryl-C₁₋₃-alkyl-sulfonylaminogruppe,
eine N-(C₁₋₃-Alkyl)-C₁₋₃-alkyl-carbonylamino-, N-(C₁₋₃-Alkyl)-arylcarbonylamino-, N-(C₁₋₃-Alkyl)-aryl-C₁₋₃-alkyl-carbonylamino-, N-(C₁₋₃-Alkyl)-C₁₋₃-alkyloxy-carbonylamino-, N-(Aminocarbonyl)-C₁₋₃-alkylamino-, N-(C₁₋₃-Alkyl-aminocarbonyl)-C₁₋₃-alkylamino -, N-[Di-(C₁₋₃-alkyl)aminocarbonyl]-C₁₋₃-alkylamino-, N-(C₁₋₃-Alkyl)-C₁₋₃-alkyl-sulfonylamino-, N-(C₁₋₃-Alkyl)-arylsulfonylamino-, oder N-(C₁₋₃-Alkyl)-aryl-C₁₋₃-alkyl-sulfonylaminogruppe,
eine 2-Oxo-imidazolidin-1-yl-, 2,4-Dioxo-imidazolidin-1-yl-, 2,5-Dioxoimidazolidin-1-yl- oder 2-Oxo-hexahydropyrimidin-1 -ylgruppe, in der das Stickstoffatom in 3-Stellung jeweils durch eine Methyl- oder Ethylgruppe substituiert sein kann,
eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-, Piperazin-1-yl-carbonyl- oder 4-(C₁₋₃-Alkyl)-piperazin-1 -yl-carbonylgruppe,
eine C₁₋₃-Alkyl-carbonyl- oder eine Arylcarbonylgruppe,
eine Carboxy-C₁₋₃-alkyl-, C₁₋₃-Alkyloxy-carbonyl-C₁₋₃-alkyl-, Cyan-C₁₋₃-alkyl-, Aminocarbonyl-C₁₋₃-alkyl-, C₁₋₃-Alkyl-aminocarbonyl-C₁₋₃-alkyl-, Di-(C₁₋₃-alkyl)-aminocarbonyl-C₁₋₃-alkyl-, Pyrrolidin-1-yl-carbonyl-C₁₋₃-alkyl-, Piperidin-1-yl-carbonyl-C₁₋₃-alkyl-, Morpholin-4-yl-carbonyl-C₁₋₃-alkyl-, Piperazin-1 -yl-carbonyl-C₁₋₃-alkyl- oder 4-(C₁₋₃-Alkylrpiperazin-1-yl-carbonyl-C₁₋₃-alkylgruppe,
eine Carboxy-C₁₋₃-alkyloxy-, C₁₋₃-Alkyloxy-carbonyl-C₁₋₃alkyloxy-, Cyan-C₁₋₃-alkyloxy-, Aminocarbonyl-C₁₋₃-alkyloxy-, C₁₋₃-Alkyl-aminocarbonyl-C₁₋₃-alkyloxy-, Di-( C₁₋₃-alkyl)-aminocarbonyl-C₁₋₃-alkyloxy-, Pyrrolidin-1-yl-carbonyl-C₁₋₃-alkyloxy-, Piperidin-1-yl-carbonyl-C₁₋₃-alkyloxy-, Morpholin-4-yl-carbonyl-C₁₋₃-alkyloxy-, Piperazin-1-yl-carbonyl-C₁₋₃-alkyloxy- oder 4-(C₁₋₃-Alkyl)-piperazin-1-yl-carbonyl-C₁₋₃-alkyloxygruppe,
eine Hydroxy-C₁₋₃-alkyl-, C₁₋₃-Alkyloxy-C₁₋₃-alkyl-, Amino-C₁₋₃-alkyl-, C₁₋₃-Alkylamino-C₁₋₃-alkyl-, Di-(C₁₋₃-alkyl)-amino-C₁₋₃-alkyl-, Pyrrolidin-1-yl-C₁₋₃-alkyl-, Piperidin-1-yl-C₁₋₃-alkyl-, Morpholin-4-yl-C₁₋₃-alkyl-, Piperazin-1-yl-C₁₋₃-alkyl- oder 4-(C₁₋₃-Alkyl)-piperazin-1-yl-C₁₋₃-alkyl-gruppe,
eine Hydroxy-C₁₋₃-alkyloxy-, C₁₋₃-Alkyloxy-C₁₋₃-alkyloxy-, C₁₋₃-Alkyl-sulfanyl-C₁₋₃-alkyloxy-, C₁₋₃-Alkylsulfinyl-C₁₋₃-alkyloxy-, C₁₋₃-Alkylsulfonyl-C₁₋₃-alkyloxy-, Amino-C₁₋₃-alkyloxy-, C₁₋₃-Alkylamino-C₁₋₃-alkyloxy-, Di-(C₁₋₃-alkyl)-amino-C₁₋₃-alkyloxy-, Pyrrolidin-1-yl-C₁₋₃-alkyloxy-, Piperidin-1-yl-C₁₋₃-alkyloxy-, Morpholin-4-yl-C₁₋₃-alkyloxy-, Piperazin-1-yl-C₁₋₃-alkyloxy- oder 4-(C₁₋₃-Alkyl)-piperazin-1-yl-C₁₋₃-alkyloxygruppe,
eine Mercapto-, C₁₋₃-Alkylsulfanyl-, C₁₋₃-Alkysulfinyl-, C₁₋₃-Alkylsulfonyl-, C₁₋₃-Alkylsulfonyloxy-, Arylsulfonyloxy-, Trifluormethylsulfanyl-, Trifluor= methylsulfinyl- oder Trifluormethylsulfonylgruppe,
eine Sulfo-, Aminosulfonyl-, C₁₋₃-Alkyl-aminosulfonyl-, Di-(C₁₋₃-alkyl)-aminosulfonyl-, Pyrrolidin-1-yl-sulfonyl-, Piperidin-1-yl-sulfonyl-, Morpholin-4-yl-sulfonyl-, Piperazin-1-yl-sulfonyl- oder 4-(C₁₋₃-Alkyl)-piperazin-1-yl-sulfonylgruppe,
eine durch 1 bis 3 Fluoratome substituierte Methyl- oder Methoxygruppe,
eine durch 1 bis 5 Fluoratome substituierte Ethyl- oder Ethoxygruppe,
eine C₂₋₄-Alkenyl- oder C₂₋₄-Alkinylgruppe,
eine C₃₋₄-Alkenyloxy- oder C₃₋₄-Alkinyloxygruppe,
eine C₃₋₆-Cycloalkyl- oder C₃₋₆-Cycloalkyloxygruppe,
eine C₃₋₆-Cycloalkyl-C₁₋₃-alkyl- oder C₃₋₆-Cycloalkyl-C₁₋₃-alkyloxygruppe oder
eine Aryl-, Aryloxy-, Aryl-C₁₋₃-alkyl- oder Aryl-C₁₋₃-alkyloxygruppe,
R¹¹ und R¹², die gleich oder verschieden sein können, jeweils ein Wasserstoffatom, ein Fluor-, Chlor-, Brom- oder Iodatom, eine C₁₋₃-Alkyl-, Trifluormethyl-, Hydroxy-, C₁₋₃-Alkyloxy- oder Cyangruppe, oder
R¹¹ zusammen mit R¹², sofern diese an benachbarte Kohlenstoffatome gebunden sind, auch eine Methylendioxy-, Difluormethylendioxy-, Ethylendioxy- oder eine geradkettige C₃₋₅-Alkylengruppe und
R¹³ ein Wasserstoffatom, ein Fluor-, Chlor- oder Bromatom, eine Trifluormethyl-, C₁₋₃-Alkyl- oder C₁₋₃-Alkyloxygruppe bedeuten,
R² ein Wasserstoff-, Fluor- oder Chloratom,
eine C₁₋₆-Alkylgruppe,
eine C₂₋₄-Alkenylgruppe,
eine C₃₋₄-Alkinylgruppe,
eine C₃₋₆-Cycloalkylgruppe,
eine C₃₋₆-Cycloalkyl-C₁₋₃-alkylgruppe,
eine Tetrahydrofuran-3-yl-, Tetrahydropyran-3-yl-, Tetrahydropyran-4-yl-, Tetrahydrofuranylmethyl- oder Tetrahydropyranylmethylgruppe,
eine Arylgruppe,
eine Aryl-C₁₋₄-alkylgruppe,
eine Aryl-C₂₋₃-alkenylgruppe,
eine Arylcarbonylgruppe,
eine Arylcarbonyl-C₁₋₂-alkylgruppe,
eine Heteroarylgruppe,
eine Heteroaryl-C₁₋₃-alkylgruppe,
eine Furanylcarbonyl-, Thienylcarbonyl-, Thiazolylcarbonyl- oder Pyridylcarbonylgruppe,
eine Furanylcarbonylmethyl-, Thienylcarbonylmethyl-, Thiazolylcarbonylmethyl- oder Pyridylcarbonylmethylgruppe,
eine C₁₋₄-Alkyl-carbonyl-Gruppe,
eine C₁₋₄-Alkyl-carbonyl-C₁₋₂-alkyl-Gruppe,
eine C₃₋₆-Cycloalkyl-carbonyl-Gruppe,
eine C₃₋₆-Cycloalkyl-carbonyl-C₁₋₂-alkyl-Gruppe,
eine Aryl-A- oder Aryl-A-C₁₋₃-alkyl-gruppe, wobei A ein Sauerstoff- oder Schwefelatom, eine Imino-, C₁₋₃-Alkylimino-, Sulfinyl- oder Sulfonylgruppe bedeutet,
eine Gruppe R_{b}, wobei
R_{b} eine Cyano-, Carboxy-, C₁₋₃-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl-, Morpholin-4-ylcarbonyl-, Piperazin-1-ylcarbonyl-, 4-Methylpiperazin-1-ylcarbonyl-, 4-Ethylpiperazin-1-ylcarbonyl-, Hydroxy-, Mercapto-, C₁₋₃-Alkyloxy-, C₁₋₃-Alkylsulfenyl-, C₁₋₃-Alkylsulfinyl-, C₁₋₃-Alkylsulfonyl-, Amino-, C₁₋₃-Alkylamino-, Di-(C₁₋₃-alkyl)-emino-, Pyrrolidin-1-yl-, Piperidin-1-yl-, Morpholin-4-yl-, Piperazin-1-yl-, 4-Methyl-piperazin-1-yl- oder 4-Ethyl-piperazin-1-yl-gruppe bedeutet,
oder eine durch eine Gruppe R_{b} substituierte C₁₋₄-Alkylgruppe, wobei R_{b} wie oben erwähnt definiert ist,
Y ein Stickstoffatom oder eine Gruppe der Formel C-R⁵,
wobei R⁵ wie R² definiert ist und jeweils einer der beiden Reste R² und R⁵ ein Wasserstoffatom oder ein C₁₋₃-Alkylgruppe sein muss,
R³ eine C₃₋₈-Alkylgruppe,
eine durch eine Gruppe R_{c} substituierte C₁₋₃-Alkylgruppe, wobei
R_{c} eine gegebenenfalls durch eine oder zwei C₁₋₃-Alkylgruppen substituierte C₃₋₇-Cycloalkylgruppe,
eine gegebenenfalls durch eine oder zwei C₁₋₃-Alkylgruppen substituierte C₅₋₇-Cycloalkenylgruppe,
eine Arylgruppe oder
eine Furanyl-, Thienyl-, Oxazolyl-, Isoxazolyl-, Thiazolyl-, Isothiazolyl-, Pyridyl-, Pyridazinyl-, Pyrimidyl- oder Pyrazinylgruppe bedeutet, wobei die vorstehend erwähnten heterocyclischen Reste jeweils durch eine oder zwei C₁₋₃-Alkylgruppen oder durch ein Fluor-, Chlor-, Brom- oder Iodatom oder durch eine Trifluormethyl-, Cyan- oder C₁₋₃-Alkyloxygruppe substituiert sein können,
eine C₃₋₈-Alkenylgruppe,
eine durch ein Fluor-, Chlor- oder Bromatom oder durch eine Trifluormethylgruppe substituierte C₃₋₆-Alkenylgruppe,
eine C₃₋₈-Alkinylgruppe,
eine Arylgruppe oder
eine Aryl-C₂₋₄-alkenylgruppe,
und
R⁴ eine Azetidin-1-yl- oder Pyrrolidin-1-ylgruppe, die in 3-Stellung durch eine Amino-, C₁₋₃-Alkylamino- oder eine Di-(C₁₋₃-alkyl)amino-Gruppe substituiert ist und zusätzlich durch eine oder zwei C₁₋₃-Alkylgruppen substituiert sein kann,
eine Piperidin-1-yl- oder Hexahydroazepin-1-ylgruppe, die in 3-Stellung oder in 4-Stellung durch eine Amino-, C₁₋₃-Alkylamino- oder eine Di-(C₁₋₃-alkyl)amino-Gruppe substituiert ist und zusätzlich durch eine oder zwei C₁₋₃-Alkylgruppen substituiert sein kann,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyanthiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-aminogruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1yl-, Piperidin-1-yl- oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine gegebenenfalls am Kohlenstoffgerüst durch eine oder zwei C₁₋₃-Alkylgruppen substituierte Piperazin-1-yl- oder [1,4]Diazepan-1-ylgruppe,
eine gegebenenfalls am Kohlenstoffgerüst durch eine oder zwei C₁₋₃-Alkylgruppen substituierte 3-Imino-piperazin-1-yl-, 3-Imino-[1,4]diazepan-1-yl- oder 5-Imino-[1,4]diazepan-1-ylgruppe,
eine gegebenenfalls durch eine oder zwei C₁₋₃-Alkylgruppen substituierte [1,4]Diazepan-1-ylgruppe, die in 6-Stellung durch eine Aminogruppe substituiert ist,
eine C₃₋₇-Cycloalkylgruppe, die durch eine Amino-, C₁₋₃-Alkylamino- oder Di-(C₁₋₃-alkyl)-aminogruppe substituiert ist,
eine C₃₋₇-Cycloalkylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkylgruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkylgruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkylaminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine N-(C₃₋₇-Cycloalkyl)-N-(C₁₋₃-alkyl)-aminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine C₃₋₇-Cycloalkylaminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl)-N-(C₁₋₃-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkyl-aminogruppe, in der der Cycloalkylteil durch eine Amino-, C₁₋₃-Alkylamino- oder Di-(C₁₋₃-alkyl)-aminogruppe substituiert ist,
eine N-(C₃₋₇-Cycloalkyl-C₁₋₂-alkyl)-N-(C₁₋₂-alkyl)-aminogruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkyl-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl-C₁₋₂-alkyl)-N-(C₁₋₂-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine R¹⁹-C₂₋₄-Alkylamino-Gruppe, in der R¹⁹ durch mindestens zwei Kohlenstoffatome vom Stickstoffatom des C₂₋₄-Alkylamino-Teils getrennt ist und
R¹⁹ eine Amino-, C₁₋₃-Alkylamino- oder Di-(C₁₋₃-alkyl)-aminogruppe darstellt,
eine R¹⁹-C₂₋₄-Alkylamino-Gruppe, in der das Stickstoffatom des C₂₋₄-Alkylamino-Teils durch eine C₁₋₃-Alkylgruppe substituiert ist und R¹⁹ durch mindestens zwei Kohlenstoffatome vom Stickstoffatom des C₂₋₄-Alkylamino-Teils getrennt ist, wobei R¹⁹ wie vorstehend erwähnt definiert ist,
eine durch den Rest R²⁰ substituierte Aminogruppe, in der
R²⁰ eine Azetidin-3-yl, Azetidin-2-ylmethyl-, Azetidin-3-ylmethyl-, Pyrrolidin-3-yl-, Pyrrolidin-2-ylmethyl-, Pyrrolidin-3-ylmethyl-, Piperidin-3-yl-, Piperidin-4-yl-, Piperidin-2-ylmethyl-, Piperidin-3-ylmethyl- oder Pipendin-4-ylmethylgruppe darstellt, wobei die für R²⁰ erwähnten Reste jeweils durch eine oder zwei C₁₋₃-Alkylgruppen substituiert sein können,
eine durch den Rest R²⁰ und eine C₁₋₃-Alkylgruppe substituierte Aminogruppe, in der R²⁰ wie vorstehend erwähnt definiert ist, wobei die für R²⁰ erwähnten Reste jeweils durch eine oder zwei C₁₋₃-Alkylgruppen substituiert sein können,
eine R¹⁹-C₃₋₄-alkyl-gruppe, in der der C₃₋₄-Alkylteil geradkettig ist und zusätzlich durch eine oder zwei C₁₋₃-Alkylgruppen substituiert sein kann, wobei R¹⁹ wie vorstehend erwähnt definiert ist,
eine 3-Amino-2-oxo-piperidin-5-yl- oder 3-Amino-2-oxo-1-methyl-piperidin-5-yl-Gruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino-oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂alkyl-, Piperidin-3-yl-, Piperidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können, bedeuten,
wobei unter den bei der Definition der vorstehend genannten Reste erwähnten Arylgruppen Phenyl- oder Naphthylgruppen zu verstehen sind, welche durch Rₕ mono-oder disubstituiert sein können, wobei die Substituenten gleich oder verschieden sein können und Rₕ ein Fluor-, Chlor-, Brom- oder Iodatom, eine Trifluormethyl-, Cyan-, Nitro-, Amino-, Aminocarbonyl-, Aminosulfonyl-, Methylsulfonyl, Acetylamino-, Methylsulfonylamino-, C₁₋₃-Alkyl-, Cyclopropyl-, Ethenyl-, -Ethinyl-, Hydroxy-, C₁₋₃-Alkyloxy-, Difluormethoxy- oder Trifluormethoxygruppe darstellt,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder lsochinolinylgruppe zu verstehen ist, in der eine bis drei Methingruppen durch Stickstoffatome ersetzt sind,
und die vorstehend erwähnten Heteroarylgruppen durch Rₕ mono- oder disubstituiert sein können, wobei die Substituenten gleich oder verschieden sein können und Rₕ wie vorstehend erwähnt definiert ist,
wobei, soweit nichts anderes erwähnt wurde, die vorstehend erwähnten Alkyl-, Alkenyl- und Alkinylgruppen geradkettig oder verzweigt sein können,
und wobei die Wasserstoffatome der in den Definitionen enthaltenen Methyl- oder Ethylgruppen ganz oder teilweise durch Fluoratome ersetzt sein können, deren Tautomere, Enantiomere, Diastereomere, deren Gemische, deren Salze, und deren Prodrugs worin die bei der Definition der vorstehend erwähnten Reste erwähnten Carboxygruppen eine in-vivo in eine Carboxygruppe-überführbare Gruppe oder durch eine unter physiologischen Bedingungen negativ geladene Gruppe ersetzt sind, oder die bei der Definition der vorstehend erwähnten Reste erwähnten Amino- und Iminogruppen durch einen in-vivo abspaltbaren Rest substituiert sind

2. Verbindungen der allgemeinen Formel I gemäß Anspruch 1, in der
R¹ eine durch eine Gruppe Rₐ substituierte Methylgruppe, wobei
Rₐ eine 3,4-Dihydro-chinolinylgruppe,
eine 3,4-Dihydro-isochinolinylgruppe,
eine 1,4-Dihydro-chinazolinyl- oder 4-Oxo-1,4-dihydro-chinazolinylgruppe,
eine 3,4-Dihydro-chinazolinyl- oder 4-Oxo-3,4-dihydro-chinazolinylgruppe,
eine 1*H*-Benzo[*d*][1,2]oxazinyl- oder 1-Oxo-1*H*-benzo[*d*][1,2]oxazinylgruppe,
eine 4*H*-Benzo[*e*][1,3]oxazinyl- oder 4-Oxo-4*H*-benzo[*e*][1,3]oxazinylgruppe,
eine 4*H*-Benzo[*d*][1,3]oxazinyl- oder 4-Oxo-4*H*-benzo[*d*][1,3]oxazinylgruppe,
eine 2*H*-Benzo[1,4]oxazinyl- oder 2-Oxo-2*H*-benzo[1,4]oxazinylgruppe,
eine 4*H*-Benzo[*e*][1,3]thiazinyl- oder 4-Oxo-4*H*-benzo[*e*][1,3]thiazinylgruppe,
eine 4*H*-Benzo[*d*][1,3]thiazinyl- oder 2*H*-Benzo [1,4]thiazinylgruppe,
eine 2-Oxo-2*H*-benzo[*e*][1,3]oxazinyl- oder 2,2-Dioxo-1*H*-benzo[*c*][1,2]thiazinylgruppe,
eine 2,3-Dihydro-1*H*-benzo[*e*][1,4]diazepinyl- oder 2-Oxo-2,3-dihydro-1*H-*benzo[*e*][1,4]diazepinylgruppe,
eine 4,5-Dihydro-3*H*-benzo[*b*][1,4]diazepinyl- oder 4-Oxo-4,5-dihydro-3*H-*benzo[*b*][1,4]diazepinylgruppe,
eine 5-Oxo-4,5-dihydro-3*H*-benzo[*e*][1,4]diazepinylgruppe,
eine 2,3-Dihydro-benzo[*f*][1,4]oxazepinyl- oder 2,3-Dihydro-benzo[*b*][1,4]-oxazepinylgruppe,
eine 2,3-Dihydro-benzo[*f*][1,4]thiazepinyl- oder 2,3-Dihydro-benzo[*b*][1,4]-thiazepinylgruppe,
eine 5-Oxo-4,5-dihydro-benzo[*f*][1,3,4]oxadiazepinylgruppe,
eine 11*H*-Dibenzo[*b*,*e*]azepinyl- oder 11-Oxo-11*H*-dibenzo[*b,e*]azepinylgruppe.
eine 11*H* Benzo[*e*]pyrido[3,2-*b*]azepinyl- oder eine 5*H*-1,9,10-Triaza-dibenzo-[a,d]cycloheptenylgruppe,
eine 5*H*-Dibenzo[*b,e*][1,4]diazepinyl- oder Dibenzo[*b*,*f*][1,4]oxazepinylgruppe,
eine Dibenzo[*b*,*f*][1,4]thiazepinyl-, 5-Oxo-dibenzo[b,*f*][1,4]thiazepinyl- oder 5,5-Dioxo-dibenzo[*b*,*f*][1,4]thiazepinylgruppe,
eine 5H-Dibenzo[*a,d*]cycloheptenyl- oder 5*H*-Dibenzo[*b*,*f*]azepinylgruppe,
eine Phenanthridinyl-, Benzo[*c*][1,5]naphthyridinyl-, Benzo[*h*][1,6]naphthyridinyl-, Benzo[*c*][1,8]naphthyridinyl-, Benzo[*f*][1,7]naphthyridinyl-oder 1,5,9-Triaza-phenanthrenylgruppe,
eine 1,2,3,4-Tetrahydro-phenanthridinyl-, 1,2,3,4,4a,10b-Hexahydro-phenanthridinyl-, 2,3-Dihydro-1*H*-4-aza-cyclopenta[a]naphthyl- oder 8,9,10,11-Tetrahydro-7H-6-aza-cyclohepta[*a*]naphthylgruppe,
eine 2,3-Dihydro-1*H*-4-oxa-10-aza-phenanthrenyl- oder 1-Oxo-2,3-dihydro-1*H-*4-oxa-10-aza-phenanthrenylgruppe,
eine Phenanthrenyl-, Benzo[*h*]chinolinyl-, Benzo[*f*]chinolinyl- oder Benzo[*f*]chinoxalinylgruppe,
eine 5*H*-Benzo[*e*]pyrrolo[1,2-*a*][1,4]diazepinyl-, Thieno[3,2-*b*][1,4]benzoxazepinyl-, 5*H*-Dibenzo[*d,f*][1,3]diazepinyl- oder 5-Oxa-7-aza-dibenzo[*a,c*]-cycloheptenylgruppe,
eine Naphtho[1,2-*d*]oxazolyl-, Naphtho[2,1-*d*]oxazolyl -, Naphtho[1,2-*d*]thiazolyl-, Naphtho[2,1-*d*]thiazolyl-, Naphtho[1,2-*d*]imidazolyl-, Naphtho[1,2-*b*]furanyl- oder,Naphtho[2,1-*b*]furanylgruppe,
oder eine Furo[3,2-*c*]isochinolinyl-, Pyrazolo[1,5-*c*]chinazolinyl- oder 1*H-*Perimidinylgruppe bedeutet,
wobei die Benzogruppen der vorstehend erwähnten Reste Rₐ durch die Gruppen R¹⁰ bis R¹³ substituiert sind und die Alkyleneinheiten der vorstehend erwähnten Reste Rₐ durch ein oder zwei Fluoratome oder ein oder zwei C₁₋₃-Alkyl- oder C₁₋₃-Alkyloxy-carbonylgruppen substituiert sein können und die Iminogruppen der vorstehend erwähnten Reste Rₐ durch eine C₁₋₃-Alkylgruppe substituiert sein können und
R¹⁰ ein Wasserstoffatom,
ein Fluor-, Chlor-, Brom- oder lodatom,
eine C₁₋₃-Alkyl- oder Cyclopropylgruppe,
eine Hydroxy-, C₁₋₃-Alkyloxy- oder Cyclopropyloxygruppe,
eine Nitro-, Amino-, C₁₋₃-Alkylamino- oder Di-(C₁₋₃-alkyl)aminogruppe,
eine C₁₋₃-Alkyl-carbonylamino- oder C₁₋₃-Alkyl-sulfonylaminogruppe,
eine Cyan-, Carboxy-, C₁₋₃-Alkyloxy-carbonyl-, Aminocarbonyl-, C₁₋₃-Alkyl-aminocarbonyl- oder Di-(C₁₋₃-alkyl)-aminocarbonylgruppe,
eine Mercapto-,C₁₋₃-Alkylsulfanyl-, C₁₋₃-Alkysulfinyl-, C₁₋₃-Alkylsulfonyl-oder Aminosulfonylgruppe oder
eine Difluormethyl-, Trifluormethyl-, Difluormethoxy- oder Trifluormethoxygruppe und
R¹¹, R¹² und R¹³, die gleich oder verschieden sein können, jeweils ein Wasserstoffatom, ein Fluor-, Chlor oder Bromatom, eine Methyl-, Trifluormethyl- oder Methoxygruppe bedeuten,
R² ein Wasserstoffatom oder
eine C₁₋₃-Alkyl-, Cyclopropyl-, Trifluormethyl-, Cyanomethyl- oder 2-Cyanoethylgruppe,
Y ein Stickstoffatom oder eine Gruppe der Formel C-R⁵,
wobei R⁵ ein Wasserstoffatom oder eine C₁₋₃-Alkylgruppe bedeutet,
R³ eine 2-Buten-1-yl- oder 3-Methyl-2-buten-1-ylgruppe,
eine 1-Buten-1-ylgruppe,
eine 2-Butin-1-ylgruppe oder
eine 1-Cyclopenten-1-ylmethylgruppe
und
R⁴ eine (3-Amino-piperidin-1-yl)gruppe bedeuten,
wobei, soweit nichts anderes erwähnt wurde, die vorstehend erwähnten Alkylgruppen geradkettig oder verzweigt sein könnnen,
deren Tautomere, Enantiomere, Diastereomere, deren Gemische und deren Salze..

3. Verbindungen der allgemeinen Formel I gemäß Anspruch 2, in der
R¹ eine durch eine Gruppe Rₐ substituierte Methylgruppe, wobei
Rₐ eine 3,4-Dihydro-chinolin-2-ylgruppe,
eine 3,4-Dihydro-isochinolin-1-ylgruppe,
eine 1,4-Dihydro-chinazolin-2-yl- oder 4-Oxo-1,4-dihydro-chinazolin-2-ylgruppe,
eine 3,4-Dihydro-chinazolin-2-yl- oder 4-Oxo-3,4-dihydro-chinazolin-2-ylgruppe,
eine 1*H*-Benzo[*d*][1,2]oxazin-4-yl- oder 1-Oxo-1*H*-benzo[*d*][1,2]oxazin-4-ylgruppe,
eine 4*H*-Benzo[*e*][1,3]oxazin-2-yl- oder 4-Oxo-4*H*-benzo[*e*][1,3]oxazin-2-ylgruppe,
eine 4*H*-Benzo[*d*][1,3]oxazin-2-yl- oder 4-Oxo-4*H*-benzo[*d*][1,3]oxazin-2-ylgruppe,
eine 2*H*-Benzo[1,4]oxazin-3-yl- oder 2-Oxo-2*H*-benzo[1,4]oxazin-3-ylgruppe,
eine 4*H*-Benzo[*e*][1,3]thiazin-2-yl- oder 4-Oxo-4*H*-benzo[*e*][1,3]thiazin-2-ylgruppe,
eine 4*H*-Benzo[*d*][1,3]thiazin-2-yl- oder 2*H*-Benzo[1,4]thiazin-3-ylgruppe,
eine 2-Oxo-2*H*-benzo[*e*][1,3]oxazin-4-yl- oder 2,2-Dioxo-1*H*-benzo[*c*][1 ,2]thiazin-4-ylgruppe,
eine 2,3-Dihydro-1*H*-benzo[*e*][1,4]diazepin-5-yl- oder 2-Oxo-2,3-dihydro-1*H-*benzo[*e*][1,4]diazepin-5-ylgruppe,
eine 4,5-Dihydro-3*H*-benzo[*b*][1,4]diazepin-2-yl- oder 4-Oxo-4,5-dihydro-3*H* benzo[*b*][1,4]diazepin-2-ylgruppe,
eine 5-Oxo-4,5-dihydro-3*H*-benzo[*e*][1,4]diazepin-2-ylgruppe,
eine 2,3-Dihydro-benzo[*f*][1,4]oxazepin-5-yl- oder 2,3-Dihydro-benzo[*b*][1,4]-oxazepin-4-ylgruppe,
eine 2,3-Dihydro-benzo[*f*][1,4]thiazepin-5-yl- oder 2,3-Dihydro-benzo[*b*][1,4]-thiazepin-4-ylgruppe,
eine 5-Oxo-4,5-dihydro-benzo[*f*][1,3,4]oxadiazepin-2-ylgruppe,
eine 11*H* Dibenzo[*b,e*]azepin-6-yl- oder 11-Oxo-11*H*-dibenzo[*b,e*]azepin-6-ylgruppe,
eine 11*H*-Benzo[*e*]pyrido[3,2-*b*]azepin-6-yl- oder eine 5*H*-1,9,10-Triazadibenzo[a,d]cyclohepten-11-ylgruppe,
eine 5*H*-Dibenzo[*b,e*][1,4]diazepin-11-yl- oder Dibenzo[*b,f*][1,4]oxazepin-11-ylgruppe,
eine Dibenzo[*b,f*][1,4]thiazepin-11-yl-, 5-Oxo-dibenzo[*b*,*f*][1,4]thiazepin-11-yl-oder 5,5-Dioxo-dibenzo[*b*,*f*][1,4]thiazepin-11-ylgruppe,
eine 5H-Dibenzo[*a*,*d*]cyclohepten-10-yl- oder 5*H*-Dibenzo[*b*,*f*]azepin-10-ylgruppe,
eine Phenanthridin-6-yl-, Benzo[*c*][1,5]naphthyridin-6-yl-, Benzo[*h*][1,6]naphthyridin-5-yl-, Benzo[*c*][1,8]naphthyridin-6-yl-, Benzo[*f*][1,7]naphthyridin-5-yl-oder 1,5,9-Triaza-phenanthren-10-ylgruppe,
eine 1,2,3,4-Tetrahydro-phenanthridin-6-yl, 1,2,3,4,4a,10b-Hexahydrophenanthridin-6-yl-, 2,3-Dihydro-1*H*-4-aza-cyclopenta[*a*]naphth-5-yl- oder 8,9,10,11 -Tetrahydro-7*H*-6-aza-cyclohepta[*a*]naphth-5-ylgruppe,
eine 2,3-Dihydro-1*H*-4-oxa-10-aza-phenanthren-9-yl- oder 1-Oxo-2,3-dihydro-1*H*-4-oxa-10-aza-phenanthren-9-ylgruppe,
eine Phenanthren-9-yl-, Benzo[*h*]chinolin-6-yl-, Benzo[*f*]chinolin-6-yl- oder Benzo[*f*]chinoxalin-6-ylgruppe,
eine 5*H*-Benzo[*e*]pyrrolo[1,2-*a*][1,4]diazepin-11-yl-, Thieno[3,2-*b*][1,4]benzoxazepin-9-yl-, 5*H*-Dibenzo[*d*,*f*][1,3]diazepin-6-yl- oder 5-Oxa-7-aza-dibenzo[*a*,*c*]cyclohepten-6-ylgruppe,
eine Naphtho[1,2-*d*]oxazol-2-yl-, Naphtho[2,1-*d*]oxazol-2-yl-, Naphtho[1,2-*d*]thiazol-2-yl-, Naphtho[2,1-*d*]thiazol-2-yl-, Naphtho[1,2-*d*]imidazol-2-yl-, Naphtho[1,2-*b*]furan-2-yl- oder Naphtho[2,1-*b*]furan-2-ylgruppe,
oder eine Furo[3,2-*c*]isochinolin-5-yl-, Pyrazolo[1,5-*c*]chinazolin-5-yl- oder 1*H-*Perimidin-2-ylgruppe bedeutet,
wobei die Benzogruppen der vorstehend erwähnten Reste Rₐ durch die Gruppen R¹⁰ bis R¹³ substituiert sind und die Alkyleneinheiten der vorstehend erwähnten Reste Rₐ durch ein oder zwei Fluoratome oder ein oder zwei
Methylgruppen substituiert sein können und die Iminogruppen der vorstehend erwähnten Reste Rₐ durch eine Methylgruppe substituiert sein können und
R¹⁰ ein Wasserstoffatom,
ein Fluor-, Chlor-, Brom- oder lodatom,
eine Methyl- oder Ethylgruppe,
eine Hydroxy-, Methoxy- oder Ethoxygruppe oder
eine Difluormethyl-, Trifluormethyl-, Difluormethoxy-, oder Trifluormethoxygruppe und
R¹¹ R¹² und R¹³, die gleich oder verschieden sein können, jeweils ein Wasserstoff-,Fluor-, Chlor- oder Bromatom oder eine Methyl-, Trifluormethyl- oder Methoxygruppe bedeuten,
R² ein Wasserstoffatom oder
eine Methyl-, Cyanomethyl-, Trifluormethyl-, Ethyl-, 2-Cyano-ethyl-, Propyl-, Cyclopropyl- oder Isopropylgruppe,
Y ein Stickstoffatom oder eine Gruppe der Formel C-R⁵,
wobei R⁵ ein Wasserstoffatom oder eine Methyl-, Ethyl-, Propyl- oder Isopropylgruppe bedeutet,
R³ eine 2-Buten-1-yl- oder 3-Methyl-2-buten-1-ylgruppe,
eine 1-Buten-1-ylgruppe,
eine 2-Butin-1-ytgruppe oder
eine 1-Cyclopenten-1-ylmethylgruppe
und
R⁴ eine (3-Amino-piperidin-1-yl)gruppe bedeuten,
deren Tautomere, Enantiomere, Diastereomere, deren Gemische und deren Salze.

4. Verbindungen der allgemeinen Formel 1 gemäß Anspruch 1, in der
R¹ eine 4-Oxo-3,4-dihydro-chinazolin-2-ylmethylgruppe,
eine Dibenzo[*b,f*][1,4]oxazepin-11-ylmethylgruppe,
eine Phenanthridin-6-ylmethylgruppe,
eine Phenanthren-9-ylmethylgruppe oder
eine Naphtho[1,2-*d*]oxazot-2-ylmethyl- oder Naphtho[2,1-*d*]oxazol-2-ylmethylgruppe,
R² ein Wasserstoffatom oder eine Methylgruppe,
Y ein Stickstoffatom,
R³ eine 2-Butin-1-ylgruppe
und
R⁴ eine (3-Amino-piperidin-1-yl)gruppe bedeuten,
deren Tautomere, Enantiomere, Diastereomere, deren Gemische und deren Salze.

5. Folgende Verbindungen der allgemeinen Formel 1 gemäß Anspruch 1:
(1) 2-(3-Amino-piperidin-1-yl)-3-(2-butin-1-yl)-5-[(dibenzo[*b,f*][1,4]oxazepin-11-yl)methyl]-3,5-dihydro-imidazo[4,5-*d*]pyridazin-4-on 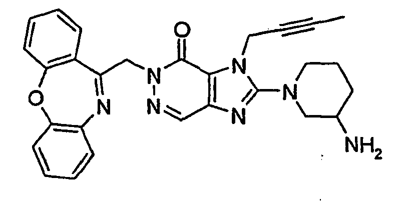
(2) 2-(3-Amino-piperidin-1-yl)-3-(2-butin-1-yl)-5-[(phenanthridin-6-yl)methyl]-3,5-dihydro-imidazo[4,5-*d*]pyridazin-4-on 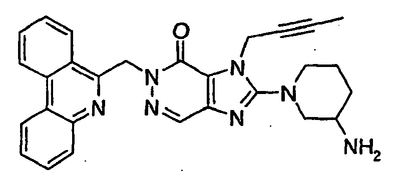
(3) 2-(3-Amino-piperidin-1 -yl)-3-(2-butin-1 -yl)-5-[(phenanthren-9-yl)methyl]-3,5-dihydro-imidazo[4,5-*d*]pyridazin-4-on 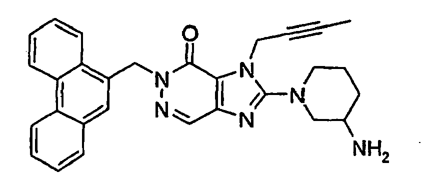
(4) 2-((*R*)-3-Amino-piperidin-1-yl)-3-(2-butin-1-yl)-5-[(phenanthridin-6-yl)methyl]-7-methyl-3,5-dihydro-imidazo[4,5-*d*]pyridazin-4-on 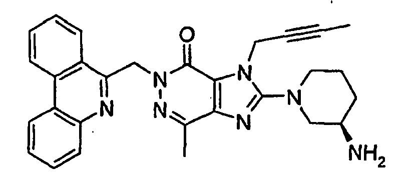
(5) 2-((*R*)-3-Amino-piperidin-1-yl)-3-(2-butin-1-yl)-5-[(dibenzo[*b,f*][1,4]oxazepin-11-yl)methyl]-7-methyl-3,5-dihydro-imidazo[4,5-*d*]pyridazin-4-on 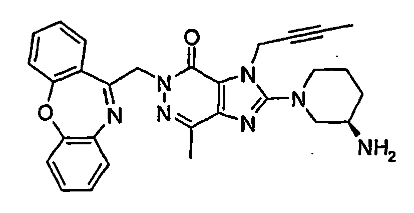
(6) 2-((*S*)-3-Amino-piperidin-1-yl)-3-(2-butin-1-yl)-5-[(dibenzo[b,*f*][1,4]oxazepin-11-yl)methyl]-3,5-dihydro-imidazo[4,5-*d*]pyridazin-4-on 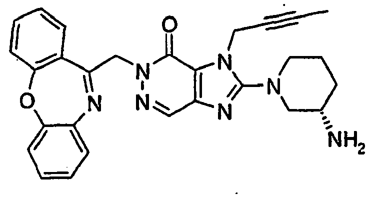
(7) 2-((*R*)-3-Amino-piperidin-1-yl)-3-(2-butin-1-yl)-5-[(dibenzo[*b*,*f*][1,4]oxazepin-11-yl)methyl]-3,5-dihydro-imidazo[4,5-*d*]pyridazin-4-on 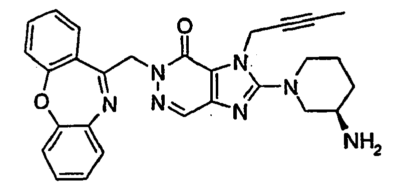
(8) 2-((*R*)-3-Amino-piperidin-1-yl)-3-(2-butin-1-yl)5-[(naphtho[2,1-*d*]oxazol-2-yl)methyl]-3,5-dihydro-imidazo[4,5-*d*]pyridazin-4-on 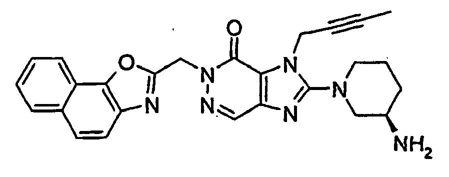
(9) 2-((*R*)-3-Amino-piperidin-1-yl)-3-(2-butin-1-yl)-5-[(naphtho[1,2-*d*]oxazol-2-yl)methyl]-3,5-dihydro-Imidazo[4,5-*d*]pyridazin-4-on 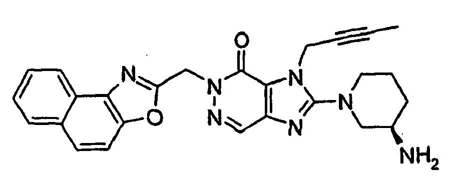
(10) 2-((*R*)-3-Amino-piperidin-1-yl)-3-(2-butin-1-yl)-5-[(4-oxo-3,4-dihydro-chinazolin-2-yl) methyl]-3, 5-dihydro-imidazo[4,5-*d*]pyridazin-4-on 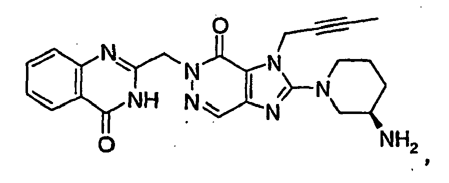
deren Enantiomere, deren Gemische und deren Salze.

6. Physiologisch verträgliche Salze der Verbindungen gemäß den Ansprüchen 1 bis 5 mit anorganischen oder organischen Säuren.

7. Arzneimittel, enthaltend eine Verbindung nach mindestens einem der Ansprüche 1 bis 5 oder ein Salz gemäß Anspruch 6 neben gegebenenfalls einem oder mehreren inerten Trägerstoffen und/oder Verdünnungsmiffeln.

8. Verwendung einer Verbindung nach mindestens einem der Ansprüche 1 bis 5 oder eines Salzes gemäß Anspruch 6 zur Herstellung eines Arzneimittels, das zur Behandlung von Diabetes mellitus Typ I und Typ II, Arthritis, Adipositas, Allograft Transplantation und durch Calcitonin verursachte Osteoporose geeignet ist.

9. Verfahren zur Herstellung eines Arzneimittels gemäß Anspruch 7, **dadurch gekennzeichnet, daß** auf nichtchemischem Wege eine Verbindung nach mindestens einem der Ansprüche 1 bis 5 oder ein Salz gemäß Anspruch 6 in einen oder mehrere inerte Trägerstoffe und/oder Verdünnungsmittel eingearbeitet wird.

10. Verfahren zur Herstellung der Verbindungen der Formel I gemäß den Ansprüchen 1 bis 6, **dadurch gekennzeichnet, daß**
a) eine Verbindung der allgemeinen Formel 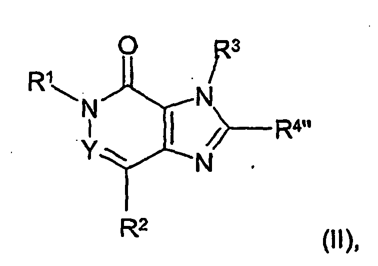 in der R¹, R², Y und R³ wie eingangs erwähnt definiert sind und
R⁴, eine der eingangs für R⁴ erwähnten Gruppen bedeutet, die eine Imino-, Amino-oder Alkylaminogruppe enthalten, wobei die Imino-, Amino- bzw. Alkylaminogruppe durch eine Schutzgruppe substituiert ist, entschützt wird und
gewünschtenfalls anschließend ein während den Umsetzungen zum Schutze von reaktiven Gruppen verwendeter Schutzrest abgespalten wird und/oder
eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure, übergeführt wird.

## Claims

1. Compounds of general formula 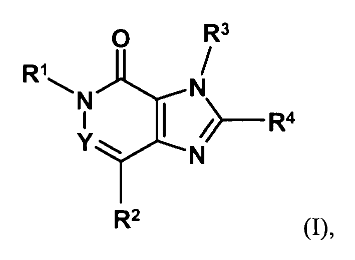 wherein
R¹ denotes a C₁₋₃-alkyl group substituted by a group Rₐ, where
Rₐ denotes a 3,4-dihydro-quinolinyl, 3,4-dihydro-isoquinolinyl, 1,4-dihydro-quinazolinyl, 3,4-dihydro-quinazolinyl, 1*H*-benzo[*d*][1,2]oxazinyl, 4*H-*benzo[*e*][1,3]oxazinyl, *4H* benzo[*d*][1,3]oxazinyl or *2H* benzo[1,4]oxazinyl group, wherein in each case
in the benzo moiety one to three methyne groups may each be replaced by a nitrogen atom and in the heterocyclyl moiety a methylene group may be replaced by a carbonyl group,
a 4*H*-benzo[*e*][1,3]thiazinyl, 4*H* benzo[*d*][1,3]thiazinyl or 2H-benzo [1,4]thiazinyl group wherein in each case
in the benzo moiety one to three methyne groups may each be replaced by a nitrogen atom and in the heterocyclyl moiety a methylene group may be replaced by a carbonyl group and the sulphur atom may be replaced by a sulphinyl or sulphonyl group,
a 2-oxo-2*H*-benzo[*e*][1,3]oxazinyl or 2,2-dioxo-1*H*-benzo[*c*][1,2]thiazinyl group wherein in each case in the benzo moiety
one to three methyne groups may each be replaced by a nitrogen atom,
a 2,3-dihydro-1*H*-benzo[*e*][1,4]diazepinyl, 4,5-dihydro-3*H*-benzo[*b*][1,4]diazepinyl or 5-oxo-4,5-dihydro-3*H*-benzo[*e*][1,4]diazepinyl group wherein in each case
in the benzo moiety one to three methyne groups may each be replaced by a nitrogen atom and in the heterocyclyl moiety a methylene group may be replaced by a carbonyl group,
a 2,3-dihydro-benzo[*f*][1,4]oxazepinyl or 2,3-dihydro-benzo[*b*][1,4]oxazepinyl group wherein in each case
in the benzo moiety one to three methyne groups may each be replaced by a nitrogen atom and in the heterocyclyl moiety a methylene group may be replaced by a carbonyl group,
a 2,3-dihydro-benzo[*b*][1,4]thiazepinyl or 2,3-dihydro-benzo[*f*][1,4]thiazepinyl group wherein in each case
in the benzo moiety one to three methyne groups may each be replaced by a nitrogen atom and in the heterocyclyl moiety a methylene group may be replaced by a carbonyl group and the sulphur atom may be replaced by a sulphinyl or sulphonyl group,
a 5-oxo-4,5-dihydro-benzo[*f*][1,3,4]oxadiazepinyl group wherein
in the benzo moiety one to three methyne groups may each be replaced by a nitrogen atom,
a 11*H*-dibenzo[*b*,*e*]azepinyl or 5*H*-dibenzo[*a*,*d*]cycloheptenyl group wherein in each case
in the benzo moiety one to three methyne groups may each be replaced by a nitrogen atom and the methylene group in the heterocyclyl moiety may be replaced by an oxygen or sulphur atom, a carbonyl, sulphinyl or sulphonyl group or by an imino group substituted by Rₓ, where
Rₓ denotes a hydrogen atom or a C₁₋₄-alkyl, C₂₋₄-alkenyl, C₂₋₄-alkynyl, C₃₋₆-cycloalkyl, C₃₋₆-cycloalkyl-C₁₋₃-alkyl, aryl, aryl-C₁₋₃-alkyl, hydroxy-C₂₋₄-alkyl, C₁₋₃-alkyloxy-C₂₋₄-alkyl, C₃₋₆-cycloalkyloxy-C₂-₄-alkyl, amino-C₂₋₄-alkyl, C₁₋₃-alkylamino-C₂₋₄-alkyl, di-(C₁₋₃-alkyl)-amino-C₂₋₄-alkyl, C₁₋₃-alkylcarbonyl, C₁₋₃-alkyloxy-carbonyl, C₁₋₃-alkyloxy-carbonyl-C₁₋₃-alkyl, aryl-carbonyl, C₁₋₃-alkyl-sulphonyl or aryl-sulphonyl group,
a phenanthridinyl group wherein
in the benzo moiety one to three methyne groups may each be replaced by a nitrogen atom, and
a 1,2,3,4-tetrahydro-phenanthridinyl, 1,2,3,4,4a,10b-hexahydro-phenanthridinyl, 2,3-dihydro-1*H*-4-aza-cyclopenta[*a*]naphthyl or a 8,9,10,11-tetrahydro-7*H*-6-aza-cyclohepta[*a*]naphthyl group wherein in each case
in the benzo moiety one to three methyne groups may each be replaced by a nitrogen atom and one or two methylene groups may each be replaced by an oxygen atom or a carbonyl group, while, if two methylene groups are each replaced by an oxygen atom, the oxygen atoms must be separated from one another by at least two methylene units,
a phenanthrenyl group wherein
in each case one to three of the methyne groups in position 1 to 4 and 5 to 8 may each be replaced by a nitrogen atom,
a 1,2,3,4-tetrahydro-phenanthrenyl or a 1,2,3,4,5,6,7,8-octahydrophenanthrenyl group wherein
in each case one or two of the methylene groups in position 1 to 4 and 5 to 8 may each be replaced by an oxygen atom or a carbonyl group, while, if two methylene groups are each replaced by an oxygen atom, the oxygen atoms must be separated from one another by at least two methylene units,
a 5*H*-benzo[*e*]pyrrolo[1,2-*a*][1,4]diazepinyl, thieno[3,2-*b*][1,4]benzoxazepinyl, 5*H*-dibenzo[*d*,*f*][1,3]diazepinyl or a 5-oxa-7-aza-dibenzo[*a*,*c*]cycloheptenyl group wherein in each case
in the benzo moiety one to three methyne groups may each be replaced by a nitrogen atom,
a naphtho[1,2-*d*]oxazolyl, naphtho[2,1-*d*]oxazolyl, naphtho[1,2-*d*]thiazolyl, naphtho[2,1-*d*]thiazolyl, naphtho[1,2-*d*]imidazolyl, naphtho[1,2-*b*]furanyl or naphtho[2,1-*b*]furanyl group wherein in each case
in the naphthyl moiety one to three methyne groups may each be replaced by a nitrogen atom,
or a furo[3,2-*c*]isoquinolinyl, pyrazolo[1,5-*c*]quinazolinyl or 1*H*-perimidinyl group,
while the methylene and methyne groups of the above mentioned radicals Rₐ may be substituted by the groups R¹⁰ to R¹³ and additionally by a C₁₋₃-alkyl group and the imino groups of the above mentioned radicals Rₐ may be substituted by the groups Rₓ as hereinbefore defined and
R¹⁰ denotes a hydrogen atom,
a fluorine, chlorine, bromine or iodine atom,
a C₁₋₄-alkyl, hydroxy, or C₁₋₄-alkyloxy group,
a nitro, amino, C₁₋₃-alkylamino, di-(C₁₋₃-alkyl)amino, cyano-C₁₋₃-alkylamino, N-(cyano-C₁₋₃-alkyl)-N-(C₁₋₃-alkyl)-amino, C₁₋₃-alkyloxycarbonyl-C₁₋₃-alkylamino, pyrrolidin-1-yl, piperidin-1-yl, morpholin-4-yl, piperazin-1-yl, or 4-(C₁₋₃-alkyl)-piperazin-1-yl group,
a C₁₋₃-alkyl-carbonylamino, arylcarbonylamino, aryl-C₁₋₃-alkylcarbonylamino, C₁₋₃-alkyloxy-carbonylamino, aminocarbonylamino, C₁₋₃-alkylaminocarbonylamino, di-(C₁₋₃-alkyl)aminocarbonylamino, pyrrolidin-1-yl-carbonylamino, piperidin-1-yl-carbonylamino, morpholin-4-yl-carbonylamino, piperazin-1-yl-carbonylamino or 4-(C₁₋₃-alkyl)-piperazin-1-yl-carbonylamino, C₁₋₃-alkyl-sulphonylamino, bis-(C₁₋₃-alkylsulphonyl)-amino, aminosulphonylamino, C₁₋₃-alkylamino-sulphonylamino, di-(C₁₋₃-alkyl)amino-sulphonylamino, pyrrolidin-1-yl-sulphonylamino, piperidin-1-yl-sulphonylamino, morpholin-4-yl-sulphonylamino, piperazin-1-yl-sulphonylamino or 4-(C₁₋₃-alkyl)-piperazin-1-yl-sulphonylamino, (C₁₋₃-alkylamino)thiocarbonylamino, (C₁₋₃-alkyloxy-carbonylamino)carbonylamino, arylsulphonylamino or aryl-C₁₋₃-alkylsulphonylamino group,
an N-(C₁₋₃-alkyl)-C₁₋₃-alkyl-carbonylamino, N -( C₁₋₃-alkyl)-arylcarbonylamino, N-(C₁₋₃-alkyl)-aryl-C₁₋₃-alkyl-carbonylamino, N-(C₁₋₃-alkyl)-C₁₋₃-alkyloxy-carbonylamino, N-( aminocarbonyl)-C₁₋₃-alkylamino, N-(C₁₋₃-alkyl-aminocarbonyl)-C₁₋₃-alkylamino , N-[di-(C₁₋₃-alkyl)aminocarbonyl]-C₁₋₃-alkylamino, N-(C₁₋₃-alkyl)-C₁₋₃-alkylsulphonylamino, N-(C₁₋₃-alkyl)-arylsulphonylamino, or N-(C₁₋₃-alkyl)-aryl-C₁₋₃-alkyl-sulphonylamino group,
a 2-oxo-imidazolidin-1-yl, 2,4-dioxo-imidazolidin-1-yl, 2,5-dioxoimidazolidin-1-yl or 2-oxo-hexahydropyrimidin-1-yl group wherein the nitrogen atom in the 3 position may be substituted in each case by a methyl or ethyl group,
a cyano, carboxy, C₁₋₃-alkyloxy-carbonyl, aminocarbonyl, C₁₋₃-alkylaminocarbonyl, di-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl or 4-(C₁₋₃-alkyl)-piperazin-1-yl-carbonyl group,
a C₁₋₃-alkyl-carbonyl or an arylcarbonyl group,
a carboxy-C₁₋₃-alkyl, C₁₋₃-alkyloxy-carbonyl-C₁₋₃-alkyl, cyano-C₁₋₃-alkyl, aminocarbonyl-C₁₋₃-alkyl, C₁₋₃ -alkyl-aminocarbonyl-C₁₋₃-alkyl, di-(C₁₋₃-alkyl)-aminocarbonyl-C₁₋₃-alkyl, pyrrolidin-1-yl-carbonyl-C₁₋₃-alkyl, piperidin-1-yl-carbonyl-C₁₋₃-alkyl, morpholin-4-yl-carbonyl-C₁₋₃-alkyl, piperazin-1-yl-carbonyl-C₁₋₃-alkyl or 4-(C₁₋₃-alkyl)-piperazin-1-yl-carbonyl-C₁₋₃-alkyl group,
a carboxy-C₁₋₃-alkyloxy, C₁₋₃-alkyloxy-carbonyl-C₁₋₃-alkyloxy, cyano-C₁₋₃-alkyloxy, aminocarbonyl-C₁₋₃-alkyloxy, C₁₋₃-alkyl-aminocarbonyl-C₁₋₃-alkyloxy, di-(C₁₋₃-alkyl)-aminocarbonyl-C₁₋₃-alkyloxy, pyrrolidin-1-yl-carbonyl-C₁₋₃-alkyloxy, piperidin-1-yl-carbonyl-C₁₋₃-alkyloxy, morpholin-4-yl-carbonyl-C₁₋₃-alkyloxy, piperazin-1-yl-carbonyl-C₁₋₃-alkyloxy or 4-(C₁₋₃-alkyl)-piperazin-1-yl-carbonyl-C₁₋₃-alkyloxy group,
a hydroxy-C₁₋₃-alkyl, C₁₋₃-alkyloxy-C₁₋₃-alkyl, amino-C₁₋₃-alkyl, C₁₋₃-alkylamino-C₁₋₃-alkyl, di-(C₁₋₃-alkyl)-amino-C₁₋₃-alkyl, pyrrolidin-1-yl-C₁₋₃-alkyl, piperidin-1-yl-C₁₋₃-alkyl, morpholin-4-yl-C₁₋₃-alkyl, piperazin-1-yl-C₁₋₃-alkyl or 4-(C₁₋₃-alkyl)-piperazin-1-yl-C₁₋₃-alkyl group,
a hydroxy-C₁₋₃-alkyloxy, C₁₋₃-alkyloxy-C₁₋₃-alkyloxy, C₁₋₃-alkyl-sulphanyl-C₁₋₃-alkyloxy, C₁₋₃-alkylsulphinyl-C₁₋₃-alkyloxy, C₁₋₃-alkylsulphonyl-C₁₋₃-alkyloxy, amino-C₁₋₃-alkyloxy, C₁₋₃-alkylamino-C₁₋₃-alkyloxy, di-(C₁₋₃-alkyl)-amino-C₁₋₃-alkyloxy, pyrrolidin-1-yl-C₁₋₃-alkyloxy, piperidin-1-yl-C₁₋₃-alkyloxy, morpholin-4-yl-C₁₋₃-alkyloxy, piperazin-1-yl-C₁₋₃-alkyloxy or 4-(C₁₋₃-alkyl)-piperazin-1-yl-C₁₋₃-alkyloxy group,
a mercapto, C₁₋₃-alkylsulphanyl, C₁₋₃-alkysulphinyl, C₁₋₃-alkylsulphonyl, C₁₋₃-alkylsulphonyloxy, arylsulphonyloxy, trifluoromethylsulphanyl, trifluoromethylsulphinyl or trifluoromethylsulphonyl group,
a sulpho, aminosulphonyl, C₁₋₃-alkyl-aminosulphonyl, di-(C₁₋₃-alkyl)-aminosulphonyl, pyrrolidin-1-yl-sulphonyl, piperidin-1-yl-sulphonyl, morpholin-4-yl-sulphonyl, piperazin-1-yl-sulphonyl or 4-(C₁₋₃-alkyl)-piperazin-1-yl-sulphonyl group,
a methyl or methoxy group substituted by 1 to 3 fluorine atoms,
an ethyl or ethoxy group substituted by 1 to 5 fluorine atoms,
a C₂₋₄-alkenyl or C₂₋₄-alkynyl group,
a C₃₋₄-alkenyloxy or C₃₋₄-alkynyloxy group,
a C₃₋₆-cycloalkyl or C₃₋₆-cycloalkyloxy group,
a C₃₋₆-cycloalkyl-C₁₋₃-alkyl or C₃₋₆-cycloalkyl-C₁₋₃-alkyloxy group or
an aryl, aryloxy, aryl-C₁₋₃-alkyl or aryl-C₁₋₃-alkyloxy group,
R¹¹ and R¹², which may be identical or different, in each case represent a hydrogen atom, a fluorine, chlorine, bromine or iodine atom, a C₁₋₃-alkyl, trifluoromethyl, hydroxy, C₁₋₃-alkyloxy or cyano group, or
R¹¹ together with R¹², if these are bound to adjacent carbon atoms, also denotes a methylenedioxy, difluoromethylenedioxy, ethylenedioxy or a straight-chain C₃₋₅-alkylene group and
R¹³ denotes a hydrogen atom, a fluorine, chlorine or bromine atom, a trifluoromethyl, C₁₋₃-alkyl or C₁₋₃-alkyloxy group,
R² denotes a hydrogen, fluorine or chlorine atom,
a C₁₋₆-alkyl group,
a C₂₋₄-alkenyl group,
a C₃₋₄-alkynyl group,
a C₃₋₆-cycloalkyl group,
a C₃₋₆-cycloalkyl-C₁₋₃-alkyl group,
a tetrahydrofuran-3-yl, tetrahydropyran-3-yl, tetrahydropyran-4-yl, tetrahydrofuranylmethyl or tetrahydropyranylmethyl group,
an aryl group,
an aryl-C₁₋₄-alkyl group,
an aryl-C₂₋₃-alkenyl group,
an arylcarbonyl group,
an arylcarbonyl-C₁₋₂-alkyl group,
a heteroaryl group,
a heteroaryl-C₁₋₃-alkyl group,
a furanylcarbonyl, thienylcarbonyl, thiazolylcarbonyl or pyridylcarbonyl group,
a furanylcarbonylmethyl, thienylcarbonylmethyl, thiazolylcarbonylmethyl or pyridylcarbonylmethyl group,
a C₁₋₄-alkyl-carbonyl group,
a C₁₋₄-alkyl-carbonyl-C₁₋₂-alkyl group,
a C₃₋₆-cycloalkyl-carbonyl group,
a C₃₋₆-cycloalkyl-carbonyl-C₁₋₂-alkyl group,
an aryl-A or aryl-A-C₁₋₃-alkyl group, where A denotes an oxygen or sulphur atom, an imino, C₁₋₃-alkylimino, sulphinyl or sulphonyl group,
a group R_{b}, where
R_{b} denotes a cyano, carboxy, C₁₋₃-alkyloxy-carbonyl, aminocarbonyl, C₁₋₃-alkylamino-carbonyl, di-(C₁₋₃-alkyl)-amino-carbonyl, pyrrolidin-1-ylcarbonyl, piperidin-1-ylcarbonyl, morpholin-4-ylcarbonyl, piperazin-1-ylcarbonyl, 4-methylpiperazin-1-ylcarbonyl, 4-ethylpiperazin-1-ylcarbonyl, hydroxy, mercapto, C₁₋₃-alkyloxy, C₁₋₃-alkylsulphenyl, C₁₋₃-alkylsulphinyl, C₁₋₃-alkylsulphonyl, amino, C₁₋₃-alkylamino, di-(C₁₋₃-alkyl)-amino, pyrrolidin-1-yl, piperidin-1-yl, morpholin-4-yl, piperazin-1-yl, 4-methyl-piperazin-1-yl or 4-ethyl-piperazin-1-yl group,
or a C₁₋₄-alkyl group substituted by a group R_{b}, where R_{b} is as hereinbefore defined,
Y denotes a nitrogen atom or a group of formula C-R⁵,
while R⁵ is defined like R² and in each case one of the two groups R² and R⁵ must be a hydrogen atom or a C₁₋₃-alkyl group,
R³ denotes a C₃₋₈-alkyl group,
a C₁₋₃-alkyl group substituted by a group R_{c}, where
R_{c} denotes a C₃₋₇-cycloalkyl group optionally substituted by one or two C₁₋₃-alkyl groups,
a C₅₋₇-cycloalkenyl group optionally substituted by one or two C₁₋₃-alkyl groups,
an aryl group or
a furanyl, thienyl, oxazolyl, isoxazolyl, thiazolyl, isothiazolyl, pyridyl, pyridazinyl, pyrimidyl or pyrazinyl group, while the above mentioned heterocyclic groups may each be substituted by one or two C₁₋₃-alkyl groups or by a fluorine, chlorine, bromine or iodine atom or by a trifluoromethyl, cyano or C₁₋₃-alkyloxy group,
a C₃₋₈-alkenyl group,
a C₃₋₆-alkenyl group substituted by a fluorine, chlorine or bromine atom or by a trifluoromethyl group,
a C₃₋₈-alkynyl group,
an aryl group or
an aryl-C₂₋₄-alkenyl group,
and
R⁴ denotes an azetidin-1-yl or pyrrolidin-1-yl group which is substituted in the 3 position by an amino, C₁₋₃-alkylamino or a di-(C₁₋₃-alkyl)amino group and may additionally be substituted by one or two C₁₋₃-alkyl groups,
a piperidin-1-yl or hexahydroazepin-1-yl group which is substituted in the 3 position or in the 4 position by an amino, C₁₋₃-alkylamino or a di-(C₁₋₃-alkyl)amino group and may additionally be substituted by one or two C₁₋₃-alkyl groups,
a 3-amino-piperidin-1-yl group wherein the piperidin-1-yl-moiety is additionally substituted by an aminocarbonyl, C₁₋₂-alkyl-aminocarbonyl, di-(C₁₋₂-alkyl)aminocarbonyl, pyrrolidin-1-yl-carbonyl, (2-cyano-pyrolidin-1-yl-)carbonyl, thiazolidin-3-yl-carbonyl, (4-cyano-thiazolidin-3-yl)carbonyl, piperidin-1-ylcarbonyl or morpholin-4-ylcarbonyl group,
a 3-amino-piperidin-1-yl group wherein the piperidin-1-yl moiety in the 4 position or in the 5 position is additionally substituted by a hydroxy or methoxy group,
a 3-amino-piperidin-1-yl group wherein the methylene group in the 2 position or in the 6 position is replaced by a carbonyl group,
a piperidin-1-yl or hexahydroazepin-1-yl group substituted in the 3 position by an amino, C₁₋₃-alkylamino or di-(C₁₋₃-alkyl)-amino- group, wherein in each case two hydrogen atoms on the carbon skeleton of the piperidin-1-yl or hexahydroazepin-1-yl- group are replaced by a straight-chain alkylene bridge, this bridge containing 2 to 5 carbon atoms if the two hydrogen atoms are located on the same carbon atom, or 1 to 4 carbon atoms, if the hydrogen atoms are located on adjacent carbon atoms, or 1 to 4 carbon atoms, if the hydrogen atoms are located on carbon atoms which are separated by one atom, or 1 to 3 carbon atoms if the two hydrogen atoms are located on carbon atoms separated by two atoms,
an azetidin-1-yl, pyrrolidin-1-yl, piperidin-1-yl or hexahydroazepin-1-yl group which is substituted by an amino-C₁₋₃-alkyl, C₁₋₃-alkylamino-C₁₋₃-alkyl or a di-(C₁₋₃-alkyl)-amino-C₁₋₃-alkyl group,
a piperazin-1-yl or [1,4]diazepan-1-yl group optionally substituted at the carbon skeleton by one or two C₁₋₃-alkyl groups,
a 3-imino-piperazin-1-yl, 3-imino-[1,4]diazepan-1-yl or 5-imino-[1,4]diazepan-1-yl group optionally substituted at the carbon skeleton by one or two C₁₋₃-alkyl groups,
a [1,4]diazepan-1-yl group optionally substituted by one or two C₁₋₃-alkyl groups, which is substituted in the 6 position by an amino group,
a C₃₋₇-cycloalkyl group which is substituted by an amino, C₁₋₃-alkylamino or di-(C₁₋₃-alkyl)-amino group,
a C₃₋₇-cycloalkyl group which is substituted by an amino-C₁₋₃-alkyl, C₁₋₃-alkylamino-C₁₋₃-alkyl or a di-(C₁₋₃-alkyl)amino-C₁₋₃-alkyl group,
a C₃₋₇-cycloalkyl-C₁₋₂-alkyl group wherein the cycloalkyl moiety is substituted by an amino, C₁₋₃-alkylamino or di-(C₁₋₃-alkyl)-amino group,
a C₃₋₇-cycloalkyl-C₁₋₂-alkyl group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃-alkyl group,
a C₃₋₇-cycloalkylamino group wherein the cycloalkyl moiety is substituted by an amino, C₁₋₃-alkylamino or di-(C₁₋₃-alkyl)-amino group, while the two nitrogen atoms at the cycloalkyl moiety are separated from one another by at least two carbon atoms,
an N-(C₃₋₇cycloalkyl)-N-(C₁₋₃-alkyl)-amino group wherein the cycloalkyl moiety is substituted by an amino, C₁₋₃-alkylamino or di-(C₁₋₃-alkyl)-amino group, while the two nitrogen atoms at the cycloalkyl moiety are separated from one another by at least two carbon atoms,
a C₃₋₇-cycloalkylamino group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃-alkyl group,
an N-(C₃₋₇-cycloalkyl)-N-(C₁₋₃-alkyl)-amino group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃-alkyl group,
a C₃₋₇-cycloalkyl-C₁₋₂-alkyl-amino group wherein the cycloalkyl moiety is substituted by an amino, C₁₋₃-alkylamino or di-(C₁₋₃-alkyl)-amino group,
an N-(C₃₋₇-cycloalkyl-C₁₋₂-alkyl)-N-(C₁₋₂-alkyl)-amino group wherein the cycloalkyl moiety is substituted by an amino, C₁₋₃-alkylamino or di-(C₁₋₃-alkyl)-amino group,
a C₃₋₇-cycloalkyl-C₁₋₂-alkyl-amino group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃-alkyl group,
an N-(C₃₋₇-cycloalkyl-C₁₋₂-alkyl)-N-(C₁₋₂-alkyl)-amino group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃-alkyl group,
an R¹⁹-C₂₋₄-alkylamino group wherein R¹⁹ is separated from the nitrogen atom of the C₂₋₄-alkylamino moiety by at least two carbon atoms and
R¹⁹ denotes an amino, C₁₋₃-alkylamino or di-(C₁₋₃-alkyl)-amino group,
an R¹⁹-C₂₋₄-alkylamino group wherein the nitrogen atom of the C₂₋₄-alkylamino moiety is substituted by a C₁₋₃-alkyl group and R¹⁹ is separated from the nitrogen atom of the C₂₋₄-alkylamino moiety by at least two carbon atoms, while R¹⁹ is as hereinbefore defined,
an amino group substituted by the group R²⁰ wherein
R²⁰ denotes an azetidin-3-yl, azetidin-2-ylmethyl, azetidin-3-ylmethyl, pyrrolidin-3-yl, pyrrolidin-2-ylmethyl, pyrrolidin-3-ylmethyl, piperidin-3-yl, piperidin-4-yl, piperidin-2-ylmethyl, piperidin-3-ylmethyl or piperidin-4-ylmethyl group, while the groups mentioned for R²⁰ may each be substituted by one or two C₁₋₃-alkyl groups,
an amino group substituted by the group R²⁰ and a C₁₋₃-alkyl group wherein R²⁰ is as hereinbefore defined, while the groups mentioned for R²⁰ may each be substituted by one or two C₁₋₃-alkyl groups,
an R¹⁹-C₃₋₄-alkyl group wherein the C₃₋₄-alkyl moiety is straight-chained and may additionally be substituted by one or two C₁₋₃-alkyl groups, while R¹⁹ is as hereinbefore defined,
a 3-amino-2-oxo-piperidin-5-yl or 3-amino-2-oxo-1-methyl-piperidin-5-yl group,
a pyrrolidin-3-yl, piperidin-3-yl, piperidin-4-yl, hexahydroazepin-3-yl or hexahydroazepin-4-yl group which is substituted in the 1 position by an amino, C₁₋₃-alkylamino or di-(C₁₋₃-alkyl)amino group,
or an azetidin-2-yl-C₁₋₂-alkyl, azetidin-3-yl-C₁₋₂-alkyl, pyrrolidin-2-yl-C₁₋₂-alkyl, pyrrolidin-3-yl, pyrrolidin-3-yl-C₁₋₂-alkyl, piperidin-2-yl-C₁₋₂-alkyl, piperidin-3-yl, piperidin-3-yl-C₁₋₂-alkyl, piperidin-4-yl or piperidin-4-yl-C₁₋₂-alkyl group, while the above mentioned groups may each be substituted by one or two C₁₋₃-alkyl groups,
while by the aryl groups mentioned in the definition of the above groups are meant phenyl or naphthyl groups which may be mono- or disubstituted by Rₕ, while the substituents may be identical or different and Rₕ denotes a fluorine, chlorine, bromine or iodine atom, a trifluoromethyl, cyano, nitro, amino, aminocarbonyl, aminosulphonyl, methylsulphonyl, acetylamino, methylsulphonylamino, C₁₋₃-alkyl, cyclopropyl, ethenyl, ethynyl, hydroxy, C₁₋₃-alkyloxy, difluoromethoxy or trifluoromethoxy group,
by the heteroaryl groups mentioned in the definition of the above groups are meant a pyrrolyl, furanyl, thienyl, pyridyl, indolyl, benzofuranyl, benzothiophenyl, quinolinyl or isoquinolinyl group,
or a pyrrolyl, furanyl, thienyl or pyridyl group, wherein one or two methyne groups are replaced by nitrogen atoms,
or an indolyl, benzofuranyl, benzothiophenyl, quinolinyl or isoquinolinyl group,
wherein one to three methyne groups are replaced by nitrogen atoms,
and the above mentioned heteroaryl groups may be mono- or disubstituted by Rₕ, while the substituents may be identical or different and Rₕ is as hereinbefore defined,
while, unless otherwise stated, the above mentioned alkyl, alkenyl and alkynyl groups may be straight-chain or branched,
and the hydrogen atoms of the methyl or ethyl groups contained in the definitions may be wholly or partly replaced by fluorine atoms,
the tautomers, enantiomers, diastereomers, the mixtures thereof, the salts thereof, and the prodrugs thereof wherein the carboxy groups mentioned in the definition of the above mentioned groups are replaced by a group that may be converted *in vivo* into a carboxy group or by a group that is negatively charged under physiological conditions, or
the amino and imino groups mentioned in the definition of the above mentioned groups are substituted by a group that can be cleaved *in vivo.*

2. Compounds of general formula I according to claim 1, wherein
R¹ denotes a methyl group substituted by a group Rₐ, where
Rₐ denotes a 3,4-dihydro-quinolinyl group,
a 3,4-dihydro-isoquinolinyl group,
a 1,4-dihydro-quinazolinyl or 4-oxo-1,4-dihydro-quinazolinyl group,
a 3,4-dihydro-quinazolinyl or 4-oxo-3,4-dihydro-quinazolinyl group,
a 1*H*-benzo[*d*][1,2]oxazinyl or 1-oxo-1*H*benzo[*d*][1,2]oxazinyl group,
a 4*H*-benzo[*e*][1,3]oxazinyl or 4-oxo-4*H*-benzo[*e*][1,3]oxazinyl group,
a 4*H*-benzo[*d*][1,3]oxazinyl or 4-oxo-4*H*-benzo[*d*][1,3]oxazinyl group,
a 2*H*-benzo[1,4]oxazinyl or 2-oxo-2*H*-benzo[1,4]oxazinyl group,
a 4*H*-benzo[*e*][1,3]thiazinyl or 4-oxo-4*H*-benzo[*e*][1,3]thiazinyl group,
a 4*H*-benzo[*d*][1,3]thiazinyl or 2*H*-benzo [1,4]thiazinyl group,
a 2-oxo-2*H*-benzo[*e*][1,3]oxazinyl or 2,2-dioxo-1*H*-benzo[*c*][1,2]thiazinyl group,
a 2,3-dihydro-1*H*-benzo[*e*][1,4]diazepinyl or 2-oxo-2,3-dihydro-1*H-*benzo[*e*][1,4]diazepinyl group,
a 4,5-dihydro-3*H*-benzo[*b*][1,4]diazepinyl or 4-oxo-4,5-dihydro-3*H-*benzo[*b*][1,4]diazepinyl group,
a 5-oxo-4,5-dihydro-3*H*-benzo[*e*][1,4]diazepinyl group,
a 2,3-dihydro-benzo[*f*][1,4]oxazepinyl or 2,3-dihydro-benzo[*b*][1,4]oxazepinyl group,
a 2,3-dihydro-benzo[*f*][1,4]thiazepinyl or 2,3-dihydro-benzo[*b*][1,4]thiazepinyl group,
a 5-oxo-4,5-dihydro-benzo[*f*][1,3,4]oxadiazepinyl group,
an 11*H*-dibenzo[*b*,*e*]azepinyl or 11-oxo-11*H*-dibenzo[*b*,*e*]azepinyl group,
an 11*H*-benzo[*e*]pyrido[3,2-*b*]azepinyl or a 5*H*-1,9,10-triaza-dibenzo[a,d]-cycloheptenyl group,
a 5*H*-dibenzo[*b*,*e*][1,4]diazepinyl or dibenzo[*b*,*f*][1,4]oxazepinyl group,
a dibenzo[*b*,*f*][1,4]thiazepinyl, 5-oxo-dibenzo[*b*,*f*][1,4]thiazepinyl or 5,5-dioxo-dibenzo[*b*,*f*][1,4]thiazepinyl group,
a 5*H*-dibenzo[*a*,*d*]cycloheptenyl or 5*H*-dibenzo[*b*,*f*]azepinyl group,
a phenanthridinyl, benzo[*c*][1,5]naphthyridinyl, benzo[*h*][1,6]naphthyridinyl, benzo[*c*][1,8]naphthyridinyl, benzo[*f*][1,7]naphthyridinyl or 1,5,9-triazaphenanthrenyl group,
a 1,2,3,4-tetrahydro-phenanthridinyl, 1,2,3,4,4a,10b-hexahydro-phenanthridinyl, 2,3-dihydro-1*H*-4-aza-cyclopenta[a]naphthyl or 8,9,10,11-tetrahydro-7*H*-6-aza-cyclohepta[α]naphthyl group,
a 2,3-dihydro-1*H*-4-oxa-10-aza-phenanthrenyl or 1-oxo-2,3-dihydro-1*H*-4-oxa-10-aza-phenanthrenyl group,
a phenanthrenyl, benzo[*h*]quinolinyl, benzo[*f*]quinolinyl or benzo[*f*]quinoxalinyl group,
a 5*H*-benzo[*e*]pyrrolo[1,2-*a*][1,4]diazepinyl, thieno[3,2-*b*][1,4]benzoxazepinyl, 5*H*-dibenzo[*d*,*f*]1,3]diazepinyl or 5-oxa-7-aza-dibenzo[*a*,*c*]cycloheptenyl group,
a naphtho[1,2-*d*]oxazolyl, naphtho[2,1-*d*]oxazolyl , naphtho[1,2-*d*]thiazolyl, naphtho[2,1-*d*]thiazolyl, naphtho[1,2-*d*]imidazolyl, naphtho[1,2-*b*]furanyl or naphtho[2,1-*b*]furanyl group,
or a furo[3,2-*c*]isoquinolinyl, pyrazolo[1,5-*c*]quinazolinyl or 1*H*-perimidinyl group,
while the benzo groups of the above mentioned radicals Rₐ are substituted by the groups R¹⁰ to R¹³ and the alkylene units of the above mentioned groups Rₐ may be substituted by one or two fluorine atoms or one or two C₁₋₃-alkyl or C₁₋₃-alkyloxy-carbonyl groups and the imino groups of the above mentioned radicals Rₐ may be substituted by a C₁₋₃-alkyl group and
R¹⁰ denotes a hydrogen atom,
a fluorine, chlorine, bromine or iodine atom,
a C₁₋₃-alkyl or cyclopropyl group,
a hydroxy, C₁₋₃-alkyloxy or cyclopropyloxy group,
a nitro, amino, C₁₋₃-alkylamino or di-(C₁₋₃-alkyl)amino group,
a C₁₋₃-alkyl-carbonylamino or C₁₋₃-alkyl-sulphonylamino group,
a cyano, carboxy, C₁₋₃-alkyloxy-carbonyl, aminocarbonyl, C₁₋₃-alkylaminocarbonyl or di-(C₁₋₃-alkyl)-aminocarbonyl group,
a mercapto, C₁₋₃-alkylsulphanyl, C₁₋₃-alkysulphinyl, C₁₋₃-alkylsulphonyl or aminosulphonyl group or
a difluoromethyl, trifluoromethyl, difluoromethoxy or trifluoromethoxy group and
R¹¹, R¹² and R¹³, which may be identical or different, in each case represent a hydrogen atom, a fluorine, chlorine or bromine atom, a methyl, trifluoromethyl or methoxy group,
R² denotes a hydrogen atom or
a C₁₋₃-alkyl, cyclopropyl, trifluoromethyl, cyanomethyl or 2-cyano-ethyl group,
Y denotes a nitrogen atom or a group of formula C-R⁵,
while R⁵ denotes a hydrogen atom or a C₁₋₃-alkyl group,
R³ denotes a 2-buten-1-yl or 3-methyl-2-buten-1-yl group,
a 1-buten-1-yl group,
a 2-butyn-1-yl group or
a 1-cyclopenten-1-ylmethyl group
and
R⁴ denotes a (3-amino-piperidin-1-yl) group,
while, unless otherwise stated, the above mentioned alkyl groups may be straight-chain or branched,
the tautomers, enantiomers, diastereomers, the mixtures thereof and the salts thereof.

3. Compounds of general formula I according to claim 2, wherein
R¹ denotes a methyl group substituted by a group Rₐ, where
Rₐ denotes a 3,4-dihydro-quinolin-2-yl group,
a 3,4-dihydro-isoquinolin-1-yl group,
a 1,4-dihydro-quinazolin-2-yl or 4-oxo-1,4-dihydro-quinazolin-2-yl group,
a 3,4-dihydro-quinazolin-2-yl or 4-oxo-3,4-dihydro-quinazolin-2-yl group,
a 1*H*-benzo[*d*][1,2]oxazin-4-yl or 1-oxo-1*H*-benzo[*d*][1,2]oxazin-4-yl group,
a 4*H*-benzo[*e*][1,3]oxazin-2-yl or 4-oxo-4*H*-benzo[*e*][1,3]oxazin-2-yl group,
a 4*H*-benzo[*d*][1,3]oxazin-2-yl or 4-oxo-4*H*-benzo[*d*][1,3]oxazin-2-yl group,
a 2*H*-benzo[1,4]oxazin-3-yl or 2-oxo-2*H*-benzo[1,4]oxazin-3-yl group,
a 4*H*-benzo[*e*][1,3]thiazin-2-yl or 4-oxo-4*H*-benzo[*e*][1,3]thiazin-2-yl group,
a 4*H*-benzo[*d*][1,3]thiazin-2-yl or 2*H*-benzo[1,4]thiazin-3-yl group,
a 2-oxo-2*H*-benzo[*e*][1,3]oxazin-4-yl or 2,2-dioxo-1*H*-benzo[*c*][1,2]thiazin-4-yl group,
a 2,3-dihydro-1*H*-benzo[*e*][1,4]diazepin-5-yl or 2-oxo-2,3-dihydro-1*H-*benzo[*e*][1,4]diazepin-5-yl group,
a 4,5-dihydro-3*H*-benzo[*b*][1,4]diazepin-2-yl or 4-oxo-4,5-dihydro-3*H-*benzo[*b*][1,4]diazepin-2-yl group,
a 5-oxo-4,5-dihydro-3*H*-benzo[*e*][1,4]diazepin-2-yl group,
a 2,3-dihydro-benzo[*f*][1,4]oxazepin-5-yl or 2,3-dihydro-benzo[*b*][1,4]-oxazepin-4-yl group,
a 2,3-dihydro-benzo[*f*][1,4]thiazepin-5-yl or 2,3-dihydro-benzo[*b*][1,4]-thiazepin-4-yl group,
a 5-oxo-4,5-dihydro-benzo[*f*][1,3,4]oxadiazepin-2-yl group,
an 11*H*-dibenzo[*b,e*]azepin-6-yl or 11-oxo-11*H*-dibenzo[*b,e*]azepin-6-yl group,
an 11*H* benzo[*e*]pyrido[3,2-*b*]azepin-6-yl or a *5H-*1,9,10-triaza-dibenzo[a,d]-cyclohepten-11-yl group,
a 5*H*-dibenzo[*b,e*][1,4]diazepin-11-yl or dibenzo[*b,f*][1,4]oxazepin-11-yl group,
a dibenzo[*b,f*][1,4]thiazepin-11-yl, 5-oxo-dibenzo[*b,f*][1,4]thiazepin-11-yl or 5,5-dioxo-dibenzo[*b,f*][1,4]thiazepin-11-yl group,
a 5*H*-dibenzo[*a,d*]cyclohepten-10-yl or 5*H*-dibenzo[*b,f*]azepin-10-yl group,
a phenanthridin-6-yl, benzo[*c*][1,5]naphthyridin-6-yl, benzo[*h*][1,6]naphthyridin-5-yl, benzo[*c*][1,8]naphthyridin-6-yl, benzo[*f*][1,7]naphthyridin-5-yl or 1,5,9-triaza-phenanthren-10-yl group,
a 1,2,3,4-tetrahydro-phenanthridin-6-yl, 1,2,3,4,4a,10b-hexahydrophenanthridin-6-yl, 2,3-dihydro-1*H*-4-aza-cyclopenta[*a*]naphth-5-yl or 8,9,10,11-tetrahydro-7*H*-6-aza-cyclohepta[*a*]naphth-5-yl group,
a 2,3-dihydro-1*H*-4-oxa-10-aza-phenanthren-9-yl or 1-oxo-2,3-dihydro-1*H* 4-oxa-10-aza-phenanthren-9-yl group,
a phenanthren-9-yl, benzo[*h*]quinolin-6-yl, benzo[*f*]quinolin-6-yl or benzo[*f*]quinoxalin-6-yl group,
a 5*H*-benzo[*e*]pyrrolo[1,2-*a*][1,4]diazepin-11-yl, thieno[3,2-*b*][1,4]benzoxazepin-9-yl, 5*H*-dibenzo[*d,f*][1,3]diazepin-6-yl or 5-oxa-7-aza-dibenzo[*a,c*]cyclohepten-6-yl group,
a naphtho[1,2-*d*]oxazol-2-yl, naphtho[2,1-*d*]oxazol-2-yl, naphtho[1,2-d]thiazol-2-yl, naphtho[2,1-*d*]thiazol-2-yl, naphtho[1,2-*d*]imidazol-2-yl, naphtho[1,2-*b*]furan-2-yl or naphtho[2,1-*b*]furan-2-yl group,
or a furo[3,2-*c*]isoquinolin-5-yl, pyrazolo[1,5-*c*]quinazolin-5-yl or 1*H-*perimidin-2-yl group,
while the benzo groups of the above mentioned radicals Rₐ are substituted by the groups R¹⁰ to R¹³ and the alkylene units of the above mentioned groups Rₐ may be substituted by one or two fluorine atoms or one or two methyl groups and the imino groups of the above mentioned radicals Rₐ may be substituted by a methyl group and
R¹⁰ denotes a hydrogen atom,
a fluorine, chlorine, bromine or iodine atom,
a methyl or ethyl group,
a hydroxy, methoxy or ethoxy group or
a difluoromethyl, trifluoromethyl, difluoromethoxy, or trifluoromethoxy group and
R¹¹, R¹² and R¹³, which may be identical or different, each denote a hydrogen, fluorine, chlorine or bromine atom or a methyl, trifluoromethyl or methoxy group,
R² denotes a hydrogen atom or
a methyl, cyanomethyl, trifluoromethyl, ethyl, 2-cyano-ethyl, propyl, cyclopropyl or isopropyl group,
Y denotes a nitrogen atom or a group of formula C-R⁵,
wherein R⁵ denotes a hydrogen atom or a methyl, ethyl, propyl or isopropyl group,
R³ denotes a 2-buten-1-yl or 3-methyl-2-buten-1-yl group,
a 1-buten-1-yl group,
a 2-butyn-1-yl group or
a 1-cyclopenten-1-ylmethyl group
and
R⁴ denotes a (3-amino-piperidin-1-yl) group,
the tautomers, enantiomers, diastereomers, the mixtures thereof and the salts thereof.

4. Compounds of general formula I according to claim 1, wherein
R¹ denotes a 4-oxo-3,4-dihydro-quinazolin-2-ylmethyl group,
a dibenzo[*b*,*f*][1,4]oxazepin-11-ylmethyl group,
a phenanthridin-6-ylmethyl group,
a phenanthren-9-ylmethyl group or
a naphtho[1,2-*d*]oxazol-2-ylmethyl or naphtho[2,1-*d*]oxazol-2-ylmethyl group,
R² denotes a hydrogen atom or a methyl group,
Y denotes a nitrogen atom,
R³ denotes a 2-butyn-1-yl group
and
R⁴ denotes a (3-amino-piperidin-1-yl) group,
the tautomers, enantiomers, diastereomers, the mixtures thereof and the salts thereof.

5. The following compounds of general formula I according to claim 1:
(1) 2-(3-amino-piperidin-1-yl)-3-(2-butyn-1-yl)-5-[(dibenzo[*b*,*f*][1,4]oxazepin-11-yl)methyl]-3,5-dihydro-imidazo[4,5-*d*]pyridazin-4-one 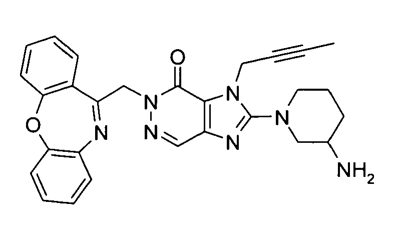
(2) 2-(3-amino-piperidin-1-yl)-3-(2-butyn-1-yl)-5-[(phenanthridin-6-yl)methyl]-3,5-dihydro-imidazo[4,5-*d*]pyridazin-4-one 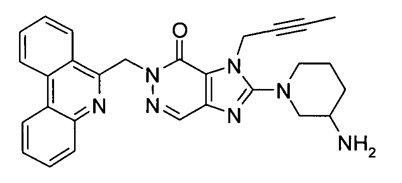
(3) 2-(3-amino-piperidin-1-yl)-3-(2-butyn-1-yl)-5-[(phenanthren-9-yl)methyl]-3,5-dihydro-imidazo[4,5-*d*]pyridazin-4-one 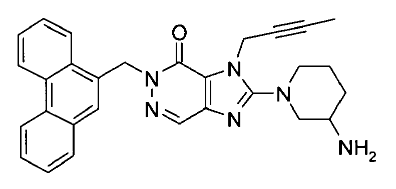
(4) 2-((*R*)-3-amino-piperidin-1-yl)-3-(2-butyn-1-yl)-5-[(phenanthridin-6-yl)methyl]-7-methyl-3,5-dihydro-imidazo[4,5-*d*]pyridazin-4-one 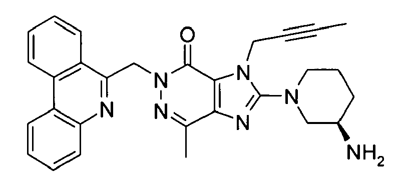
(5) 2-((*R*)-3-amino-piperidin-1-yl)-3-(2-butyn-1-yl)-5-[(dibenzo[*b*,*f*][1,4]oxazepin-11-yl)methyl]-7-methyl-3,5-dihydro-imidazo[4,5-*d*]pyridazin-4-one 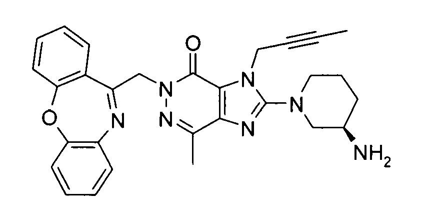
(6) 2-((*S*)-3-amino-piperidin-1-yl)-3-(2-butyn-1-yl)-5-[(dibenzo[*b,f*][1,4]oxazepin-11-yl)methyl]-3,5-dihydro-imidazo[4,5-*d*]pyridazin-4-one 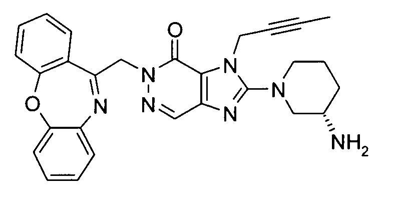
(7) 2-((*R*)-3-amino-piperidin-1-yl)-3-(2-butyn-1-yl)-5-[(dibenzo[*b,f*][1,4]oxazepin-11-yl)methyl]-3,5-dihydro-imidazo[4,5-*d*]pyridazin-4-one 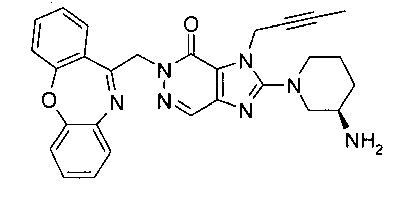
(8) 2-((*R*)-3-amino-piperidin-1-yl)-3-(2-butyn-1-yl)-5-[(naphtho[2,1-*d*]oxazol-2-yl)methyl]-3,5-dihydro-imidazo[4,5-*d*]pyridazin-4-one 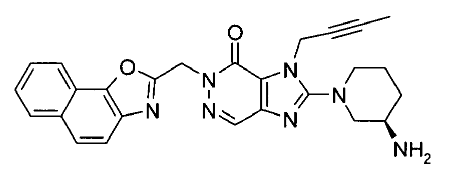
(9) 2-((*R*)-3-amino-piperidin-1-yl)-3-(2-butyn-1-yl)-5-[(naphtho[1,2-*d*]oxazol-2-yl)methyl]-3,5-dihydro-imidazo[4,5-*d*]pyridazin-4-one 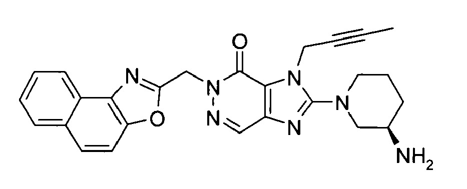
(10) 2-((*R*)-3-amino-piperidin-1-yl)-3-(2-butyn-1-yl)-5-[(4-oxo-3,4-dihydroquinazolin-2-yl)methyl]-3,5-dihydro-imidazo[4,5-*d*]pyridazin-4-one 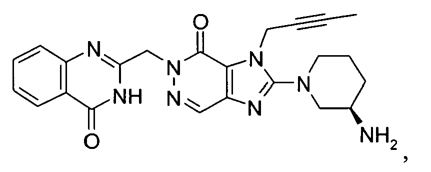
the enantiomers, the mixtures thereof and the salts thereof.

6. Physiologically acceptable salts of the compounds according to claims 1 to 5 with inorganic or organic acids.

7. Pharmaceutical compositions comprising a compound according to at least one of claims 1 to 5 or a salt according to claim 6 optionally together with one or more inert carriers and/or diluents.

8. Use of a compound according to at least one of claims 1 to 5 or a salt according to claim 6 for preparing a pharmaceutical composition which is suitable for the treatment of type I and type II diabetes mellitus, arthritis, obesity, allograft transplantation and osteoporosis caused by calcitonin.

9. Process for preparing a pharmaceutical composition according to claim 7, **characterised in that** a compound according to at least one of claims 1 to 5 or a salt according to claim 6 is incorporated in one or more inert carriers and/or diluents by a non-chemical method.

10. Process for preparing the compounds of formula I according to claims 1 to 6,
**characterised in that**
a) a compound of general formula 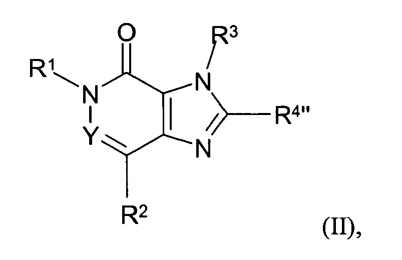
wherein R¹, R², Y and R³ are as hereinbefore defined and
R⁴" denotes one of the groups mentioned for R⁴ hereinbefore which contain an imino, amino or alkylamino group, where the imino, amino or alkylamino group is substituted by a protective group, is deprotected and
subsequently, if desired, a protective group used during the reactions to protect reactive groups is cleaved, and/or
a compound of general formula I thus obtained is resolved into its stereoisomers and/or
a compound of general formula I thus obtained is converted into its salts, particularly for pharmaceutical use into the physiologically acceptable salts thereof with an inorganic or organic acid.

## Revendications

1. Composés de la formule générale : 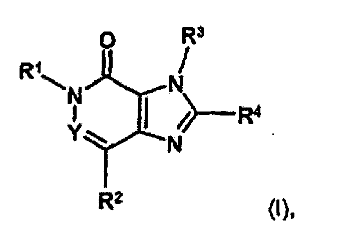 dans laquelle :
R¹ représente un groupe alkyle en C₁₋₃, substitué par un groupe Rₐ, où
Rₐ représente un groupe 3,4-dihydroquinoléinyle, 3,4-dihydroisoquinoléinyle, 1,4-dihydroquinazolinyle, 3,4-dihydroquinazolinyle, 1H-benzo[d][1,2]oxazinyle, 4H-benzo[e][1,3]oxazinyle, 4H-benzo[d][1,3]oxazinyle ou 2H-benzo[1,4]oxazinyle, où
dans chaque partie benzo, un à trois groupes méthine peuvent être remplacés par un atome d'azote et, dans la partie hétérocyclyle, un groupe méthylène peut être remplacé par un groupe carbonyle,
un groupe 4H-benzo[e][1,3]thiazinyle, 4H-benzo[d][1,3]thiazinyle ou 2H-benzo[1,4]thiazinyle, où
dans chaque partie benzo, un à trois groupes méthine peuvent être remplacés par un atome d'azote et dans la partie hétérocyclyle, un groupe méthylène peut être remplacé par un groupe carbonyle, et l'atome de soufre peut être remplacé par un groupe sulfinyle ou sulfonyle,
un groupe 2-oxo-2H-benzo[e][1,3]oxazinyle ou 2,2-dioxo-1H-benzo[c]-[1,2]thiazinyle, où
dans chaque partie benzo, un à trois groupes méthine peuvent être remplacés par un atome d'azote,
un groupe 2,3-dihydro-1H-benzo[e][1,4]diazépinyle, 4,5-dihydro-3H-benzo[b][1,4]diazépinyle ou 5-oxo-4,5-dihydro-3H-benzo[e][1,4]diazépinyle, où
dans chaque partie benzo, un à trois groupes méthine peuvent être remplacés par un atome d'azote et dans chaque partie hétérocyclyle, un groupe méthylène peut être remplacé par un groupe carbonyle,
un groupe 2,3-dihydrobenzo[f][1,4]oxazépinyle ou 2,3-dihydro-benzo[b]-[1,4]oxazépinyle, où
dans chaque partie benzo, un à trois groupes méthine peuvent être remplacés par un atome d'azote et dans chaque partie hétérocyclyle, un groupe méthylène peut être remplacé par un groupe carbonyle,
un groupe 2,3-dihydrobenzo[b][1,4]thiazépinyle ou 2,3-dihydro-benzo[f]-[1,4]thiazépinyle, où
dans chaque partie benzo, un à trois groupes méthine peuvent être remplacés par un atome d'azote et dans chaque partie hétérocyclyle, un groupe méthylène peut être remplacé par un groupe carbonyle, et l'atome de soufre peut être remplacé par un groupe sulfinyle ou sulfonyle,
un groupe 5-oxo-4,5-dihydrobenzo[f][1,3,4]oxadiazépinyle, dans lequel
dans la partie benzo, un à trois groupes méthine peuvent être remplacés par un atome d'azote,
un groupe 11*H*-dibenzo[b,e]azépinyle ou 5H-dibenzo[a,d]cycloheptènyle, où
dans chaque partie benzo, un à trois groupes méthine peuvent être remplacés par un atome d'azote, et le groupe méthylène dans la partie hétérocycyle peut être remplacé par un atome d'oxygène ou de soufre, un groupe carbonyle, sulfinyle ou sulfonyle, ou par un groupe imino substitué par Rₓ, où
Rₓ représente un atome d'hydrogène ou un groupe alkyle en C₁₋₄, alcényle en C₂₋₄, alcynyle en C₂₋₄, cycloalkyle en C₃₋₆, (cycloalkyle en C₃₋₆)alkyle en C₁₋₃, aryle, aryl(alkyle en C₁₋₃), hydroxyalkyle en C₂₋₄, (alcoxy en C₁₋₃)alkyle en C₂₋₄, (cycloalcoxy en C₃₋₆)alkyle en C₂₋₄, aminoalkyle en C₂₋₄, (alkylamino en C₁₋₃)alkyle en C₂₋₄, di(alkyle en C₁₋₃)aminoalkyle en C₂₋₄, (alkyle en C₁₋₃)carbonyle, (alcoxy en C₁₋₃)carbonyle, (alcoxy en C₁₋₃)carbonylalkyle en C₁₋₃, arylcarbonyle, (alkyle en C₁₋₃)sulfonyle ou arylsulfonyle,
un groupe phénanthridinyle, dans lequel
dans la partie benzo, un à trois groupes méthine peuvent être remplacés par un atome d'azote, et
un groupe 1,2,3,4-tétrahydrophénanthridinyle, 1,2,3,4,4a-10b-hexahydrophénanthridinyle, 2,3-dihydro-1*H*-4-azacyclopenta[a]naphtyle ou 8,9,10,11-tétrahydro-7*H-*6-azacyclohepta[a]naphtyle, où
dans chaque partie benzo, un à trois groupes méthine peuvent être remplacés par un atome d'azote, et un ou deux groupes méthylène peuvent être remplacés chacun, par un atome d'oxygène ou un groupe carbonyle,
où si deux groupes méthylène sont remplacés chacun par un atome d'oxygène, les atomes d'oxygène doivent être séparés par au moins deux unités méthylène,
un groupe phénanthrényle, dans lequel
un à trois groupes méthine aux positions 1 à 4 et 5 à 8 pouvant être remplacés chacun par un atome d'azote, et
un groupe 1,2,3,4-tétrahydrophénanthényle ou un groupe 1,2,3,4,5,6,7,8-octahydrophénanthényle, où
un ou deux groupes méthylène aux positions 1 à 4 et 5 à 8 peuvent être remplacés chacun par un atome d'oxygène ou un groupe carbonyle, à condition que, si deux groupes méthylène sont remplacés chacun par un atome d'oxygène, les atomes d'oxygène doivent être séparés par au moins deux unités méthylène,
un groupe 5*H*-benzo[e]pyrrolo[1,2-a][1,4]diazépinyle, thiéno[3,2-b][1,4]-benzoxazépinyle, 5*H*-dibenzo[d,f][1,3]diazépinyle ou 5-oxa-7-azadibenzo[a,c]-cycloheptényle, où
dans chaque partie benzo, un à trois groupes méthine peuvent être remplacés chacun par un atome d'azote,
un groupe naphto[1,2-*d*]oxazolyle, naphto[2,1-*d*]oxazolyle, naphto[1,2-*d*]thiazolyle, naphto[2,1-*d*]thiazolyle, naphto[1,2-*d*]imidazolyle, naphto[1,2-*b*]furanyle ou naphto[2,1-*b*]furanyle, où
dans chaque partie naphtyle, un à trois groupes méthine peuvent être remplacés par un atome d'azote,
un groupe furo[3,2-c]isoquinoléinyle, pyrazolo[1,5-c]quinazolinyle ou 1H-perimidinyle,
les groupes méthylène et méthine du reste Rₐ mentionnés précédemment pouvant être substitués par les groupes R¹⁰ à R¹³ et, en outre, par un groupe alkyle en C₁₋₃ et les groupes imino du reste Rₐ indiqué précédemment pouvant être substitués par les restes Rₓ définis précédemment, et
R¹⁰ représente un atome d'hydrogène,
un atome de fluor, de chlore, de brome ou d'iode,
un groupe alkyle en C₁₋₄, hydroxy ou alcoxy en C₁₋₄,
un groupe nitro, amino, (alkyle en C₁₋₃)amino, di(alkyle en C₁₋₃)amino, cyano(alkyle en C₁₋₃)amino, N-(cyanoalkyle en C₁₋₃)-N-(alkyle en C₁₋₃)amino, (alkyloxy en C₁₋₃)carbonyl(alkyle en C₁₋₃)amino, pyrrolidin-1-yle, pipéridin-1-yle, morpholin-4-yle, pipérazin-1-yle ou 4-(alkyle en C₁₋₃)pipérazin-1-yle,
un groupe (alkyle en C₁₋₃)carbonylamino, arylcarbonylamino, aryl(alkyle en C₁₋₃)carbonylamino, (alkyloxy en C₁₋₃)carbonylamino, aminocarbonylamino, (alkyle en C₁₋₃)aminocarbonylamino, di(alkyle en C₁₋₃)aminocarbonylamino, pyrrolidin-1-ylcarbonylamino, pipéridin-1-ylcarbonylamino, morpholin-4-ylcarbonylamino, pipérazin-1-ylcarbonylamino ou 4-(alkyle en C₁₋₃)pipérazin-1-ylcarbonylamino, (alkyle en C₁₋₃)sulfonylamino, bis(alkylsulfonyl en C₁₋₃)amino, aminosulfonylamino, (alkyle en C₁₋₃)aminosulfonylamino, di(alkyle en C₁₋₃)aminosulfonylamino, pyrrolidin-1-ylsulfonylamino, pipéridin-1-ylsulfonylamino, morpholin-4-ylsulfonylamino, pipérazin-1-ylsulfonylamino ou 4-(alkyle en C₁₋₃)pipérazin-1-ylsulfonylamino, (alkylamino en C₁₋₃)thiocarbonylamino, (alkyloxy en C₁₋₃)carbonylamino-carbonylamino, arylsulfonylamino ou aryl(alkyle en C₁₋₃)sulfonylamino, un groupe N-(alkyle en C₁₋₃)-(alkyle en C₁₋₃)carbonylamino, N-(alkyle en C₁₋₃)-arylcarbonylamino, N-(alkyle en C₁₋₃)-aryl(alkyle en C₁₋₃)-carbonylamino, N-(alkyle en C₁₋₃)-(alkyloxy en C₁₋₃)carbonylamino, N-(aminocarbonyl)-(alkyle en C₁₋₃)amino, N-[(alkyle en C₁₋₃)aminocarbonyl]-(alkyle en C₁₋₃)amino, N-[di(alkyle en C₁₋₃)aminocarbonyl]-(alkyle en C₁₋₃)amino, N-(alkyle en C₁₋₃)-(alkyle en C₁₋₃)sulfonylamino, N-(alkyle en C₁₋₃)-arylsulfonylamino, ou N-(alkyle en C₁₋₃)-aryl(alkyle en C₁₋₃)sulfonylamino,
un groupe 2-oxoimidazolidin-1-yle, 2,4-dioxoimidazolidin-1-yle, 2,5-dioxoimidazolidin-1-yle ou 2-oxohexahydropyrimidin-1-yle, dans lesquels l'atome d'azote en position 3 peut être substitué par un groupe méthyle ou éthyle,
un groupe cyano, carboxy, (alkyloxy en C₁₋₃)carbonyle, aminocarbonyle, (alkylamino en C₁₋₃)carbonyle, di(alkyle en C₁₋₃)aminocarbonyle, pyrrolidin-1-ylcarbonyle, pipéridin-1-ylcarbonyle, morpholin-4-ylcarbonyle, pipérazin-1-ylcarbonyle, ou 4-(alkyle en C₁₋₃)pipérazin-1-ylcarbonyle,
un groupe (alkyle en C₁₋₃)carbonyle ou arylcarbonyle,
un groupe carboxy(alkyle en C₁₋₃), (alkyloxy en C₁₋₃)carbonyl(alkyle en C₁₋₃), cyanoalkyle en C₁₋₃, aminocarbonyl(alkyle en C₁₋₃), (alkylamino en C₁₋₃)carbonyl(alkyle en C₁₋₃), di(alkyle en C₁₋₃)aminocarbonyl(alkyle en C₁₋₃), pyrrolidin-1-ylcarbonyl(alkyle en C₁₋₃), pipéridin-1-ylcarbonyl(alkyle en C₁₋₃), morpholin-4-ylcarbonyl(alkyle en C₁₋₃), pipérazin-1-ylcarbonyl(alkyle en C₁₋₃), ou 4-(alkyle en C₁₋₃)pipérazin-1-ylcarbonyl(alkyle en C₁₋₃),
un groupe carboxy(alkyloxy en C₁₋₃), (alkyloxy en C₁₋₃)carbonyl(alkyloxy en C₁₋₃), cyanoalkyloxy en C₁₋₃, aminocarbonyl(alkyloxy en C₁₋₃), (alkylamino en C₁₋₃)carbonyl(alkyloxy en C₁₋₃), di(alkyle en C₁₋₃)aminocarbonyl(alkyloxy en C₁₋₃), pyrrolidin-1-ylcarbonyl(alkyloxy en C₁₋₃), pipéridin-1-ylcarbonyl(alkyloxy en C₁₋₃), morpholin-4-ylcarbonyl(alkyloxy en C₁₋₃), pipérazin-1-ylcarbonyl(alkyloxy en C₁₋₃), ou 4-(alkyle en C₁₋₃)pipérazin-1-ylcarbonyl(alkyloxy en C₁₋₃),
un groupe hydroxyalkyle en C₁₋₃, (alkyloxy en C₁₋₃)alkyle en C_{1-3,} aminoalkyle en C₁₋₃, (alkyle en C₁₋₃)aminoalkyle en C₁₋₃, di(alkyle en C₁₋₃)aminoalkyle en C₁₋₃, pyrrolidin-1-yl(alkyle en C₁₋₃), pipéridin-1-yl(alkyle en C₁₋₃), morpholin-4-yl(alkyle en C₁₋₃), pipérazin-1-yl(alkyle en C₁₋₃) ou 4-(alkyle en C₁₋₃)pipérazin-1-yl(alkyle en C₁₋₃),
un groupe hydroxyalkyloxy en C₁₋₃, (alkyloxy en C₁₋₃)alkyloxy en C₁₋₃, (alkyle en C₁₋₃)sulfanylalkyloxy en C₁₋₃, (alkyle en C₁₋₃)sulfinylalkyloxy en C₁₋₃, (alkyle en C₁₋₃)sulfonylalkyloxy en C₁₋₃, aminoalkyloxy en C₁₋₃, (alkyle en C₁₋₃)aminoalkyloxy en C₁₋₃, di(alkyle en C₁₋₃)aminoalkyloxy en C₁₋₃, pyrrolidin-1-yl(alkyloxy en C₁₋₃), pipéridin-1-yl(alkyloxy en C₁₋₃), morpholin-4-yl(alkyloxy en C₁₋₃), pipérazin-1-yl(alkyloxy en C₁₋₃), ou 4-(alkyle en C₁₋₃)pipérazin-1-yl(alkyloxy en C₁₋₃),
un groupe mercapto, (alkyle en C₁₋₃)sulfanyle, (alkyle en C₁₋₃)sulfinyle, (alkyle en C₁₋₃)sulfonyle, (alkyle en C₁₋₃)sulfonyloxy, arylsulfonyloxy, trifluorométhylsulfanyle, trifluorométhylsulfinyle ou trifluorométhylsulfonyle,
un groupe sulfo, aminosulfonyle, (alkyle en C₁₋₃)aminosulfonyle, di(alkyle en C₁₋₃)aminosulfonyle, pyrrolidin-1-ylsulfonyle, pipéridin-1-ylsulfonyle, morpholin-4-ylsulfonyle, pipérazin-1-ylsulfonyle ou 4-(alkyle en C₁₋₃)pipérazin-1- -ylsulfonyle,
un groupe méthyle ou méthoxy substitué par 1 à 3 atomes de fluor,
un groupe éthyle ou éthoxy substitué par 1 à 5 atomes de fluor,
un groupe alcényle en C₂₋₄ ou alcynyle en C₂₋₄,
un groupe alcényloxy en C₃₋₄ ou alcynyloxy en C₃₋₄,
un groupe cycloalkyle en C₃₋₆ ou cycloalkyloxy en C₃₋₆,
un groupe (cycloalkyle en C₃₋₆)alkyle en C₁₋₃ ou (cycloalkyle en C₃₋₆)-alkyloxy en C₁₋₃, ou
un groupe aryle, aryloxy, aryl(alkyle en C₁₋₃) ou aryl(alkyloxy en C₁₋₃),
R¹¹ et R¹², qui peuvent être identiques ou différents, représentent chacun un atome d'hydrogène, un atome de fluor, de chlore, de brome ou d'iode, un groupe alkyle en C₁₋₃, trifluorométhyle, hydroxy, alkyloxy en C₁₋₃ ou cyano,ou
R¹¹ avec R¹², dans la mesure où ils sont liés à des atomes de carbone voisins, représentent également un groupe méthylènedioxy, difluorométhylènedioxy, éthylènedioxy ou alkylène en C₃₋₅ linéaire, et
R¹³ représente un atome d'hydrogène, un atome de fluor, de chlore ou de brome, un groupe trifluorométhyle, alkyle en C₁₋₃, ou alkyloxy en C₁₋₃,
R² représente un atome d'hydrogène, un atome de fluor ou de chlore,
un groupe alkyle en C₁₋₆,
un groupe alcényle en C₂₋₄,
un groupe alcynyle en C₃₋₄,
un groupe cycloalkyle en C₃₋₆,
un groupe (cycloalkyle en C₃₋₆)alkyle en C₁₋₃,
un groupe tétrahydrofuran-3-yle, tétrahydropyran-3-yle, tétrahydropyran-4-yle, tétrahydrofuranylméthyle, tétrahydropyranylméthyle,
un groupe aryle,
un groupe aryl(alkyle en C₁₋₄),
un groupe aryl(alcényle en C₂₋₃),
un groupe arylcarbonyle,
un groupe arylcarbonyl(alkyle en C₁₋₂),
un groupe hétéroaryle,
un groupe hétéroaryl(alkyle en C₁₋₃),
un groupe furanylcarbonyle, thiénylcarbonyle, thiazolylcarbonyle ou pyridylcarbonyle,
un groupe furanylcarbonylméthyle, thiénylcarbonylméthyle, thiazolylcarbonylméthyle ou pyridylcarbonylméthyle,
un groupe (alkyle en C₁₋₄)carbonyle,
un groupe (alkyle en C₁₋₄)carbonyl(alkyle en C₁₋₂),
un groupe (cycloalkyle en C₃₋₆)carbonyle,
un groupe (cycloalkyle en C₃₋₆)carbonyl(alkyle en C₁₋₂),
un groupe aryl-A ou aryl-A-(alkyle en C₁₋₃), où A représente un atome d'oxygène ou de soufre, un groupe imino, (alkyle en C₁₋₃)imino, sulfinyle ou sulfonyle,
un groupe R_{b}, où
R_{b} représente un groupe cyano, carboxy, (alkyloxy en C₁₋₃)carbonyle, aminocarbonyle, (alkylamino en C₁₋₃)carbonyle, di(alkyle en C₁₋₃)aminocarbonyle, pyrrolidin-1-ylcarbonyle, pipéridin-1-ylcarbonyle, morpholin-4-ylcarbonyle, pipérazin-1-ylcarbonyle, 4-méthylpipérazin-1-ylcarbonyle, 4-éthylpipérazin-1-ylcarbonyle, hydroxy, mercapto, alkyloxy en C₁₋₃, alkylsulfényle en C₁₋₃, alkylsulfinyle en C₁₋₃, alkylsulfonyle en C₁₋₃, amino, (alkyle en C₁₋₃)amino, di(alkyle en C₁₋₃)amino, pyrrolidin-1-yle, pipéridin-1-yle, morpholin-4-yle, pipérazin-1-yle, 4-méthylpipérazin-1-yle ou 4-éthylpipérazin-1-yle,
ou un groupe alkyle en C₁₋₄ substitué par un groupe R_{b}, R_{b} étant défini comme indiqué ci-dessus,
Y représente un atome d'azote ou un groupe de la formule C-R⁵,
où R⁵ est défini comme R² et un des deux restes R² et R⁵ doit être un atome d'hydrogène ou un groupe alkyle en C₁₋₃,
R³ représente un groupe alkyle en C₃₋₈,
un groupe alkyle en C₁₋₃ substitué par un groupe R_{c} ou
R_{c} représente un groupe cycloalkyle en C₃₋₇, le cas échéant substitué par un ou deux groupes alkyle en C₁₋₃,
un groupe cycloalcényle en C₅₋₇, le cas échéant substitué par un ou deux groupes alkyle en C₁₋₃,
un groupe aryle, ou
un groupe furanyle, thiényle, oxazolyle, isoxazolyle, thiazolyle, isothiazolyle, pyridyle, pyridazinyle, pyrimidinyle ou pyrazinyle, où chacun des restes hétérocycliques indiqués ci-dessus peut être substitué, par un ou deux groupes alkyle en C₁₋₃ ou par un atome de fluor, de chlore, de brome ou d'iode, ou par un groupe trifluorométhyle, cyano ou alkyloxy en C₁₋₃,
un groupe alcényle en C₃₋₈,
un groupe alcényle en C₃₋₆, substitué par un atome de fluor, de chlore ou de brome,
ou par un groupe trifluorométhyle,
un groupe alcynyle en C₃₋₈,
un groupe aryle,
un groupe aryl(alcényle en C₂₋₄),
et
R⁴ représente un groupe azétidin-1-yle ou pyrrolidin-1-yle, qui est substitué en position 3 par un groupe amino, (alkyle en C₁₋₃)amino, di(alkyle en C₁₋₃)amino, et qui peut être en outre, substitué par un ou deux groupes alkyle en C₁₋₃,
un groupe pipéridin-1-yle ou hexahydroazépin-1-yle, qui est substitué en position 3 ou 4 par un groupe amino, (alkyle en C₁₋₃)amino ou di(alkyle en C₁₋₃)amino, et qui peut être en outre, substitué par un ou deux groupes alkyle en C₁₋₃,
un groupe 3-aminopipéridin-1-yle, dans lequel la partie pipéridin-1-yle, est substituée en outre par un groupe aminocarbonyle, (alkyle en C₁₋₂)aminocarbonyle, di(alkyle en C₁₋₂)aminocarbonyle, pyrrolidin-1-ylcarbonyle, (2-cyanopyrrolidin-1-yl)carbonyle, thiazolidin-3-ylcarbonyle, (4-cyanothiazolidin-3-yl)carbonyle, pipéridin-1-yl-carbonyle ou morpholin-4-ylcarbonyle,
un groupe 3-aminopipéridin-1-yle, dans lequel la partie pipéridin-1-yle, est substituée en outre en position 4 ou 5, par un groupe hydroxy ou méthoxy,
un groupe 3-aminopipéridin-1-yle, dans lequel le groupe méthylène en position 2 ou 6, est remplacé par un groupe carbonyle,
un groupe pipéridin-1-yle ou hexahydroazépin-1-yle, substitué en position 3 par un groupe amino, (alkyle en C₁₋₂)amino ou di(alkyle en C₁₋₂)amino, dans lesquels chaque fois deux atomes d'hydrogène sur le squelette carboné du groupe pipéridin-1-yle ou hexahydroazépin-1-yle, sont remplacés par un pont alkylène linéaire, où ce pont contient 2 à 5 atomes de carbone, lorsque les deux atomes d'hydrogène se trouvent sur le même atome de carbone, ou 1 à 4 atomes de carbone, lorsque les deux atomes d'hydrogène se trouvent sur des atomes de carbone voisins, ou 1 à 4 atomes de carbone, lorsque les deux atomes d'hydrogène se trouvent sur des atomes de carbone, qui sont séparés par un atome, ou 1 à 3 atomes de carbone, lorsque les deux atomes d'hydrogène se trouvent sur des atomes de carbone, qui sont séparés par deux atomes,
un groupe azétidin-1-yle, pyrrolidin-1-yle, pipéridin-1-yle ou hexahydroazépin-1-yle, qui est substitué par un groupe amino(alkyle en C₁₋₃), (alkyle en C₁₋₃)amino(alkyle en C₁₋₃) ou di(alkyle en C₁₋₃)amino(alkyle en C₁₋₃),
un groupe pipérazin-1-yle ou [1,4]diazépan-1-yle, substitué le cas échéant sur le squelette carboné par un ou deux groupes alkyle en C₁₋₃,
un groupe 3-iminopipérazin-1-yle, 3-imino[1,4]diazépan-1-yle ou 5-imino-[1,4]diazépan-1-yle, substitué le cas échéant sur le squelette carboné par un ou deux groupes alkyle en C₁₋₃,
un groupe [1,4]diazépan-1-yle, substitué le cas échéant sur le squelette carboné par un ou deux groupes alkyle en C₁₋₃, qui est substitué en position 6 par un groupe amino,
un groupe cycloalkyle en C₃₋₇, qui est substitué par un groupe amino, (alkyle en C₁₋₃)amino ou di(alkyle en C₁₋₃)amino,
un groupe cycloalkyle en C₃₋₇, qui est substitué par un groupe amino(alkyle en C₁₋₃), (alkyle en C₁₋₃)amino(alkyle en C₁₋₃), ou di(alkyle en C₁₋₃)amino(alkyle en C₁₋₃),
un groupe (cycloalkyle en C₃₋₇)alkyle en C₁₋₂, qui est substitué dans la partie cycloalkyle, par un groupe amino, (alkyle en C₁₋₃)amino ou di(alkyle en C₁₋₃)amino,
un groupe (cycloalkyle en C₃₋₇)alkyle en C₁₋₂, qui est substitué dans la partie cycloalkyle, par un groupe amino(alkyle en C₁₋₃), (alkyle en C₁₋₃)amino(alkyle en C₁₋₃) ou di(alkyle en C₁₋₃)amino(alkyle en C₁₋₃),
un groupe cycloalkylamino en C₃₋₇, dans lequel la partie cycloalkyle est substituée par un groupe amino, (alkyle en C₁₋₃)amino ou di(alkyle en C₁₋₃)amino, où les deux atomes d'azote sur la partie cycloalkyle sont séparés l'un de l'autre par au moins deux atomes de carbone,
un groupe N-(cycloalkyle en C₃₋₇)-N-(alkyle en C₁₋₃)amino, dans lequel la partie cycloalkyle est substituée par un groupe amino, (alkyle en C₁₋₃)amino ou di(alkyle en C₁₋₃)amino, où les deux atomes d'azote sur la partie cycloalkyle sont séparés l'un de l'autre par au moins deux atomes de carbone,
un groupe cycloalkylamino en C₃₋₇, dans lequel la partie cycloalkyle est substituée par un groupe amino(alkyle en C₁₋₃), (alkyle en C₁₋₃)amino(alkyle en C₁₋₃) ou di(alkyle en C₁₋₃)amino(alkyle en C₁₋₃),
un groupe N-(cycloalkyle en C₃₋₇)-N-(alkyle en C₁₋₃)amino, dans lequel la partie cycloalkyle est substituée par un groupe amino(alkyle en C₁₋₃), (alkyle en C₁₋₃)-amino(alkyle en C₁₋₃) ou di(alkyle en C₁₋₃)amino(alkyle en C₁₋₃),
un groupe (cycloalkyle en C₃₋₇)alkylamino en C₁₋₂, dans lequel la partie cycloalkyle est substituée par un groupe amino(alkyle en C₁₋₃)amino ou di(alkyle en C₁₋₃)amino,
un groupe N-[(cycloalkyle en C₃₋₇)(alkyle en C₁₋₂)]-N-(alkyle en C₁₋₂)amino, dans lequel la partie cycloalkyle est substituée par un groupe amino, (alkyle en C₁₋₃)amino ou di(alkyle en C₁₋₃)amino,
un groupe (cycloalkyle en C₃₋₇)(alkyle en C₁₋₂)amino dans lequel la partie cycloalkyle est substituée par un groupe amino(alkyle en C₁₋₃), (alkyle en C₁-3)amino(alkyle en C₁₋₃) ou di(alkyle en C₁₋₃)amino(alkyle en C₁₋₃),
un groupe N-[(cycloalkyle en C₃₋₇)(alkyle en C₁₋₂)]-N-(alkyle en C₁₋₂)amino, dans lequel la partie cycloalkyle est substituée par un groupe amino(alkyle en C₁₋₃), (alkyle en C₁₋₃)amino(alkyle en C₁₋₃), ou di(alkyle en C₁₋₃)amino(alkyle en C₁₋₃),
un groupe R¹⁹-(alkyle en C₂₋₄)amino, dans lequel R¹⁹ est séparé de l'atome d'azote de la partie (alkyle en C₂₋₄)amino par au moins deux atomes de carbone, et
R¹⁹ représente un groupe amino, (alkyle en C₁₋₃)amino ou di(alkyle en C₁₋₃)-amino,
un groupe R¹⁹-(alkyle en C₂₋₄)amino, dans lequel l'atome d'azote de la partie (alkyle en C₂₋₄)amino est substitué par un groupe alkyle en C₁₋₃, et R¹⁹ est séparé de l'atome d'azote de la partie (alkyle en C₂₋₄)amino par au moins deux atomes de carbone, où R¹⁹ est défini comme indiqué ci-dessus,
un groupe amino substitué par le reste R²⁰, dans lequel
R²⁰ représente un groupe azétidin-3-yle, azétidin-2-ylméthyle, azétidin-3-ylméthyle, pyrrolidin-3-yle, pyrrolidin-2-ylméthyle, pyrrolidin-3-ylméthyle, pipéridin-3-yle, pipéridin-4-yle, pipéridin-2-ylméthyle, pipéridin-3-ylméthyle ou pipéridin-4-ylméthyle, où chacun des restes mentionnés pour R²⁰ peut être substitué par un ou deux groupes alkyle en C₁₋₃,
un groupe amino substitué par le reste R²⁰ et un groupe alkyle en C₁₋₃, dans lequel R²⁰ est défini comme indiqué ci-dessus, chacun des restes mentionnés pour R²⁰ pouvant être substitué par un ou deux groupes alkyle en C₁₋₃,
un groupe R¹⁹-(alkyle en C₃₋₄), dans lequel la partie alkyle en C₃₋₄ est linéaire et peut être substituée en outre, par un ou deux groupes alkyle en C₁₋₃, où R¹⁹ est défini comme indiqué ci-dessus,
un groupe 3-amino-2-oxopipéridin-5-yle ou 3-amino-2-oxo-1-méthylpipéridin-5-yle un groupe pyrrolidin-3-yle, pyrrolidin-3-yl(alkyle en C₁₋₂), pipéridin-3-yle, pipéridin-4-yle, hexahydroazépin-3-yle ou hexahydroazépin-4-yle, qui est substitué en position 1, par groupe amino, (alkyle en C₁₋₃)amino ou di(alkyle en C₁₋₃)amino, ou
un groupe azétidin-2-yl(alkyle en C₁₋₂), azétidin-3-yl(alkyle en C₁₋₂), pyrrolidin-2-yl(alkyle en C₁₋₂), pyrrolidin-3-yle, pipéridin-2-yl(alkyle en C₁₋₂), pipéridin-3-yle, pipéridin-3-yl(alkyle en C₁₋₂), pipéridin-4-yle ou pipéridin-4-yl(alkyle en C₁₋₂), où chacun des groupes précédents peut être substitué par un ou deux groupes alkyle en C₁₋₃,
où l'on entend par groupes aryle mentionnés pour la définition des restes cités précédemment les groupes phényle et naphtyle, éventuellement mono- ou disubstitués par Rₕ, où les substituants peuvent être identiques ou différents et Rₕ représente un atome de fluor, de chlore, de brome ou d'iode, un groupe trifluorométhyle, cyano, nitro, amino, aminocarbonyle, aminosulfonyle, méthylsulfonyle, acétylamino, méthylsulfonylamino, alkyle en C₁₋₃, cyclopropyle, éthényle, éthynyle, hydroxy, alcoxy en C₁₋₃, difluorométhoxy ou trifluorométhoxy,
par groupes hétéroaryle mentionnés pour la définition des restes cités précédemment, on entend un groupe pyrrolyle, furanyle, thiényle, pyridyle, indolyle, benzofuranyle, benzothiophényle, quinoléinyle ou isoquinoléinyle,
ou un groupe pyrrolyle, furanyle, thiényle ou pyridyle, dans lequel un ou deux groupes méthine sont remplacés par un atome d'azote,
ou un groupe indolyle, benzofuranyle, benzothiényle, quinoléinyle ou isoquinoléinyle, dans lequel un à trois groupes méthine sont remplacés par un atome d'azote,
et les groupes hétéroaryle indiqués ci-dessus peuvent être mono- ou disubstitués par Rₕ, où les substituants peuvent être identiques ou différents et Rₕ est défini comme précédemment,
où sauf indication contraire, les groupes alkyle, alcényle et alcynyle peuvent être linéaires ou ramifiés,
et les atomes d'hydrogène des groupes méthyle ou éthyle présents dans les définitions, pouvant être complètement ou partiellement remplacés par des atomes de fluor,
leurs tautomères, énantiomères, diastéréoisomères, leurs mélanges, leurs sels et leurs pro-médicaments, dans la définition des restes indiqués ci-dessus, les groupes carboxy mentionnés pouvant être remplacés par un groupe convertissable *in vivo* en un groupe carboxy ou par un groupe chargé négativement dans les conditions physiologiques, ou dans la définition des restes indiqués ci-dessus, les groupes amino et imino pouvant être substitués par un reste clivable *in vivo.*

2. Composés de la formule générale 1 selon la revendication 1, dans lesquels
R¹ représente un groupe méthyle substitué par un groupe Rₐ, où
Rₐ représente un groupe 3,4-dihydroquinoléinyle,
un groupe 3,4-dihydroisoquinoléinyle,
un groupe 1,4-dihydroquinazolinyle ou 4-oxo-1,4-dihydroquinazolinyle,
un groupe 3,4-dihydroquinazolinyle ou 4-oxo-3,4-dihydroquinazolinyle,
un groupe 1*H*-benzo[d][1,2]oxazinyle ou 1-oxo-1H-benzo[d] [1,2]oxazinyle,
un groupe 4*H*-benzo[e][1,3]oxazinyle ou 4-oxo-4H-benzo[e][1,3]oxazinyle,
un groupe 4*H*-benzo[d][1,3]oxazinyle ou 4-oxo-4H-benzo[d][1,3]oxazinyle,
un groupe 2*H*-benzo[1,4]oxazinyle ou 2-oxo-2H-benzo[1,4]oxazinyle,
un groupe 4*H*-benzo[e][1,3]thiazinyle ou 4-oxo-4H-benzo[e][1,3]thiazinyle,
un groupe 4*H*-benzo[d][1,3]thiazinyle ou 2H-benzo[1,4]thiazinyle,
un groupe 2-oxo-2H-benzo[e][1,3]oxazinyle ou 2,2-dioxo-1*H* benzo[c]-[1,2]thiazinyle,
un groupe 2,3-dihydro-1H-benzo[e][1,4]diazépinyle ou 2-oxo-2,3-dihydro-1*H-*benzo[e][1,4]diazépinyle,
un groupe 4,5-dihydro-3*H*benzo[b][1,4]diazépinyle ou 4-oxo-4,5-dihydro-3*H-*benzo[b][1,4]diazépinyle,
un groupe 5-oxo-4,5-dihydro-3*H*-benzo[e][1,4]diazépinyle,
un groupe 2,3-dihydrobenzo[f][1,4]oxazépinyle ou 2,3-dihydrobenzo[b][1,4]-oxazépinyle,
un groupe 2,3-dihydrobenzo[f][1,4]thiazépinyle ou 2,3-dihydrobenzo[b][1,4]-thiazépinyle,
un groupe 5-oxo-4,5-dihydrobenzo[f][1,3,4Joxadiazépinyle,
un groupe 11*H*-dibenzo[b,e]azépinyle ou 11-oxo-11*H* dibenzo[b,e]azépinyle,
un groupe 11*H*-benzo[e]pyrido[3,2-b]azépinyle ou *5H-*1,9,10-triazadibenzo[a,d]cycloheptényle,
un groupe 5*H*-dibenzo[*b,e*][1,4]diazépinyle ou dibenzo[b,f][1,4]oxazépinyle,
un groupe dibenzo[*b*,*f*][1,4]thiazépinyle, 5-oxodibenzo[*b,f*][1,4]thiazépinyle ou 5,5-dioxodibenzo[*b,f*] [1,4]thiazépinyle,
un groupe 5*H*-dibenzo[a,d]cycloheptényle ou 5H-dibenzo[b,f]azépinyle,
un groupe phénanthridinyle, benzo[c][1,5]naphtyridinyle, benzo[h]-[1,6]naphtyridinyle, benzo[*c*][1,8]naphtyridinyle, benzo[*f*][1,7]naphtyridinyle ou 1, 5, 9-triazaphénanthrényle,
un groupe 1,2,3,4-tétrahydrophénanthridinyle, 1,2,3,4,4a,10b-hexahydrophénanthridinyle, 2,3-dihydro-1*H*-4-azacyclopenta[a]naphtyle ou 8,9, 1 0, 1 1 -tétrahydro-7H-6-azacydohepta[a]naphtyle,
un groupe 2,3-dihydro-1H-4-oxa-10-azaphénanthrényle ou 1-oxo-2,3-dihydro-1H-4-oxa-10-azaphénanthrényle,
un groupe phénanthrényle, benzo[h]quinoléinyle, benzo[f]quinoléinyle ou benzo[f]quinoxalinyle,
un groupe 5H-benzo[e]pyrrolo[1,2-a] [1,4]diazépinyle, thiéno[3,2-b][1,4]benzoxazépinyle, 5H-dibenzo[d,f][1,3]diazépinyle ou 5-oxa-7-azadibenzo-[a,c]cycloheptényle,
un groupe naphto[1,2-d]oxazolyle, naphto[2,1-d]oxazolyle, naphto[1,2-d]thiazolyle, naphto[2,1-d]thiazolyle, naphto[1,2-d]imidazolyle, naphto[1,2-b]furanyle ou naphto [2,1-b] furanyle,
un groupe furo[3,2-c]isoquinoléinyle, pyrazolo[1,5-c]quinazolinyle ou 1H-périmidinyle,
les groupes benzo des restes Rₐ indiqués ci-dessus étant substitués par les groupes R¹⁰ à R¹³ et les unités alkylène des restes Rₐ indiqués ci-dessus pouvant être substituées par un ou deux atomes de fluor ou un ou deux groupes (alkyle en C₁₋₃)-ou (alcoxy en C₁₋₃)carbonyle et les groupes imino des restes Rₐ indiqués ci-dessus pouvant être substitués par un groupe alkyle en C₁₋₃, et
R¹⁰ représente un atome d'hydrogène,
un atome de fluor, de chlore, de brome ou d'iode,
un groupe alkyle en C₁₋₃ ou cyclopropyle,
un groupe hydroxy, alkyloxy en C₁₋₃ ou cyclopropyloxy,
un groupe nitro, amino, (alkyle en C₁₋₃)amino ou di(alkyle en C₁₋₃)amino,
un groupe (alkyle en C₁₋₃)carbonylamino ou (alkyle en C₁₋₃)sulfonylamino,
un groupe cyano, carboxy, (alkyloxy en C₁₋₃)carbonyle, aminocarbonyle, (alkyle en C₁₋₃)aminocarbonyle ou di(alkyle en C₁₋₃)aminocarbonyle,
un groupe mercapto, (alkyle en C₁₋₃)sulfanyle, (alkyle en C₁₋₃)sulfinyle, (alkyle en C₁₋₃)sulfonyle ou aminosulfonyle, ou
un groupe difluorométhyle, trifluorométhyle, difluorométhoxy ou trifluorométhoxy, et
R¹¹,R¹² et R¹³, qui peuvent être identiques ou différents, représentent chacun un atome d'hydrogène, un atome de fluor, de chlore ou de brome, un groupe méthyle, trifluorométhyle ou méthoxy,
R₂ représente un atome d'hydrogène, ou
un groupe alkyle en C₁₋₃, cyclopropyle, trifluorométhyle, cyanométhyle ou 2-cyanoéthyle,
Y représente un atome d'azote ou un groupe de formule C-R⁵,
où R⁵ représente un atome d'hydrogène ou un groupe alkyle en C₁₋₃,
R³ représente un groupe 2-butèn-1-yle ou 3-méthyl-2-butèn-1-yle,
un groupe 1-butèn-1-yle,
un groupe 2-butyn-1-yle, ou
un groupe 1-cyclopentén-1-ylméthyle, et
R⁴ représente un groupe (3-aminopipéridin-1-yle),
où sauf indication contraire, les groupes alkyle indiqués ci-dessus, peuvent être linéaires ou ramifiés,
leurs tautomères, énantiomères, diastéréoisomères, leurs mélanges et leurs sels.

3. Composés de la formule générale I selon la revendication 2, dans lesquels
R¹ représente un groupe méthyle substitué par un groupe Rₐ, où
Rₐ représente un groupe 3,4-dihydroquinoléin-2-yle,
un groupe 3,4-dihydroisoquinoléin-1-yle,
un groupe 1,4-dihydroquinazolin-2-yle ou 4-oxo-1,4-dihydroquinazolin-2-yle,
un groupe 3,4-dihydroquinazolin-2-yle ou 4-oxo-3,4-dihydroquinazolin-2-yle,
un groupe 1H-benzo[d][1,2]oxazin-4-yle ou 1-oxo-1H-benzo[d][1,2]oxazin-4-yle,
un groupe 4H-benzo[e][1,3]oxazin-2-yle ou 4-oxo-4H-benzo[e][1,3]oxazin-2-yle,
un groupe 4H-benzo[d][1,3]oxazin-2-yle ou 4-oxo-4H-benzo[d][1,3]oxazin-2-yle,
un groupe 2H-benzo[1,4]oxazin-3-yle ou 2-oxo-2H-benzo[1,4]oxazin-3-yle,
un groupe 4H-benzo[e][1,3]thiazin-2-yle ou 4-oxo-4H-benzo[e][1,3]thiazin-2-yle,
un groupe 4H-benzo[d][1,3]thiazin-2-yle ou 2H-benzo[1,4]thiazin-3-yle,
un groupe 2-oxo-2H-benzo[e][1,3]oxazin-4-yle ou 2,2-dioxo-1H-benzo[c]-[1,2]thiazin-4-yle,
un groupe 2,3-dihydro-1H-benzo[e][1,4]diazépin-5-yle ou 2-oxo-2,3-dihydro-1H-benzo[e][1,4]diazépin-5-yle,
un groupe 4,5-dihydro-3H-benzo[b][1,4]diazépin-2-yle ou 4-oxo-4,5-dihydro-3H-benzo[b][1,4]diazépin-2-yle,
un groupe 5-oxo-4,5-dihydro-3H-benzo[e][1,4]diazépin-2-yle,
un groupe 2,3-dihydrobenzo[f][1,4]oxazépin-5-yle ou 2,3-dihydrobenzo[b][1,4]-oxazépin-4-yle,
un groupe 2,3-dihydrobenzo[f][1,4]thiazépin-5-yle ou 2,3-dihydrobenzo[b][1,4]-thiazépin-4-yle,
un groupe 5-oxo-4,5-dihydrobenzo[f][1,3,4]oxadiazépin-2-yle,
un groupe 11H-dibenzo[b,e]azépin-6-yle ou 11-oxo-11H-dibenzo[b,e]azépin-6-yle, un groupe 11H-benzo[e]pyrido[3,2-b]azépin-6-yle ou 5H-1,9,10-triazadibenzo[a,d]cycloheptén-11-yle,
un groupe 5H-dibenzo[b,e][1,4]diazépin-11-yle ou dibenzo[b,f][1,4]oxazépin-11-yle,
un groupe dibenzo[b,f][1,4]thiazépin-11-yle, 5-oxodibenzo[b,f][1,4]thiazépin-11-yle ou 5,5-dioxodibenzo[b,f][1,4]thiazépin-11-yle,
un groupe 5H-dibenzo[a,d]cycloheptén-10-yle ou 5H-dibenzo[b,f]azépin-10-yle,
un groupe phénanthridin-6-yle, benzo[c][1,5]naphtyridin-6-yle, benzo[h]-[1,6]naphtyridin-5-yle, benzo[c] [1,8]naphtyridin-6-yle, benzo[f][1,7]naphtyridin-5-yle ou 1,5,9-triazaphénanthrén-10-yle,
un groupe 1,2,3,4-tétrahydrophénanthridin-6-yle, 1,2,3,4,4a,10b-hexahydrophénanthridin-6-yle, 2,3-dihydro-1H-4-azacyclopenta[a]napht-5-yle ou 8,9,10,11 -tétrahydro-7H-6-azacyclohepta[a]napht-5-yle,
un groupe 2,3-dihydro-1H-4-oxa-10-azaphénanthrén-9-yle ou 1-oxo-2,3-dihydro-1H-4-oxa-10-azaphénanthrén-9-yle,
un groupe phénanthrén-9-yle, benzo[h]quinoléin-6-yle, benzo[f]quinoléin-6-yle ou benzo[f]quinoxalin-6-yle,
un groupe 5H-benzo[e]pyrrolo[1,2-a][1,4]diazépin-11-yle, thiéno[3,2-b][1,4]benzoxazépin-9-yle, 5H-dibenzo[d,f][1,3]diazépin-6-yle ou 5-oxa-7-azadibenzo[a,c]cycloheptén-6-yle,
un groupe naphto[1,2-d]oxazol-2-yle, naphto[2,1-d]oxazol-2-yle, naphto[1,2-d]thiazol-2-yle, naphto[2,1-d]thiazol-2-yle, naphto[1,2-d]imidazol-2-yle, naphto[1,2-b]furan-2-yle ou naphto[2,1-b]furan-2-yle,
un groupe furo[3,2-c]isoquinoléin-5-yle, pyrazolo[1,5-c]quinazolin-5-yle ou 1H-périmidin-2-yle,
les groupes benzo des restes Rₐ indiqués ci-dessus, étant substitués par les groupes R¹⁰ à R¹³ et les unités alkylène des restes Rₐ indiqués ci-dessus pouvant être substituées par un ou deux atomes de fluor ou un ou deux groupes méthyle et les groupes imino des restes Rₐ indiqués ci-dessus peuvent être substitués par un groupe méthyle, et
R¹⁰ représente un atome d'hydrogène,
un atome de fluor, de chlore, de brome ou d'iode,
un groupe méthyle ou éthyle,
un groupe hydroxy, méthoxy ou éthoxy,
un groupe difluorométhyle, trifluorométhyle, difluorométhoxy ou trifluorométhoxy, et
R¹¹, R¹² et R¹³, qui peuvent être identiques ou différents, représentent chacun, un atome d'hydrogène, un atome de fluor, de chlore ou de brome, un groupe méthyle, trifluorométhyle ou méthoxy,
R₂ représente un atome d'hydrogène, ou
un groupe méthyle, cyanométhyle, trifluorométhyle, éthyle, 2-cyanoéthyle, propyle, cyclopropyle ou isopropyle,
Y représente un atome d'azote ou un groupe de formule C-R⁵,
où R⁵ représente un atome d'hydrogène ou un groupe méthyle, éthyle, propyle ou isopropyle,
R³ représente un groupe 2-butèn-1-yle ou 3-méthyl-2-butèn-1-yle,
un groupe 1-butèn-1-yle,
un groupe 2-butyn-1-yle, ou
un groupe 1-cyclopentén-1-ylméthyle,
et
R⁴ représente un groupe (3-aminopipéridin-1-yle),
leurs tautomères, énantiomères, diastéréoisomères, leurs mélanges et leurs sels.

4. Composés de la formule générale I selon la revendication 1, dans lesquels
R¹ représente un groupe 4-oxo-3,4-dihydroquinazolin-2-ylméthyle,
un groupe dibenzo[*b*,*f*] [1,4]oxazépin-11-ylméthyle,
un groupe phénanthridin-6-ylméthyle,
un groupe phénanthrén-9-ylméthyle, ou
un groupe naphto[1,2-d]oxazol-2-ylméthyle ou naphto[2,1-d]oxazol-2-ylméthyle,
R₂ représente un atome d'hydrogène ou un groupe méthyle,
Y représente un atome d'azote,
R³ représente un groupe 2-butyn-3-yle, et
R⁴ représente un groupe (3-aminopipéridin-1-yle),
leurs tautomères, énantiomères, diastéréoisomères, leurs mélanges et leurs sels.

5. Composés suivants de la formule générale I selon la revendication 1 :
(1) 2-(3-Aminopipéridin-1-yl)-3-(2-butyn-1-yl)-5-[(dibenzo[b,f][1,4]oxazépin-11-yl)méthyl]-3,5-dihydroimidazo[4,5-d]pyridazin-4-one 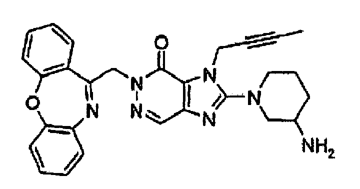
(2) 2-(3-Aminopipéridin-1-yl)-3-(2-butyn-1-yl)-5-[(phénanthridin-6-yl)méthyl]-3,5-dihydroimidazo [4, 5-d] pyridazin-4-one 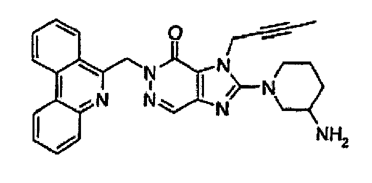
(3) 2-(3-Aminopipéridin-1-yl)-3-(2-butyn-1-yl)-5-[(phénanthrèn-9-yl)méthyl]-3,5-dihydroimidazo[4,5-d]pyridazin-4-one 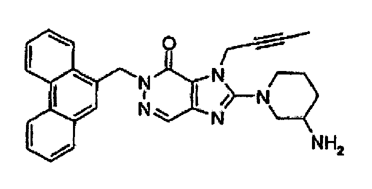
(4) 2-((R)-3-Aminopipéridin-1-yl)-3-(2-butyn-1-yl)-5-[(phénanthridin-6-yl)méthyl]-7-méthyl-3,5-dihydroimidazo[4,5-d]pyridazin-4-one 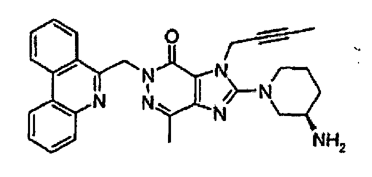
(5) 2-((R)-3-Aminopipéridin-1-yl)-3-(2-butyn-1-yl)-5-[(dibenzo[b,f][1,4]oxazépin-11-yl)méthyl]-7-méthyl-3,5-dihydroimidazo[4,5-d]pyridazin-4-one 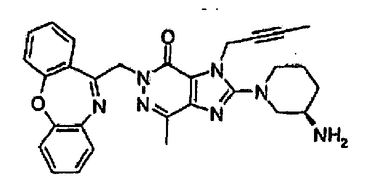
(6) 2-((S)-3-Aminopipéridin-1-yl)-3-(2-butyn-1-yl)-5-[(dibenzo[b*,*f][1,4]oxazépin-11-yl)méthyl]-3,5-dihydroimidazo[4,5-d]pyridazin-4-one 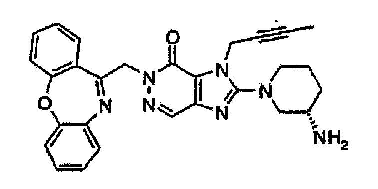
(7) 2-((R)-3-Aminopipéridin-1-yl)-3-(2-butyn-1-yl)-5-[(dibenzo[b,f][1,4]oxazépin-11-yl)méthyl]-3,5-dihydroimidazo[4,5-d]pyridazin-4-one 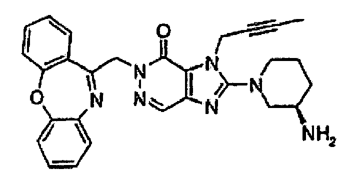
(8) 2-((R)-3-Aminopipéridin-1-yl)-3-(2-butyn-1 -yl)-5-[(naphto[2,1-d]oxazol-2-yl)méthyl]-3,5-dihydroimidazo[4,5-d]pyridazin-4-one 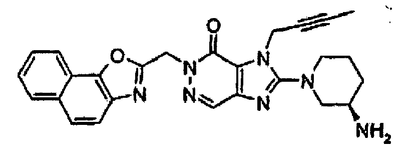
(9) 2-((R)-3-Axninopipéridin-1 -yl)-3-(2-butyn-1-yl)-5-[(naphto[1,2-d]oxazol-2-yl)méthyl]-3,5-dihydroimidazo[4,5-d]pyridazin-4-one 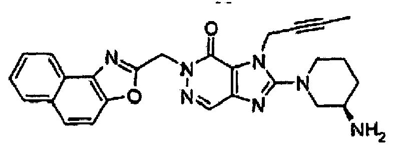
(10) 2-((R)-3-Aminopipéridin-1-yl)-3-(2-butyn-1-yl)-5-[(4-oxo-3,4-dihydroquinazolin-2-yl)méthyl]-3,5-dihydroimidazo[4,5-d]pyridazin-4-one 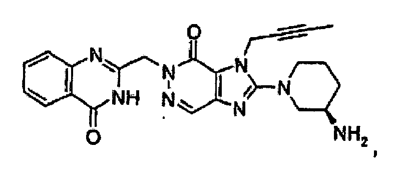
leurs énantiomères, leurs mélanges et leurs sels.

6. Sels physiologiquement acceptables des composés selon les revendications 1 à 5, avec des acides inorganiques ou organiques.

7. Composition pharmaceutique, contenant un composé selon au moins une des revendications 1 à 5, ou un sel selon la revendication 6, avec le cas échéant, un ou plusieurs supports et/ou agents de dilution inertes.

8. Utilisation d'un composé selon au moins une des revendications 1 à 5 ou d'un sel selon la revendication 6, pour la préparation d'un médicament, qui est approprié pour le traitement du diabète de type 1 et de type II, de l'arthrite, de l'adipositose, d'une transplantation d'allogreffe et de l'ostéoporose engendrée par la calcitonine.

9. Procédé de préparation d'un médicament selon la revendication 7, **caractérisé en ce que** l'on incorpore par voie non chimique, un composé selon au moins une des revendications 1 à 5 ou un sel selon la revendication 6, dans un ou plusieurs supports et/ou agents de dilution inertes.

10. Procédé de préparation des composés de formule 1 selon les revendications 1 à 6,
**caractérisé en ce que**
a) un composé de la formule générale : 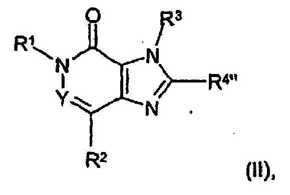
dans laquelle R¹, R², Y et R³ sont définis comme indiqué ci-dessus, et
R^{4"} représente un des groupes mentionnés pour R⁴ ci-dessus, qui contiennent un groupe imino, amino ou alkylamino, où le groupe imino, amino ou alkylamino est substitué par un groupe protecteur, est déprotégé, et
si souhaité, un reste de protection utilisé pendant les réactions pour la protection de groupes réactifs, est clivé, et/ou
un composé ainsi obtenu de la formule générale I est séparé de ses stéréoisomères, et/ou
un composé ainsi obtenu de la formule générale I est converti en son sel, en particulier pour une utilisation pharmaceutique, en son sel physiologiquement acceptable, avec un acide inorganique ou organique.
